(19)

Europäisches
Patentamt
European
Patent Office
Office européen
des brevets

(11)  **EP 3 178 848 A1**

(12)  **EUROPEAN PATENT APPLICATION**

(43) Date of publication:
14.06.2017  Bulletin 2017/24

(21) Application number: 15198715.3

(22) Date of filing: 09.12.2015

(51) Int Cl.:
*C07K 16/28* (2006.01)      *A61K 39/395* (2006.01)
*C07K 16/30* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **F. Hoffmann-La Roche AG**
**4070 Basel (CH)**

(72) Inventor: **The designation of the inventor has not yet been filed**

(74) Representative: **Cueni, Leah Noëmi et al**
**F. Hoffmann-La Roche AG**
**Patent Department**
**Grenzacherstrasse 124**
**4070 Basel (CH)**

(54)  **TYPE II ANTI-CD20 ANTIBODY FOR REDUCING FORMATION OF ANTI-DRUG ANTIBODIES**

(57)      The present invention relates to methods of treating a disease, and methods for reduction of the formation of anti-drug antibodies (ADAs) in response to the administration of a therapeutic agent comprising administration of a Type II anti-CD20 antibody, e.g. obinutuzumab, to the subject prior to administration of the therapeutic agent.

**EP 3 178 848 A1**

SEQUENCE LISTING

<110>  F. Hoffmann-La Roche AG

<120>  Treatment method

<130>  P33257

<160>  43

<170>  PatentIn version 3.5

<210>  1
<211>  297
<212>  PRT
<213>  Homo sapiens

<400>  1

```
Met Thr Thr Pro Arg Asn Ser Val Asn Gly Thr Phe Pro Ala Glu Pro
1               5                   10                  15


Met Lys Gly Pro Ile Ala Met Gln Ser Gly Pro Lys Pro Leu Phe Arg
            20                  25                  30


Arg Met Ser Ser Leu Val Gly Pro Thr Gln Ser Phe Phe Met Arg Glu
        35                  40                  45


Ser Lys Thr Leu Gly Ala Val Gln Ile Met Asn Gly Leu Phe His Ile
    50                  55                  60


Ala Leu Gly Gly Leu Leu Met Ile Pro Ala Gly Ile Tyr Ala Pro Ile
65                  70                  75                  80


Cys Val Thr Val Trp Tyr Pro Leu Trp Gly Gly Ile Met Tyr Ile Ile
                85                  90                  95


Ser Gly Ser Leu Leu Ala Ala Thr Glu Lys Asn Ser Arg Lys Cys Leu
            100                 105                 110


Val Lys Gly Lys Met Ile Met Asn Ser Leu Ser Leu Phe Ala Ala Ile
            115                 120                 125


Ser Gly Met Ile Leu Ser Ile Met Asp Ile Leu Asn Ile Lys Ile Ser
    130                 135                 140


His Phe Leu Lys Met Glu Ser Leu Asn Phe Ile Arg Ala His Thr Pro
145                 150                 155                 160


Tyr Ile Asn Ile Tyr Asn Cys Glu Pro Ala Asn Pro Ser Glu Lys Asn
                165                 170                 175
```

```
Ser Pro Ser Thr Gln Tyr Cys Tyr Ser Ile Gln Ser Leu Phe Leu Gly
        180             185             190

Ile Leu Ser Val Met Leu Ile Phe Ala Phe Phe Gln Glu Leu Val Ile
        195             200             205

Ala Gly Ile Val Glu Asn Glu Trp Lys Arg Thr Cys Ser Arg Pro Lys
    210             215             220

Ser Asn Ile Val Leu Leu Ser Ala Glu Glu Lys Lys Glu Gln Thr Ile
225             230             235             240

Glu Ile Lys Glu Glu Val Val Gly Leu Thr Glu Thr Ser Ser Gln Pro
            245             250             255

Lys Asn Glu Glu Asp Ile Glu Ile Ile Pro Ile Gln Glu Glu Glu Glu
        260             265             270

Glu Glu Thr Glu Thr Asn Phe Pro Glu Pro Pro Gln Asp Gln Glu Ser
        275             280             285

Ser Pro Ile Glu Asn Asp Ser Ser Pro
    290             295
```

```
<210>   2
<211>   112
<212>   PRT
<213>   Mus musculus

<400>   2
```

```
Gly Pro Glu Leu Val Lys Pro Gly Ala Ser Val Lys Ile Ser Cys Lys
1               5               10              15

Ala Ser Gly Tyr Ala Phe Ser Tyr Ser Trp Met Asn Trp Val Lys Leu
        20              25              30

Arg Pro Gly Gln Gly Leu Glu Trp Ile Gly Arg Ile Phe Pro Gly Asp
        35              40              45

Gly Asp Thr Asp Tyr Asn Gly Lys Phe Lys Gly Lys Ala Thr Leu Thr
        50              55              60

Ala Asp Lys Ser Ser Asn Thr Ala Tyr Met Gln Leu Thr Ser Leu Thr
65              70              75              80

Ser Val Asp Ser Ala Val Tyr Leu Cys Ala Arg Asn Val Phe Asp Gly
            85              90              95
```

Tyr Trp Leu Val Tyr Trp Gly Gln Gly Thr Leu Val Thr Val Ser Ala
              100                 105                 110

<210>    3
<211>    103
<212>    PRT
<213>    Mus musculus

<400>    3

Asn Pro Val Thr Leu Gly Thr Ser Ala Ser Ile Ser Cys Arg Ser Ser
1                   5                   10                  15

Lys Ser Leu Leu His Ser Asn Gly Ile Thr Tyr Leu Tyr Trp Tyr Leu
              20                  25                  30

Gln Lys Pro Gly Gln Ser Pro Gln Leu Leu Ile Tyr Gln Met Ser Asn
              35                  40                  45

Leu Val Ser Gly Val Pro Asp Arg Phe Ser Ser Ser Gly Ser Gly Thr
              50                  55                  60

Asp Phe Thr Leu Arg Ile Ser Arg Val Glu Ala Glu Asp Val Gly Val
65                  70                  75                  80

Tyr Tyr Cys Ala Gln Asn Leu Glu Leu Pro Tyr Thr Phe Gly Gly Gly
              85                  90                  95

Thr Lys Leu Glu Ile Lys Arg
              100

<210>    4
<211>    5
<212>    PRT
<213>    Artificial Sequence

<220>
<223>    CD20 HCDR1

<400>    4

Tyr Ser Trp Ile Asn
1                   5

<210>    5
<211>    17
<212>    PRT
<213>    Artificial Sequence

<220>
<223>    CD20 HCDR2

<400>    5

Arg Ile Phe Pro Gly Asp Gly Asp Thr Asp Tyr Asn Gly Lys Phe Lys
1                 5                 10                15

Gly

<210> 6
<211> 10
<212> PRT
<213> Artificial Sequence

<220>
<223> CD20 HCDR3

<400> 6

Asn Val Phe Asp Gly Tyr Trp Leu Val Tyr
1                 5                 10

<210> 7
<211> 16
<212> PRT
<213> Artificial Sequence

<220>
<223> CD20 LCDR1

<400> 7

Arg Ser Ser Lys Ser Leu Leu His Ser Asn Gly Ile Thr Tyr Leu Tyr
1                 5                 10                15

<210> 8
<211> 7
<212> PRT
<213> Artificial Sequence

<220>
<223> CD20 LCDR2

<400> 8

Gln Met Ser Asn Leu Val Ser
1                 5

<210> 9
<211> 9
<212> PRT
<213> Artificial Sequence

<220>
<223> CD20 LCDR3

<400> 9

Ala Gln Asn Leu Glu Leu Pro Tyr Thr
1                 5

```
<210>   10
<211>   119
<212>   PRT
<213>   Artificial Sequence

<220>
<223>   CD20 VH

<400>   10

Gln Val Gln Leu Val Gln Ser Gly Ala Glu Val Lys Lys Pro Gly Ser
1               5                   10                  15


Ser Val Lys Val Ser Cys Lys Ala Ser Gly Tyr Ala Phe Ser Tyr Ser
            20                  25                  30


Trp Ile Asn Trp Val Arg Gln Ala Pro Gly Gln Gly Leu Glu Trp Met
        35                  40                  45


Gly Arg Ile Phe Pro Gly Asp Gly Asp Thr Asp Tyr Asn Gly Lys Phe
    50                  55                  60


Lys Gly Arg Val Thr Ile Thr Ala Asp Lys Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Arg Asn Val Phe Asp Gly Tyr Trp Leu Val Tyr Trp Gly Gln Gly
            100                 105                 110


Thr Leu Val Thr Val Ser Ser
            115


<210>   11
<211>   115
<212>   PRT
<213>   Artificial Sequence

<220>
<223>   CD20 VL

<400>   11

Asp Ile Val Met Thr Gln Thr Pro Leu Ser Leu Pro Val Thr Pro Gly
1               5                   10                  15


Glu Pro Ala Ser Ile Ser Cys Arg Ser Ser Lys Ser Leu Leu His Ser
            20                  25                  30


Asn Gly Ile Thr Tyr Leu Tyr Trp Tyr Leu Gln Lys Pro Gly Gln Ser
        35                  40                  45
```

51

```
Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val
        115


<210>  12
<211>  133
<212>  PRT
<213>  Homo sapiens

<400>  12

Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
            35                  40                  45

Lys Ala Thr Glu Leu Lys His Leu Gln Cys Leu Glu Glu Glu Leu Lys
        50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Cys Gln Ser Ile
        115                 120                 125

Ile Ser Thr Leu Thr
        130
```

```
<210>   13
<211>   133
<212>   PRT
<213>   Artificial Sequence

<220>
<223>   IL2v

<400>   13


Ala Pro Ala Ser Ser Ser Thr Lys Lys Thr Gln Leu Gln Leu Glu His
1               5                   10                  15


Leu Leu Leu Asp Leu Gln Met Ile Leu Asn Gly Ile Asn Asn Tyr Lys
            20                  25                  30


Asn Pro Lys Leu Thr Arg Met Leu Thr Ala Lys Phe Ala Met Pro Lys
        35                  40                  45


Lys Ala Thr Glu Leu Lys His Leu Gln Cys Leu Glu Glu Glu Leu Lys
    50                  55                  60


Pro Leu Glu Glu Val Leu Asn Gly Ala Gln Ser Lys Asn Phe His Leu
65                  70                  75                  80


Arg Pro Arg Asp Leu Ile Ser Asn Ile Asn Val Ile Val Leu Glu Leu
            85                  90                  95


Lys Gly Ser Glu Thr Thr Phe Met Cys Glu Tyr Ala Asp Glu Thr Ala
            100                 105                 110


Thr Ile Val Glu Phe Leu Asn Arg Trp Ile Thr Phe Ala Gln Ser Ile
        115                 120                 125


Ile Ser Thr Leu Thr
        130


<210>   14
<211>   5
<212>   PRT
<213>   Artificial Sequence

<220>
<223>   CEA HCDR1

<400>   14


Glu Phe Gly Met Asn
1               5


<210>   15
```

```
<211>  17
<212>  PRT
<213>  Artificial Sequence

<220>
<223>  CEA HCDR2

<400>  15

Trp Ile Asn Thr Lys Thr Gly Glu Ala Thr Tyr Val Glu Glu Phe Lys
1               5                   10                  15


Gly



<210>  16
<211>  12
<212>  PRT
<213>  Artificial Sequence

<220>
<223>  CEA HCDR3

<400>  16

Trp Asp Phe Ala Tyr Tyr Val Glu Ala Met Asp Tyr
1               5                   10


<210>  17
<211>  11
<212>  PRT
<213>  Artificial Sequence

<220>
<223>  CEA LCDR1

<400>  17

Lys Ala Ser Ala Ala Val Gly Thr Tyr Val Ala
1               5                   10


<210>  18
<211>  7
<212>  PRT
<213>  Artificial Sequence

<220>
<223>  CEA LCDR2

<400>  18

Ser Ala Ser Tyr Arg Lys Arg
1               5


<210>  19
<211>  10
<212>  PRT
<213>  Artificial Sequence
```

```
<220>
<223>   CEA LCDR3

<400>   19

His Gln Tyr Tyr Thr Tyr Pro Leu Phe Thr
1                5                   10


<210>   20
<211>   121
<212>   PRT
<213>   Artificial Sequence

<220>
<223>   CEA VH

<400>   20

Gln Val Gln Leu Val Gln Ser Gly Ala Glu Val Lys Lys Pro Gly Ala
1                5                   10                  15


Ser Val Lys Val Ser Cys Lys Ala Ser Gly Tyr Thr Phe Thr Glu Phe
            20                  25                  30


Gly Met Asn Trp Val Arg Gln Ala Pro Gly Gln Gly Leu Glu Trp Met
            35                  40                  45


Gly Trp Ile Asn Thr Lys Thr Gly Glu Ala Thr Tyr Val Glu Glu Phe
        50                  55                  60


Lys Gly Arg Val Thr Phe Thr Thr Asp Thr Ser Thr Ser Thr Ala Tyr
65                  70                  75                  80


Met Glu Leu Arg Ser Leu Arg Ser Asp Asp Thr Ala Val Tyr Tyr Cys
                85                  90                  95


Ala Arg Trp Asp Phe Ala Tyr Tyr Val Glu Ala Met Asp Tyr Trp Gly
            100                 105                 110


Gln Gly Thr Thr Val Thr Val Ser Ser
            115                 120


<210>   21
<211>   108
<212>   PRT
<213>   Artificial Sequence

<220>
<223>   CEA VL

<400>   21

Asp Ile Gln Met Thr Gln Ser Pro Ser Ser Leu Ser Ala Ser Val Gly
```

```
              1                   5                        10                            15

              Asp Arg Val Thr Ile Thr Cys Lys Ala Ser Ala Ala Val Gly Thr Tyr
                          20                  25                  30

              Val Ala Trp Tyr Gln Gln Lys Pro Gly Lys Ala Pro Lys Leu Leu Ile
                          35                  40                  45

              Tyr Ser Ala Ser Tyr Arg Lys Arg Gly Val Pro Ser Arg Phe Ser Gly
                  50                  55                  60

              Ser Gly Ser Gly Thr Asp Phe Thr Leu Thr Ile Ser Ser Leu Gln Pro
              65                  70                  75                  80

              Glu Asp Phe Ala Thr Tyr Tyr Cys His Gln Tyr Tyr Thr Tyr Pro Leu
                          85                  90                  95

              Phe Thr Phe Gly Gln Gly Thr Lys Leu Glu Ile Lys
                          100                 105
```

```
<210>   22
<211>   598
<212>   PRT
<213>   Artificial Sequence

<220>
<223>   CEA IL2v HC

<400>   22
```

```
              Gln Val Gln Leu Val Gln Ser Gly Ala Glu Val Lys Lys Pro Gly Ala
              1                   5                        10                      15

              Ser Val Lys Val Ser Cys Lys Ala Ser Gly Tyr Thr Phe Thr Glu Phe
                          20                  25                  30

              Gly Met Asn Trp Val Arg Gln Ala Pro Gly Gln Gly Leu Glu Trp Met
                          35                  40                  45

              Gly Trp Ile Asn Thr Lys Thr Gly Glu Ala Thr Tyr Val Glu Glu Phe
                  50                  55                  60

              Lys Gly Arg Val Thr Phe Thr Thr Asp Thr Ser Thr Ser Thr Ala Tyr
              65                  70                  75                  80

              Met Glu Leu Arg Ser Leu Arg Ser Asp Asp Thr Ala Val Tyr Tyr Cys
                          85                  90                  95

              Ala Arg Trp Asp Phe Ala Tyr Tyr Val Glu Ala Met Asp Tyr Trp Gly
                          100                 105                 110
```

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
        130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
                180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
        210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
                260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
        290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
                340                 345                 350

Tyr Thr Leu Pro Pro Cys Arg Asp Glu Leu Thr Lys Asn Gln Val Ser

```
                355                          360                          365

        Leu Trp Cys Leu Val Lys Gly Phe Tyr Pro Ser Asp Ile Ala Val Glu
            370                 375                 380

        Trp Glu Ser Asn Gly Gln Pro Glu Asn Asn Tyr Lys Thr Thr Pro Pro
        385                 390                 395                 400

        Val Leu Asp Ser Asp Gly Ser Phe Phe Leu Tyr Ser Lys Leu Thr Val
                        405                 410                 415

        Asp Lys Ser Arg Trp Gln Gln Gly Asn Val Phe Ser Cys Ser Val Met
                    420                 425                 430

        His Glu Ala Leu His Asn His Tyr Thr Gln Lys Ser Leu Ser Leu Ser
                    435                 440                 445

        Pro Gly Gly Gly Gly Ser Gly Gly Gly Gly Ser Gly Gly Gly Gly
            450                 455                 460

        Ser Ala Pro Ala Ser Ser Ser Thr Lys Lys Thr Gln Leu Gln Leu Glu
        465                 470                 475                 480

        His Leu Leu Leu Asp Leu Gln Met Ile Leu Asn Gly Ile Asn Asn Tyr
                        485                 490                 495

        Lys Asn Pro Lys Leu Thr Arg Met Leu Thr Ala Lys Phe Ala Met Pro
                    500                 505                 510

        Lys Lys Ala Thr Glu Leu Lys His Leu Gln Cys Leu Glu Glu Glu Leu
                    515                 520                 525

        Lys Pro Leu Glu Glu Val Leu Asn Gly Ala Gln Ser Lys Asn Phe His
            530                 535                 540

        Leu Arg Pro Arg Asp Leu Ile Ser Asn Ile Asn Val Ile Val Leu Glu
        545                 550                 555                 560

        Leu Lys Gly Ser Glu Thr Thr Phe Met Cys Glu Tyr Ala Asp Glu Thr
                        565                 570                 575

        Ala Thr Ile Val Glu Phe Leu Asn Arg Trp Ile Thr Phe Ala Gln Ser
                        580                 585                 590

        Ile Ile Ser Thr Leu Thr
                595
```

```
<210>   23
<211>   451
<212>   PRT
<213>   Artificial Sequence

<220>
<223>   CEA HC

<400>   23


Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Glu Phe
            20                  25                  30


Gly Met Asn Trp Val Arg Gln Ala Pro Gly Gln Gly Leu Glu Trp Met
        35                  40                  45


Gly Trp Ile Asn Thr Lys Thr Gly Glu Ala Thr Tyr Val Glu Glu Phe
    50                  55                  60


Lys Gly Arg Val Thr Phe Thr Thr Asp Thr Ser Thr Ser Thr Ala Tyr
65                  70                  75                  80


Met Glu Leu Arg Ser Leu Arg Ser Asp Asp Thr Ala Val Tyr Tyr Cys
                85                  90                  95


Ala Arg Trp Asp Phe Ala Tyr Tyr Val Glu Ala Met Asp Tyr Trp Gly
        100                 105                 110


Gln Gly Thr Thr Val Thr Val Ser Ser Ala Ser Thr Lys Gly Pro Ser
        115                 120                 125


Val Phe Pro Leu Ala Pro Ser Ser Lys Ser Thr Ser Gly Gly Thr Ala
        130                 135                 140


Ala Leu Gly Cys Leu Val Lys Asp Tyr Phe Pro Glu Pro Val Thr Val
145                 150                 155                 160


Ser Trp Asn Ser Gly Ala Leu Thr Ser Gly Val His Thr Phe Pro Ala
            165                 170                 175


Val Leu Gln Ser Ser Gly Leu Tyr Ser Leu Ser Ser Val Val Thr Val
            180                 185                 190


Pro Ser Ser Ser Leu Gly Thr Gln Thr Tyr Ile Cys Asn Val Asn His
            195                 200                 205


Lys Pro Ser Asn Thr Lys Val Asp Lys Lys Val Glu Pro Lys Ser Cys
```

210 215 220

Asp Lys Thr His Thr Cys Pro Pro Cys Pro Ala Pro Glu Ala Ala Gly
225                 230             235                 240

Gly Pro Ser Val Phe Leu Phe Pro Pro Lys Pro Lys Asp Thr Leu Met
                245             250                 255

Ile Ser Arg Thr Pro Glu Val Thr Cys Val Val Val Asp Val Ser His
                260             265                 270

Glu Asp Pro Glu Val Lys Phe Asn Trp Tyr Val Asp Gly Val Glu Val
            275             280             285

His Asn Ala Lys Thr Lys Pro Arg Glu Glu Gln Tyr Asn Ser Thr Tyr
        290             295             300

Arg Val Val Ser Val Leu Thr Val Leu His Gln Asp Trp Leu Asn Gly
305             310             315                 320

Lys Glu Tyr Lys Cys Lys Val Ser Asn Lys Ala Leu Gly Ala Pro Ile
                325             330                 335

Glu Lys Thr Ile Ser Lys Ala Lys Gly Gln Pro Arg Glu Pro Gln Val
            340             345                 350

Cys Thr Leu Pro Pro Ser Arg Asp Glu Leu Thr Lys Asn Gln Val Ser
        355             360             365

Leu Ser Cys Ala Val Lys Gly Phe Tyr Pro Ser Asp Ile Ala Val Glu
        370             375             380

Trp Glu Ser Asn Gly Gln Pro Glu Asn Asn Tyr Lys Thr Thr Pro Pro
385             390             395                 400

Val Leu Asp Ser Asp Gly Ser Phe Phe Leu Val Ser Lys Leu Thr Val
                405             410                 415

Asp Lys Ser Arg Trp Gln Gln Gly Asn Val Phe Ser Cys Ser Val Met
            420             425             430

His Glu Ala Leu His Asn His Tyr Thr Gln Lys Ser Leu Ser Leu Ser
        435             440             445

Pro Gly Lys
    450

```
<210>  24
<211>  215
<212>  PRT
<213>  Artificial Sequence

<220>
<223>  CEA LC

<400>  24
```

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
            85                  90                  95

Phe Thr Phe Gly Gln Gly Thr Lys Leu Glu Ile Lys Arg Thr Val Ala
        100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
        130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
            165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys

210                               215

<210>   25
<211>   117
<212>   PRT
<213>   Artificial Sequence

<220>
<223>   FAP VH

<400>   25

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser
            115

<210>   26
<211>   108
<212>   PRT
<213>   Artificial Sequence

<220>
<223>   FAP VL

<400>   26

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Thr Ser Ser
            20                  25                  30

```
Tyr Leu Ala Trp Tyr Gln Gln Lys Pro Gly Gln Ala Pro Arg Leu Leu
        35                  40                  45


Ile Asn Val Gly Ser Arg Arg Ala Thr Gly Ile Pro Asp Arg Phe Ser
        50                  55                  60


Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Gly Ile Met Leu Pro
                85                  90                  95


Pro Thr Phe Gly Gln Gly Thr Lys Val Glu Ile Lys
                100                 105


<210>   27
<211>   594
<212>   PRT
<213>   Artificial Sequence

<220>
<223>   FAP IL2v HC

<400>   27

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
        20                  25                  30


Ala Met Ser Trp Val Arg Gln Ala Pro Gly Lys Gly Leu Glu Trp Val
        35                  40                  45


Ser Ala Ile Ile Gly Ser Gly Ala Ser Thr Tyr Tyr Ala Asp Ser Val
        50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Gly Trp Phe Gly Gly Phe Asn Tyr Trp Gly Gln Gly Thr Leu
                100                 105                 110


Val Thr Val Ser Ser Ala Ser Thr Lys Gly Pro Ser Val Phe Pro Leu
        115                 120                 125


Ala Pro Ser Ser Lys Ser Thr Ser Gly Gly Thr Ala Ala Leu Gly Cys
```

```
              130                        135                        140

    Leu Val Lys Asp Tyr Phe Pro Glu Pro Val Thr Val Ser Trp Asn Ser
    145                 150                 155                 160

    Gly Ala Leu Thr Ser Gly Val His Thr Phe Pro Ala Val Leu Gln Ser
                    165                 170                 175

    Ser Gly Leu Tyr Ser Leu Ser Ser Val Val Thr Val Pro Ser Ser Ser
                    180                 185                 190

    Leu Gly Thr Gln Thr Tyr Ile Cys Asn Val Asn His Lys Pro Ser Asn
                    195                 200                 205

    Thr Lys Val Asp Lys Lys Val Glu Pro Lys Ser Cys Asp Lys Thr His
                    210                 215                 220

    Thr Cys Pro Pro Cys Pro Ala Pro Glu Ala Ala Gly Gly Pro Ser Val
    225                 230                 235                 240

    Phe Leu Phe Pro Pro Lys Pro Lys Asp Thr Leu Met Ile Ser Arg Thr
                    245                 250                 255

    Pro Glu Val Thr Cys Val Val Val Asp Val Ser His Glu Asp Pro Glu
                    260                 265                 270

    Val Lys Phe Asn Trp Tyr Val Asp Gly Val Glu Val His Asn Ala Lys
                    275                 280                 285

    Thr Lys Pro Arg Glu Glu Gln Tyr Asn Ser Thr Tyr Arg Val Val Ser
                    290                 295                 300

    Val Leu Thr Val Leu His Gln Asp Trp Leu Asn Gly Lys Glu Tyr Lys
    305                 310                 315                 320

    Cys Lys Val Ser Asn Lys Ala Leu Gly Ala Pro Ile Glu Lys Thr Ile
                    325                 330                 335

    Ser Lys Ala Lys Gly Gln Pro Arg Glu Pro Gln Val Tyr Thr Leu Pro
                    340                 345                 350

    Pro Cys Arg Asp Glu Leu Thr Lys Asn Gln Val Ser Leu Trp Cys Leu
                    355                 360                 365

    Val Lys Gly Phe Tyr Pro Ser Asp Ile Ala Val Glu Trp Glu Ser Asn
                    370                 375                 380
```

Gly Gln Pro Glu Asn Asn Tyr Lys Thr Thr Pro Pro Val Leu Asp Ser
385                 390             395                 400

Asp Gly Ser Phe Phe Leu Tyr Ser Lys Leu Thr Val Asp Lys Ser Arg
            405             410             415

Trp Gln Gln Gly Asn Val Phe Ser Cys Ser Val Met His Glu Ala Leu
            420             425             430

His Asn His Tyr Thr Gln Lys Ser Leu Ser Leu Ser Pro Gly Gly Gly
        435             440             445

Gly Gly Ser Gly Gly Gly Gly Ser Gly Gly Gly Ser Ala Pro Ala
    450             455             460

Ser Ser Ser Thr Lys Lys Thr Gln Leu Gln Leu Glu His Leu Leu Leu
465             470             475             480

Asp Leu Gln Met Ile Leu Asn Gly Ile Asn Asn Tyr Lys Asn Pro Lys
            485             490             495

Leu Thr Arg Met Leu Thr Ala Lys Phe Ala Met Pro Lys Lys Ala Thr
            500             505             510

Glu Leu Lys His Leu Gln Cys Leu Glu Glu Glu Leu Lys Pro Leu Glu
        515             520             525

Glu Val Leu Asn Gly Ala Gln Ser Lys Asn Phe His Leu Arg Pro Arg
        530             535             540

Asp Leu Ile Ser Asn Ile Asn Val Ile Val Leu Glu Leu Lys Gly Ser
545             550             555             560

Glu Thr Thr Phe Met Cys Glu Tyr Ala Asp Glu Thr Ala Thr Ile Val
            565             570             575

Glu Phe Leu Asn Arg Trp Ile Thr Phe Ala Gln Ser Ile Ile Ser Thr
        580             585             590

Leu Thr


<210>  28
<211>  447
<212>  PRT
<213>  Artificial Sequence

<220>
<223>  FAP HC

<400> 28

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
            35                  40                  45

Ser Ala Ile Ile Gly Ser Gly Ala Ser Thr Tyr Tyr Ala Asp Ser Val
            50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser Ala Ser Thr Lys Gly Pro Ser Val Phe Pro Leu
            115                 120                 125

Ala Pro Ser Ser Lys Ser Thr Ser Gly Gly Thr Ala Ala Leu Gly Cys
        130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
        210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245             250                 255

Pro Glu Val Thr Cys Val Val Val Asp Val Ser His Glu Asp Pro Glu
                260             265                 270

Val Lys Phe Asn Trp Tyr Val Asp Gly Val Glu Val His Asn Ala Lys
                275             280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Gly Ala Pro Ile Glu Lys Thr Ile
                325                 330                 335

Ser Lys Ala Lys Gly Gln Pro Arg Glu Pro Gln Val Cys Thr Leu Pro
                340                 345                 350

Pro Ser Arg Asp Glu Leu Thr Lys Asn Gln Val Ser Leu Ser Cys Ala
                355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Val Ser Lys Leu Thr Val Asp Lys Ser Arg
                405                 410                 415

Trp Gln Gln Gly Asn Val Phe Ser Cys Ser Val Met His Glu Ala Leu
                420                 425                 430

His Asn His Tyr Thr Gln Lys Ser Leu Ser Leu Ser Pro Gly Lys
                435                 440                 445

<210>  29
<211>  215
<212>  PRT
<213>  Artificial Sequence

<220>
<223>  FAP LC

<400>  29

Glu Ile Val Leu Thr Gln Ser Pro Gly Thr Leu Ser Leu Ser Pro Gly

|     |     | 1   |     |     | 5   |     |     |     |     | 10  |     |     |     |     | 15  |     |
| --- | --- | --- | --- | --- | --- | --- | --- | --- | --- | --- | --- | --- | --- | --- | --- | --- |

Glu Arg Ala Thr Leu Ser Cys Arg Ala Ser Gln Ser Val Thr Ser Ser
            20                      25                      30

Tyr Leu Ala Trp Tyr Gln Gln Lys Pro Gly Gln Ala Pro Arg Leu Leu
            35                      40                      45

Ile Asn Val Gly Ser Arg Arg Ala Thr Gly Ile Pro Asp Arg Phe Ser
        50                      55                      60

Gly Ser Gly Ser Gly Thr Asp Phe Thr Leu Thr Ile Ser Arg Leu Glu
65                      70                      75                      80

Pro Glu Asp Phe Ala Val Tyr Tyr Cys Gln Gln Gly Ile Met Leu Pro
                85                      90                      95

Pro Thr Phe Gly Gln Gly Thr Lys Val Glu Ile Lys Arg Thr Val Ala
            100                     105                     110

Ala Pro Ser Val Phe Ile Phe Pro Pro Ser Asp Glu Gln Leu Lys Ser
        115                     120                     125

Gly Thr Ala Ser Val Val Cys Leu Leu Asn Asn Phe Tyr Pro Arg Glu
        130                     135                     140

Ala Lys Val Gln Trp Lys Val Asp Asn Ala Leu Gln Ser Gly Asn Ser
145                     150                     155                     160

Gln Glu Ser Val Thr Glu Gln Asp Ser Lys Asp Ser Thr Tyr Ser Leu
            165                     170                     175

Ser Ser Thr Leu Thr Leu Ser Lys Ala Asp Tyr Glu Lys His Lys Val
            180                     185                     190

Tyr Ala Cys Glu Val Thr His Gln Gly Leu Ser Ser Pro Val Thr Lys
            195                     200                     205

Ser Phe Asn Arg Gly Glu Cys
        210                 215

<210>   30
<211>   225
<212>   PRT
<213>   Homo sapiens

<400>   30

Asp Lys Thr His Thr Cys Pro Pro Cys Pro Ala Pro Glu Leu Leu Gly

```
     1                   5                          10                          15


         Gly Pro Ser Val Phe Leu Phe Pro Pro Lys Pro Lys Asp Thr Leu Met
                     20                  25                  30


         Ile Ser Arg Thr Pro Glu Val Thr Cys Val Val Val Asp Val Ser His
                     35                  40                  45


         Glu Asp Pro Glu Val Lys Phe Asn Trp Tyr Val Asp Gly Val Glu Val
                 50                  55                  60


         His Asn Ala Lys Thr Lys Pro Arg Glu Glu Gln Tyr Asn Ser Thr Tyr
         65                  70                  75                  80


         Arg Val Val Ser Val Leu Thr Val Leu His Gln Asp Trp Leu Asn Gly
                         85                  90                  95


         Lys Glu Tyr Lys Cys Lys Val Ser Asn Lys Ala Leu Pro Ala Pro Ile
                     100                 105                 110


         Glu Lys Thr Ile Ser Lys Ala Lys Gly Gln Pro Arg Glu Pro Gln Val
                     115                 120                 125


         Tyr Thr Leu Pro Pro Ser Arg Asp Glu Leu Thr Lys Asn Gln Val Ser
             130                 135                 140


         Leu Thr Cys Leu Val Lys Gly Phe Tyr Pro Ser Asp Ile Ala Val Glu
         145                 150                 155                 160


         Trp Glu Ser Asn Gly Gln Pro Glu Asn Asn Tyr Lys Thr Thr Pro Pro
                         165                 170                 175


         Val Leu Asp Ser Asp Gly Ser Phe Phe Leu Tyr Ser Lys Leu Thr Val
                     180                 185                 190


         Asp Lys Ser Arg Trp Gln Gln Gly Asn Val Phe Ser Cys Ser Val Met
                     195                 200                 205


         His Glu Ala Leu His Asn His Tyr Thr Gln Lys Ser Leu Ser Leu Ser
             210                 215                 220


         Pro
         225


         <210>   31
         <211>   20
         <212>   PRT
         <213>   Homo sapiens
```

<400> 31

Met Tyr Arg Met Gln Leu Leu Ser Cys Ile Ala Leu Ser Leu Ala Leu
1                   5                   10                  15

Val Thr Asn Ser
            20

<210>   32
<211>   5
<212>   PRT
<213>   Artificial Sequence

<220>
<223>   CD3 HCDR1

<400>   32

Thr Tyr Ala Met Asn
1                   5

<210>   33
<211>   19
<212>   PRT
<213>   Artificial Sequence

<220>
<223>   CD3 HCDR2

<400>   33

Arg Ile Arg Ser Lys Tyr Asn Asn Tyr Ala Thr Tyr Tyr Ala Asp Ser
1                   5                   10                  15

Val Lys Gly

<210>   34
<211>   14
<212>   PRT
<213>   Artificial Sequence

<220>
<223>   CD3 HCDR3

<400>   34

His Gly Asn Phe Gly Asn Ser Tyr Val Ser Trp Phe Ala Tyr
1                   5                   10

<210>   35
<211>   14
<212>   PRT
<213>   Artificial Sequence

<220>

<223> CD3 LCDR1

<400> 35

Gly Ser Ser Thr Gly Ala Val Thr Thr Ser Asn Tyr Ala Asn
1               5                   10


<210> 36
<211> 7
<212> PRT
<213> Artificial Sequence

<220>
<223> CD3 LCDR2

<400> 36

Gly Thr Asn Lys Arg Ala Pro
1               5


<210> 37
<211> 9
<212> PRT
<213> Artificial Sequence

<220>
<223> CD3 LCDR3

<400> 37

Ala Leu Trp Tyr Ser Asn Leu Trp Val
1               5


<210> 38
<211> 125
<212> PRT
<213> Artificial Sequence

<220>
<223> CD3 VH

<400> 38

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Thr Tyr
            20                  25                  30


Ala Met Asn Trp Val Arg Gln Ala Pro Gly Lys Gly Leu Glu Trp Val
            35                  40                  45


Ser Arg Ile Arg Ser Lys Tyr Asn Asn Tyr Ala Thr Tyr Tyr Ala Asp
        50                  55                  60


Ser Val Lys Gly Arg Phe Thr Ile Ser Arg Asp Asp Ser Lys Asn Thr

```
           65                    70                   75                   80

Leu Tyr Leu Gln Met Asn Ser Leu Arg Ala Glu Asp Thr Ala Val Tyr
                85                   90                   95

Tyr Cys Val Arg His Gly Asn Phe Gly Asn Ser Tyr Val Ser Trp Phe
               100                  105                  110

Ala Tyr Trp Gly Gln Gly Thr Leu Val Thr Val Ser Ser
               115                  120                  125

<210>   39
<211>   109
<212>   PRT
<213>   Artificial Sequence

<220>
<223>   CD3 VL

<400>   39

Gln Ala Val Val Thr Gln Glu Pro Ser Leu Thr Val Ser Pro Gly Gly
1                 5                   10                  15

Thr Val Thr Leu Thr Cys Gly Ser Ser Thr Gly Ala Val Thr Thr Ser
              20                  25                  30

Asn Tyr Ala Asn Trp Val Gln Glu Lys Pro Gly Gln Ala Phe Arg Gly
            35                  40                  45

Leu Ile Gly Gly Thr Asn Lys Arg Ala Pro Gly Thr Pro Ala Arg Phe
        50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
               100                  105

<210>   40
<211>   215
<212>   PRT
<213>   Artificial Sequence

<220>
<223>   CEA CD3 bsAb LC(CEA)

<400>   40
```

```
Asp Ile Gln Met Thr Gln Ser Pro Ser Ser Leu Ser Ala Ser Val Gly
1               5               10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
            35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

```
<210>   41
<211>   214
<212>   PRT
<213>   Artificial Sequence

<220>
<223>   CEA CD3 bsAb LC(CD3)
```

<400> 41

Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Glu Lys Pro Gly Gln Ala Phe Arg Gly
        35                  40                  45

Leu Ile Gly Gly Thr Asn Lys Arg Ala Pro Gly Thr Pro Ala Arg Phe
        50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Ser Ser Ala
            100                 105                 110

Ser Thr Lys Gly Pro Ser Val Phe Pro Leu Ala Pro Ser Ser Lys Ser
            115                 120                 125

Thr Ser Gly Gly Thr Ala Ala Leu Gly Cys Leu Val Lys Asp Tyr Phe
            130                 135                 140

Pro Glu Pro Val Thr Val Ser Trp Asn Ser Gly Ala Leu Thr Ser Gly
145                 150                 155                 160

Val His Thr Phe Pro Ala Val Leu Gln Ser Ser Gly Leu Tyr Ser Leu
                165                 170                 175

Ser Ser Val Val Thr Val Pro Ser Ser Ser Leu Gly Thr Gln Thr Tyr
            180                 185                 190

Ile Cys Asn Val Asn His Lys Pro Ser Asn Thr Lys Val Asp Lys Lys
        195                 200                 205

Val Glu Pro Lys Ser Cys
    210

<210>    42
<211>    694
<212>    PRT
<213>    Artificial Sequence

<220>
<223>   CEA CD3 bsAB HC(CEA-CD3-Fc)

<400>   42

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
                20                  25                  30

Gly Met Asn Trp Val Arg Gln Ala Pro Gly Gln Gly Leu Glu Trp Met
            35                  40                  45

Gly Trp Ile Asn Thr Lys Thr Gly Glu Ala Thr Tyr Val Glu Glu Phe
        50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
            115                 120                 125

Val Phe Pro Leu Ala Pro Ser Ser Lys Ser Thr Ser Gly Gly Thr Ala
        130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
                180                 185                 190

Pro Ser Ser Ser Leu Gly Thr Gln Thr Tyr Ile Cys Asn Val Asn His
            195                 200                 205

Lys Pro Ser Asn Thr Lys Val Asp Lys Lys Val Glu Pro Lys Ser Cys
        210                 215                 220

Asp Gly Gly Gly Gly Ser Gly Gly Gly Gly Ser Glu Val Gln Leu Leu

225                          230                          235                          240

Glu Ser Gly Gly Gly Leu Val Gln Pro Gly Gly Ser Leu Arg Leu Ser
            245                 250                 255

Cys Ala Ala Ser Gly Phe Thr Phe Ser Thr Tyr Ala Met Asn Trp Val
            260                 265                 270

Arg Gln Ala Pro Gly Lys Gly Leu Glu Trp Val Ser Arg Ile Arg Ser
            275                 280                 285

Lys Tyr Asn Asn Tyr Ala Thr Tyr Tyr Ala Asp Ser Val Lys Gly Arg
            290                 295                 300

Phe Thr Ile Ser Arg Asp Asp Ser Lys Asn Thr Leu Tyr Leu Gln Met
305                 310                 315                 320

Asn Ser Leu Arg Ala Glu Asp Thr Ala Val Tyr Tyr Cys Val Arg His
            325                 330                 335

Gly Asn Phe Gly Asn Ser Tyr Val Ser Trp Phe Ala Tyr Trp Gly Gln
            340                 345                 350

Gly Thr Leu Val Thr Val Ser Ser Ala Ser Val Ala Ala Pro Ser Val
            355                 360                 365

Phe Ile Phe Pro Pro Ser Asp Glu Gln Leu Lys Ser Gly Thr Ala Ser
            370                 375                 380

Val Val Cys Leu Leu Asn Asn Phe Tyr Pro Arg Glu Ala Lys Val Gln
385                 390                 395                 400

Trp Lys Val Asp Asn Ala Leu Gln Ser Gly Asn Ser Gln Glu Ser Val
            405                 410                 415

Thr Glu Gln Asp Ser Lys Asp Ser Thr Tyr Ser Leu Ser Ser Thr Leu
            420                 425                 430

Thr Leu Ser Lys Ala Asp Tyr Glu Lys His Lys Val Tyr Ala Cys Glu
            435                 440                 445

Val Thr His Gln Gly Leu Ser Ser Pro Val Thr Lys Ser Phe Asn Arg
            450                 455                 460

Gly Glu Cys Asp Lys Thr His Thr Cys Pro Pro Cys Pro Ala Pro Glu
465                 470                 475                 480

Ala Ala Gly Gly Pro Ser Val Phe Leu Phe Pro Pro Lys Pro Lys Asp
485 490 495

Thr Leu Met Ile Ser Arg Thr Pro Glu Val Thr Cys Val Val Val Asp
500 505 510

Val Ser His Glu Asp Pro Glu Val Lys Phe Asn Trp Tyr Val Asp Gly
515 520 525

Val Glu Val His Asn Ala Lys Thr Lys Pro Arg Glu Glu Gln Tyr Asn
530 535 540

Ser Thr Tyr Arg Val Val Ser Val Leu Thr Val Leu His Gln Asp Trp
545 550 555 560

Leu Asn Gly Lys Glu Tyr Lys Cys Lys Val Ser Asn Lys Ala Leu Gly
565 570 575

Ala Pro Ile Glu Lys Thr Ile Ser Lys Ala Lys Gly Gln Pro Arg Glu
580 585 590

Pro Gln Val Tyr Thr Leu Pro Pro Cys Arg Asp Glu Leu Thr Lys Asn
595 600 605

Gln Val Ser Leu Trp Cys Leu Val Lys Gly Phe Tyr Pro Ser Asp Ile
610 615 620

Ala Val Glu Trp Glu Ser Asn Gly Gln Pro Glu Asn Asn Tyr Lys Thr
625 630 635 640

Thr Pro Pro Val Leu Asp Ser Asp Gly Ser Phe Phe Leu Tyr Ser Lys
645 650 655

Leu Thr Val Asp Lys Ser Arg Trp Gln Gln Gly Asn Val Phe Ser Cys
660 665 670

Ser Val Met His Glu Ala Leu His Asn His Tyr Thr Gln Lys Ser Leu
675 680 685

Ser Leu Ser Pro Gly Lys
690

<210> 43
<211> 451
<212> PRT
<213> Artificial Sequence

<220>
<223> CEA CD3 bsAB HC(CEA-Fc)

77

<400> 43

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
            35                  40                  45

Gly Trp Ile Asn Thr Lys Thr Gly Glu Ala Thr Tyr Val Glu Glu Phe
        50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
            115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
            195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

```
Gly Pro Ser Val Phe Leu Phe Pro Pro Lys Pro Lys Asp Thr Leu Met
            245                 250             255

Ile Ser Arg Thr Pro Glu Val Thr Cys Val Val Val Asp Val Ser His
            260                 265             270

Glu Asp Pro Glu Val Lys Phe Asn Trp Tyr Val Asp Gly Val Glu Val
            275                 280             285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
            325                 330             335

Glu Lys Thr Ile Ser Lys Ala Lys Gly Gln Pro Arg Glu Pro Gln Val
            340                 345             350

Cys Thr Leu Pro Pro Ser Arg Asp Glu Leu Thr Lys Asn Gln Val Ser
            355                 360             365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
            405                 410             415

Asp Lys Ser Arg Trp Gln Gln Gly Asn Val Phe Ser Cys Ser Val Met
            420                 425             430

His Glu Ala Leu His Asn His Tyr Thr Gln Lys Ser Leu Ser Leu Ser
    435                 440                 445

Pro Gly Lys
    450
```

**Description**

**Field of the Invention**

The present invention relates to methods of treating a disease, and methods for reduction of the formation of anti-drug antibodies (ADAs) in response to the administration of a therapeutic agent.

**Background**

The number of biotechnology-derived therapeutic agents available for use in clinical settings has dramatically increased in recent years, and includes recombinant human cytokines (e.g. $\alpha$ and $\beta$ interferon, interleukin-2), cellular growth factors (e.g. GM-CSF), hormones (e.g. glucagon), neuromuscular antagonists (e.g. botulinum toxin), blood products (e.g. clotting factor VIII), recombinant receptors (e.g. etanercept) and monoclonal antibodies. Although therapeutic proteins are generally considered safe and non-toxic, antibodies against these therapeutic agents, known as anti-drug antibodies (ADAs), can develop during treatment.

ADAs have been observed in connection with various therapeutic agents, such as erythropoietin, factor VIII, insulin, immunotoxins and monoclonal antibodies (Schellekens and Casadevall, J Neurol (2004), 251 [Suppl 2]:II/4-II/9; Mossoba et al., Clin Cancer Res (2011) 17(11): 3697-3705; Hsu et al., British Journal of Dermatology (2014) 170, 261-273). ADA formation is frequent for example in autoimmune patients treated with TNF blockers and impacts clinical outcome (Schaeverbecke et al., Rheumatology (2015) doi: 10.1093/rheumatology/kev277).

The development of ADAs may influence serum concentrations and function of therapeutic agents. The presence of ADAs may increase clearance of the therapeutic agent through formation of immune complexes between therapeutic agent and antibody (neutralizing, non-neutralizing or both), thus reducing the therapeutic agent's half-life. Furthermore, the activity and effectiveness of the therapeutic agent may be decreased through binding of antibody to the therapeutic agent. ADAs can also be associated with allergic or hypersensitivity reactions and other adverse events.

Since these adverse events associated with immune responses can influence the safety and efficacy profile of therapeutics, identification and development of strategies to overcome or inhibit ADAs is of great interest.

Several protein engineering approaches have been investigated to reduce the immunogenicity of protein therapeutics, including for example masking or alteration of protein B cell epitopes or modification of protein T cell epitopes. However, clinical safety and success of these approaches has not been tested and will require a significant degree of time to evaluate. Therefore, there exists an immediate need to develop new interventions using FDA-approved reagents to prevent ADA responses.

Chemotherapy-based approaches aimed at host immune suppression have been reported (Mossoba et al., Clin Cancer Res (2011) 17(11): 3697-3705).

The anti-CD20 antibody rituximab has been used in combination with methotrexate and intravenous immune globulin to achieve tolerance to enzyme replacement therapy in a Morbus Pompe patient (Mendelsohn et al., NEJM (2009) 360:2, 194-195). However, in a clinical trial, host pretreatment with rituximab did not inhibit the human immune response against the immunotoxin LMB-1 (Hassan et al., Clin Cancer Res (2004) 10, 16-18).

**Summary of the Invention**

The present invention is based on the surprising finding that the formation of ADAs in response to administration of an immunogenic therapeutic agent to a subject can effectively and sustainably be prevented by pre-treatment of said subject with a Type II anti-CD20 antibody, such as obinutuzumab.

Accordingly, in a first aspect the present invention provides a method for reducing the formation of anti-drug antibodies (ADAs) against a therapeutic agent in a subject, comprising administration of an anti-CD20 antibody to the subject prior to administration of the therapeutic agent. In one embodiment the period of time between the administration of the anti-CD20 antibody and administration of the therapeutic agent is sufficient for reduction of the number of B-cells in the subject in response to the administration of the CD20 antibody.

In a further aspect, the invention provides a method of treating a disease in a subject, the method comprising a treatment regimen comprising

(i) administration to the subject of a Type II anti-CD20 antibody,
and consecutively after a period of time
(ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

In one embodiment, the treatment regimen effectively reduces the formation of anti-drug antibodies (ADAs) in the subject in response to the administration of the therapeutic agent as compared to a corresponding treatment regimen without the administration of the anti-CD20 antibody.

In a further aspect, the invention provides a Type II anti-CD20 antibody for use in a method for reducing the formation of anti-drug antibodies (ADAs) against a therapeutic agent in a subject, comprising administration of the anti-CD20 antibody to the subject prior to administration of the therapeutic agent.

In one embodiment, the period of time between the administration of the anti-CD20 antibody and administration of the therapeutic agent is sufficient for reduction of the number of B-cells in the subject in response to the administration of the CD20 antibody.

In a further aspect, the invention provides a Type II anti-CD20 antibody for use in a method of treating a disease in a subject, the method comprising a treatment regimen comprising

(i) administration to the subject of the anti-CD20 antibody,
and consecutively after a period of time
(ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

In one embodiment, the treatment regimen effectively reduces the formation of anti-drug antibodies (ADAs) against the therapeutic agent in the subject (in response to the administration of the therapeutic agent) as compared to a corresponding treatment regimen without the administration of the anti-CD20 antibody.

In a further aspect, the invention provides the use of a Type II anti-CD20 antibody in the manufacture of a medicament for reduction of the formation of anti-drug antibodies (ADAs) against a therapeutic agent in a subject, wherein the medicament is to be used in a treatment regimen comprising

(i) administration to the subject of the anti-CD20 antibody,
and consecutively after a period of time
(ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

In one embodiment, the treatment regimen effectively reduces the formation of anti-drug antibodies (ADAs) against the therapeutic agent in the subject as compared to a corresponding treatment regimen without the administration of the anti-CD20 antibody.

In still a further aspect, the invention provides a kit for the reduction of the formation of anti-drug antibodies (ADAs) against a therapeutic agent in a subject, comprising a package comprising a Type II anti-CD20 antibody composition and instructions for using the anti-CD20 antibody composition in a treatment regimen comprising

(i) administration to the subject of the anti-CD20 antibody composition,
and consecutively after a period of time
(ii) administration to the subject of a therapeutic agent,

wherein the period of time between the administration of the anti-CD20 antibody composition and the administration of the therapeutic agent is sufficient for reduction of the number of B-cells in the subject in response to the administration of the CD20 antibody.

In one embodiment, the treatment regimen effectively reduces the formation of anti-drug antibodies (ADAs) against the therapeutic agent in the subject as compared to a corresponding treatment regimen without the administration of the anti-CD20 antibody composition. In one embodiment, the kit further comprises a therapeutic agent composition.

The invention in a further aspect as provides a therapeutic agent for use in a method of treating a disease in a subject, the method comprising a treatment regimen comprising

(i) administration to the subject of a Type II anti-CD20 antibody,
and consecutively after a period of time
(ii) administration to the subject of the therapeutic agent,

wherein the period of time between the administration of the anti-CD20 antibody and the administration of the therapeutic agent is sufficient for reduction of the number of B-cells in the subject in response to the administration of the CD20 antibody.

In one embodiment, the treatment regimen effectively reduces the formation of anti-drug antibodies (ADAs) in the subject in response to the administration of the therapeutic agent as compared to a corresponding treatment regimen without the administration of the anti-CD20 antibody.

The invention in still a further aspect provides the use of a therapeutic agent in the manufacture of a medicament for treatment of a disease in a subject, wherein the treatment comprises a treatment regimen comprising

> (i) administration to the subject of a Type II anti-CD20 antibody,
> and consecutively after a period of time
> (ii) administration to the subject of the therapeutic agent,

wherein the period of time between the administration of the anti-CD20 antibody and the administration of the therapeutic agent is sufficient for reduction of the number of B-cells in the subject in response to the administration of the CD20 antibody.

In one embodiment, the treatment regimen effectively reduces the formation of anti-drug antibodies (ADAs) in the subject in response to the administration of the therapeutic agent as compared to a corresponding treatment regimen without the administration of the anti-CD20 antibody.

The invention in a further aspect provides a kit for the treatment of a disease in a subject, comprising a package comprising a therapeutic agent composition and instructions for using the therapeutic agent composition in a treatment regimen comprising

> (i) administration to the subject of a Type II anti-CD20 antibody,
> and consecutively after a period of time
> (ii) administration to the subject of the therapeutic agent composition,

wherein the period of time between the administration of the anti-CD20 antibody and the administration of the therapeutic agent composition is sufficient for reduction of the number of B-cells in the subject in response to the administration of the CD20 antibody.

In one embodiment, the treatment regimen effectively reduces the formation of anti-drug antibodies (ADAs) against the therapeutic agent in the subject as compared to a corresponding treatment regimen without the administration of the anti-CD20 antibody composition. In one embodiment, the kit further comprises a Type II anti-CD20 antibody composition.

The methods, uses, Type II anti-CD20 antibodies, therapeutic agents and kits of the invention may incorporate, singly or in combination, any of the features described hereinbelow.

In one embodiment, the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

In a more specific embodiment, the anti-CD20 antibody comprises the heavy chain variable region sequence of SEQ ID NO: 10 and the light chain variable region sequence of SEQ ID NO: 11.

In one embodiment, the anti-CD20 antibody is an IgG antibody, particularly an IgG$_1$ antibody.

In one embodiment, the anti-CD20 antibody is engineered to have an increased proportion of non-fucosylated oligosaccharides in the Fc region as compared to a non-engineered antibody. In one embodiments, at least about 40% of the N-linked oligosaccharides in the Fc region of the anti-CD20 antibody are non-fucosylated.

In a particular embodiments the anti-CD20 antibody is obinutuzumab.

In one embodiment, the therapeutic agent comprises a polypeptide.

In one embodiment, the therapeutic agent comprises an antibody.

In one embodiment, the antibody specifically binds to carcinoembryonic antigen (CEA).

In one embodiment, the antibody comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19.

In one embodiment, the antibody comprises the heavy chain variable region sequence of SEQ ID NO: 20 and the light chain variable region sequence of SEQ ID NO: 21.

In one embodiment, the therapeutic agent comprises a cytokine.

In one embodiment, the cytokine is interleukin-2 (IL-2).

In one embodiment, the cytokine is a mutant human IL-2 polypeptide comprising the amino acid substitutions F42A, Y45A and L72G (numbering relative to the human IL-2 sequence SEQ ID NO: 12).

In one embodiment, the therapeutic agent comprises an immunoconjugate.

In one embodiment, the immunoconjugate comprises (a) an antibody that specifically binds to CEA and comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19, and (b) a mutant human IL-2 polypeptide comprising the amino acid substitutions F42A, Y45A and L72G (numbering relative to the human IL-2 sequence SEQ ID NO: 12).

In a particular embodiment, the therapeutic agent comprises CEA-IL2v.

**Brief Description of the Drawings**

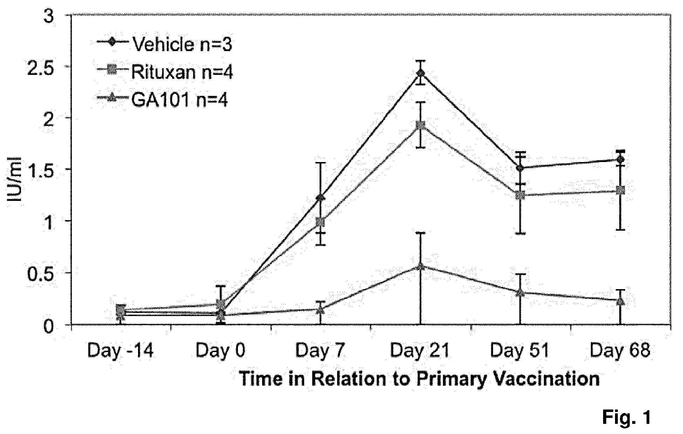

Figure 1. Prior treatment with obinutuzumab but not rituximab or vehicle results in the attenuation of tetanus toxoid specific de novo IgG antibody responses in cynomolgus monkeys. Rituxan indicates rituximab and GA101 obinutuzumab, respectively.

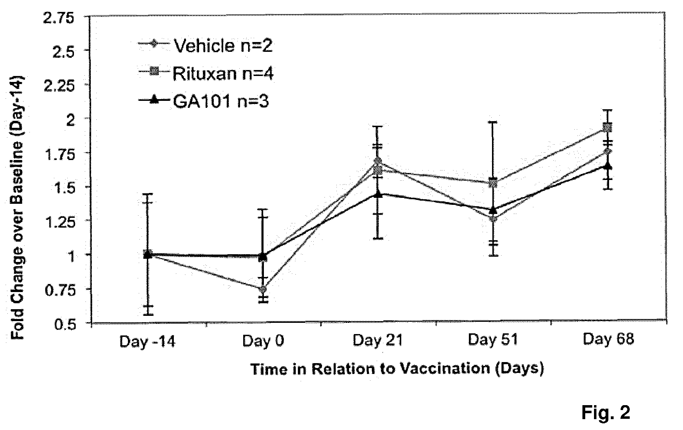

Figure 2. Memory recall responses by measles specific IgG antibody production in response to immune re-challenge with a measles/rubella booster vaccination in animals with baseline titers to measles is not affected by either obinutuzumab or rituximab in cynomolgus monkeys. Rituxan indicates rituximab and GA101 obinutuzumab, respectively.

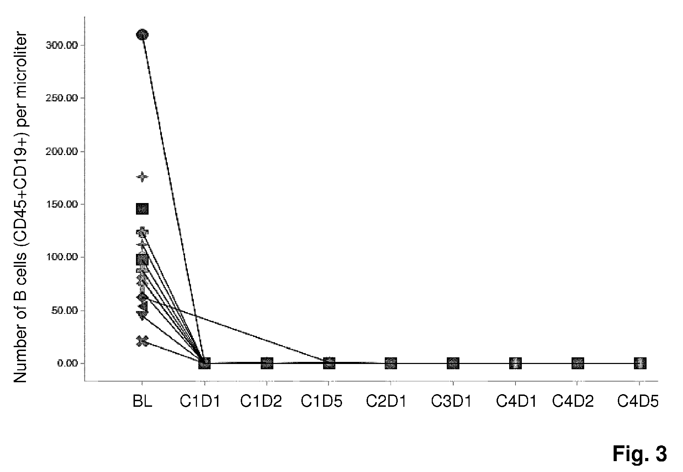

Figure 3. B cell counts (CD45+CD19+) in peripheral blood samples before start of obinutuzumab pre-treatment (BL = baseline), before start of treatment with RO6895882 (C1D1 = Cycle 1 Day 1) and during treatment with RO6895882. Lines/symbols represent individual patients. From the C1D1 time points onwards, no B cells were detectable in the peripheral blood samples.

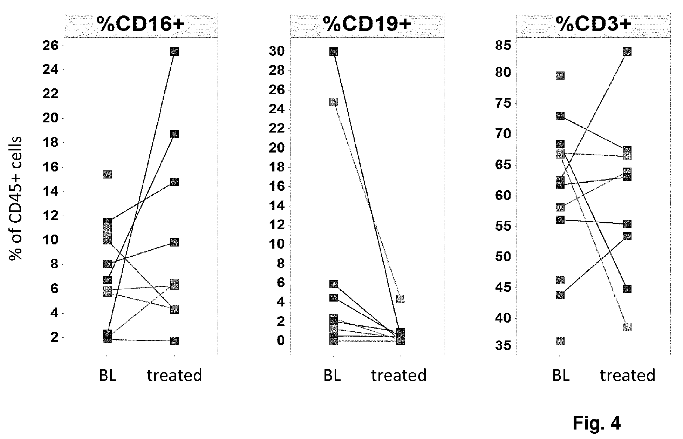

Figure 4. Reduction of CD19+ cells (B cells) detected by flow cytometry in tumor biopsies collected at baseline (BL) and after treatment with obinutuzumab (treated). On-treatment samples were obtained either before or during treatment with R06895882. The percentage of CD45+ cells (lymphocytes) staining positive for CD19 (B lymphocytes) was strongly reduced. No clear change was observed for the percentage of CD16+ cells (Natural Killer Cells) or CD3+ cells (T lymphocytes). Lines represent individual patients.

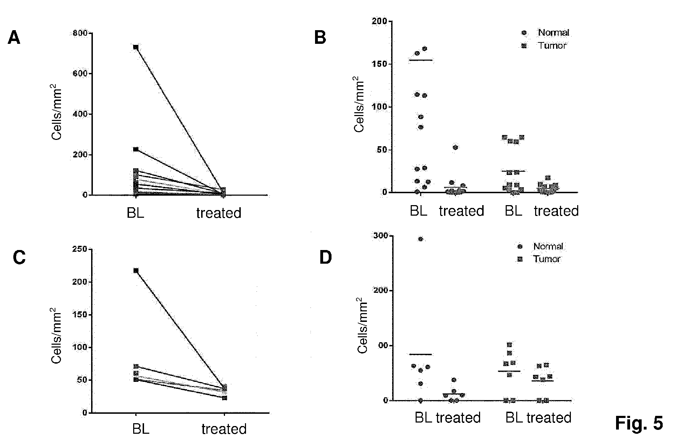

Figure 5. Reduction of B cells in tumor biopsies collected at baseline (BL) and after treatment (treated) with obinutuzumab measured by immunohistochemistry. On-treatment samples were obtained either before or during treatment with RO6895882. The density of B lymphocytes was measured by staining with CD20 (A, B) and PAX 5 (C, D). Both methods detected a depletion of B lymphocytes in tumor and surrounding normal tissue. Lines represent individual patients.

**Detailed Description of the Invention**

**Definitions**

Terms are used herein as generally used in the art, unless otherwise defined in the following.

CD20 (also known as B-lymphocyte antigen CD20, B-lymphocyte surface antigen B1, Leu-16, Bp35, BM5, and LF5; the human protein is characterized in UniProt database entry P11836) is a hydrophobic transmembrane protein with a molecular weight of approximately 35 kD expressed on pre-B and mature B lymphocytes (Valentine, M.A. et al., J. Biol. Chem. 264 (1989) 11282-11287; Tedder, T.F., et al., Proc. Natl. Acad. Sci. U.S.A. 85 (1988) 208-212; Stamenkovic, L, et al., J. Exp. Med. 167 (1988) 1975-1980; Einfeld, D.A., et al., EMBO J. 7 (1988) 711-717; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members.

Alternative splicing of this gene results in two transcript variants which encode the same protein.

The term "CD20" as used herein, refers to any native CD20 from any vertebrate source, including mammals such as primates (e.g. humans) and rodents (e.g., mice and rats), unless otherwise indicated. The term encompasses "full-length," unprocessed CD20 as well as any form of CD20 that results from processing in the cell. The term also encompasses naturally occurring variants of CD20, e.g., splice variants or allelic variants. The amino acid sequence of an exemplary human CD20 is shown in SEQ ID NO: 1.

The terms "anti-CD20 antibody" and "an antibody that binds to CD20" refer to an antibody that is capable of binding CD20 with sufficient affinity such that the antibody is useful as a diagnostic and/or therapeutic agent in targeting CD20. In one embodiment, the extent of binding of an anti-CD20 antibody to an unrelated, non-CD20 protein is less than about 10% of the binding of the antibody to CD20 as measured, e.g., by a radioimmunoassay (RIA). In certain embodiments, an antibody that binds to CD20 has a dissociation constant (Kd) of $\leq 1\mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In certain embodiments, an anti-CD20 antibody binds to an epitope of CD20 that is conserved among CD20 from different species.

By "Type II anti-CD20 antibody" is meant an anti-CD20 antibody having binding properties and biological activities of Type II anti-CD20 antibodies as described in Cragg et al., Blood 103 (2004) 2738-2743; Cragg et al., Blood 101 (2003) 1045-1052, Klein et al., mAbs 5 (2013),22-33, and summarized in Table 1 below.

**Table 1. Properties of type I and type II anti-CD20 antibodies**

| type I anti-CD20 antibodies | type II anti-CD20 antibodies |
|---|---|
| Bind class I CD20 epitope | Bind class II CD20 epitope |
| Localize CD20 to lipid rafts | Do not localize CD20 to lipid rafts |
| High CDC * | Low CDC * |
| ADCC activity * | ADCC activity * |
| Full binding capacity to B cells | Approx. half binding capacity to B cells |
| Weak homotypic aggregation | Homotypic aggregation |
| Low cell death induction | Strong cell death induction |
| *if IgG$_1$ isotype | |

Examples of type II anti-CD20 antibodies include e.g. obinutuzumab (GA101), tositumumab (B1), humanized B-Ly1 antibody IgG$_1$ (a chimeric humanized IgG1 antibody as disclosed in WO 2005/044859), 11B8 IgG1 (as disclosed in WO 2004/035607) and AT80 IgG1.

Examples of type I anti-CD20 antibodies include e.g. rituximab, ofatumumab, veltuzumab, ocaratuzumab, ocrelizumab, PRO131921, ublituximab, HI47 IgG3 (ECACC, hybridoma), 2C6 IgG1 (as disclosed in WO 2005/103081), 2F2 IgG1 (as disclosed in WO 2004/035607 and WO 2005/103081) and 2H7 IgG1 (as disclosed in WO 2004/056312).

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 1; variable region of the murine light chain (VL): SEQ ID NO: 2 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

As used herein, the term "cytokine" refers to a molecule that mediates and/or regulates a biological or cellular function or process (e.g. immunity, inflammation, and hematopoiesis). The term "cytokine" as used herein includes "lymphokines," "chemokines," "monokines," and "interleukins". Examples of useful cytokines include, but are not limited to, GM-CSF, IL-1$\alpha$, IL-1$\beta$, IL-2, IL-3, IL-4, IL-5, IL-6, IL-7, IL-8, IL-10, IL-12, IL-15, IFN-$\alpha$, IFN-$\beta$, IFN-$\gamma$, MIP-1$\alpha$, MIP-1$\beta$, TGF-$\beta$, TNF-$\alpha$, and TNF-$\beta$. A particular cytokines is IL-2. The term "cytokine" as used herein is meant to also include cytokine variants comprising one or more amino acid mutations in the amino acid sequences of the corresponding wild-type cytokine, such as for example the IL-2 variants described in Sauvé et al., Proc Natl Acad Sci USA 88, 4636-40 (1991); Hu et al., Blood 101, 4853-4861 (2003) and US Pat. Publ. No. 2003/0124678; Shanafelt et al., Nature Biotechnol 18, 1197-1202 (2000); Heaton et al., Cancer Res 53, 2597-602 (1993) and US Pat. No. 5,229,109; US Pat. Publ. No. 2007/0036752; WO 2008/0034473; WO 2009/061853; or in WO 2012/107417.

The term "interleukin-2" or "IL-2" as used herein, refers to any native IL-2 from any vertebrate source, including mammals such as primates (e.g. humans) and rodents (e.g., mice and rats), unless otherwise indicated. The term encompasses unprocessed IL-2 as well as any form of IL-2 that results from processing in the cell. The term also encompasses naturally occurring variants of IL-2, e.g. splice variants or allelic variants. The amino acid sequence of an exemplary human IL-2 is shown in SEQ ID NO: 12. Unprocessed human IL-2 additionally comprises an N-terminal 20 amino acid signal peptide having the sequence of SEQ ID NO: 31, which is absent in the mature IL-2 molecule. The term "interleukin-2" as used herein is meant to also include IL-2 variants comprising one or more amino acid mutations in the amino acid sequences of the corresponding wild-type cytokine, such as for example the IL-2 variants described in Sauvé et al., Proc Natl Acad Sci USA 88, 4636-40 (1991); Hu et al., Blood 101, 4853-4861 (2003) and US Pat. Publ. No. 2003/0124678; Shanafelt et al., Nature Biotechnol 18, 1197-1202 (2000); Heaton et al., Cancer Res 53, 2597-602 (1993) and US Pat. No. 5,229,109; US Pat. Publ. No. 2007/0036752; WO 2008/034473; WO 2009/061853; or in WO 2012/107417.

The term "IL-2 mutant" or "mutant IL-2 polypeptide" as used herein is intended to encompass any mutant forms of various forms of the IL-2 molecule including full-length IL-2, truncated forms of IL-2 and forms where IL-2 is linked to another molecule such as by fusion or chemical conjugation. "Full-length" when used in reference to IL-2 is intended to mean the mature, natural length IL-2 molecule. For example, full-length human IL-2 refers to a molecule that has 133 amino acids (see e.g. SEQ ID NO: 12). The various forms of IL-2 mutants are characterized in having a at least one amino acid mutation affecting the interaction of IL-2 with CD25. This mutation may involve substitution, deletion, truncation or modification of the wild-type amino acid residue normally located at that position. Mutants obtained by amino acid substitution are preferred. Unless otherwise indicated, an IL-2 mutant may be referred to herein as an IL-2 mutant peptide sequence, an IL-2 mutant polypeptide, IL-2 mutant protein or IL-2 mutant analog. Designation of various forms of IL-2 is herein made with respect to the sequence shown in SEQ ID NO: 12. Various designations may be used herein to indicate the same mutation. For example a mutation from phenylalanine at position 42 to alanine can be indicated as 42A, A42, $A_{42}$, F42A, or Phe42Ala.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to CD25. Amino acid sequence deletions and insertions include amino- and/or carboxy-terminal deletions and insertions of amino acids. Particular amino acid mutations are amino acid substitutions. For the purpose of altering e.g. the binding characteristics of an IL-2 polypeptide or an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc region to glycine can be indicated as 329G, G329, $G_{329}$, P329G, or Pro329Gly.

The term "CD25" or "α-subunit of the IL-2 receptor" as used herein, refers to any native CD25 from any vertebrate source, including mammals such as primates (e.g. humans) and rodents (e.g., mice and rats), unless otherwise indicated. The term encompasses "full-length", unprocessed CD25 as well as any form of CD25 that results from processing in the cell. The term also encompasses naturally occurring variants of CD25, e.g. splice variants or allelic variants. In certain embodiments CD25 is human CD25. The amino acid sequence of human CD25 is shown in UniProt (www.uniprot.org) accession no. P01589, or NCBI (www.ncbi.nlm.nih.gov/) RefSeq NP_000408.

The term "high-affinity IL-2 receptor" as used herein refers to the heterotrimeric form of the IL-2 receptor, consisting of the receptor γ-subunit (also known as common cytokine receptor γ-subunit, $γ_c$, or CD132), the receptor β-subunit (also known as CD122 or p70) and the receptor α-subunit (also known as CD25 or p55). The term "intermediate-affinity IL-2 receptor" by contrast refers to the IL-2 receptor including only the γ-subunit and the β-subunit, without the α-subunit (for a review see e.g. Olejniczak and Kasprzak, Med Sci Monit 14, RA179-189 (2008)).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., receptor and a ligand). The affinity of a molecule X for its partner Y can generally be represented by the dissociation constant ($K_D$), which is the ratio of dissociation and association rate constants ($k_{off}$ and $k_{on}$, respectively). Thus, equivalent affinities may comprise different rate constants, as long as the ratio of the rate constants remains the same. Affinity can be measured by well established methods known in the art. A particular method for measuring affinity is Surface Plasmon Resonance (SPR).

"Reduction" (and grammatical variations thereof such as "reduce" or "reducing"), for example reduction of the number of B cells or the formation of ADAs, refers to a decrease in the respective quantity, as measured by appropriate methods known in the art. For clarity the term includes also reduction to zero (or below the detection limit of the analytical method), i.e. complete abolishment or elimination. Conversely, "increased" refers to an increase in the respective quantity.

By "regulatory T cell" or "T$_{reg}$ cell" is meant a specialized type of CD4$^+$ T cell that can suppress the responses of other T cells. T$_{reg}$ cells are characterized by expression of the $\alpha$-subunit of the IL-2 receptor (CD25) and the transcription factor forkhead box P3 (FOXP3) (Sakaguchi, Annu Rev Immunol 22, 531-62 (2004)) and play a critical role in the induction and maintenance of peripheral self-tolerance to antigens, including those expressed by tumors. T$_{reg}$ cells require IL-2 for their function and development and induction of their suppressive characteristics.

As used herein, the term "antigen binding moiety" refers to a polypeptide molecule that specifically binds to an antigenic determinant. In one embodiment, an antigen binding moiety is able to direct the entity to which it is attached (e.g. a cytokine or a second antigen binding moiety) to a target site, for example to a specific type of tumor cell or tumor stroma bearing the antigenic determinant. Antigen binding moieties include antibodies and fragments thereof as further defined herein. Preferred antigen binding moieties include an antigen binding domain of an antibody, comprising an antibody heavy chain variable region and an antibody light chain variable region. In certain embodiments, the antigen binding moieties may include antibody constant regions as further defined herein and known in the art. Useful heavy chain constant regions include any of the five isotypes: $\alpha$, $\delta$, $\varepsilon$, $\gamma$, or $\mu$. Useful light chain constant regions include any of the two isotypes: $\kappa$ and $\lambda$.

By "specifically binds" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance technique (analyzed on a BIAcore instrument) (Liljeblad et al., Glyco J 17, 323-329 (2000)), and traditional binding assays (Heeley, Endocr Res 28, 217-229 (2002)).

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, free in blood serum, and/or in the extracellular matrix (ECM).

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity.

Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

As used herein, the term "effector moiety" refers to a polypeptide, e.g., a protein or glycoprotein, that influences cellular activity, for example, through signal transduction or other cellular pathways. Accordingly, the effector moiety can be associated with receptor-mediated signaling that transmits a signal from outside the cell membrane to modulate a response in a cell bearing one or more receptors for the effector moiety. In one embodiment, an effector moiety can elicit a cytotoxic response in cells bearing one or more receptors for the effector moiety. In another embodiment, an effector moiety can elicit a proliferative response in cells bearing one or more receptors for the effector moiety. In another embodiment, an effector moiety can elicit differentiation in cells bearing receptors for the effector moiety. In another embodiment, an effector moiety can alter expression (*i.e.* upregulate or downregulate) of an endogenous cellular protein in cells bearing receptors for the effector moiety. Non-limiting examples of effector moieties include cytokines, growth factors, hormones, enzymes, substrates, and cofactors. An effector moiety can be associated with an antigen binding moiety such as an antibody in a variety of configurations to form an immunoconjugate.

The term "cytotoxic agent" as used herein refers to a substance that inhibits or prevents a cellular function and/or causes cell death or destruction. Cytotoxic agents include, but are not limited to, radioactive isotopes (e.g., $At^{211}$, $I^{131}$, $I^{125}$, $Y^{90}$, $R^{186}$, $R^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "antibody" herein is used in the broadest sense and encompasses various antibody structures, including but not limited to monoclonal antibodies, polyclonal antibodies, multispecific antibodies (e.g. bispecific antibodies), and antibody fragments so long as they exhibit the desired antigen binding activity.

The terms "full length antibody," "intact antibody," and "whole antibody" are used herein interchangeably to refer to an antibody having a structure substantially similar to a native antibody structure or having heavy chains that contain an Fc region as defined herein.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$, diabodies, linear antibodies, single-chain antibody molecules (e.g. scFv), and multispecific antibodies formed from antibody fragments.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five classes, called $\alpha$ (IgA), $\delta$ (IgD), $\epsilon$ (IgE), $\gamma$ (IgG), or $\mu$ (IgM), some of which may be further divided into subclasses, e.g. $\gamma_1$ (IgGi), $\gamma_2$ (IgG$_2$), $\gamma_3$ (IgG$_3$), $\gamma_4$ (IgG$_4$), $\alpha_1$ (IgA$_1$) and $\alpha_2$ (IgA$_2$). The light chain of an immunoglobulin may be assigned to one of two types, called kappa ($\kappa$) and lambda ($\kappa$), based on the amino acid sequence of its constant domain. An immunoglobulin essentially consists of two Fab molecules and an Fc domain, linked via the immunoglobulin hinge region.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Preferably, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen binding specificity.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));

(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));

(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and

(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra*.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a "cleaved variant heavy chain"). This may be the case where the final two C-terminal amino acids of the heavy chain are glycine (G446) and lysine (K447, numbering according to Kabat EU index). Therefore, the C-terminal lysine (Lys447), or the C-terminal glycine (Gly446) and lysine (K447), of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991 (see also above). A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting heterodimerization" is a manipulation of the peptide backbone or the post-translational modifications of a polypeptide, e.g. an immunoglobulin heavy chain, that reduces or prevents the association of the polypeptide with an identical polypeptide to form a homodimer. A modification promoting heterodimerization as used herein particularly includes separate modifications made to each of two polypeptides desired to form a dimer, wherein the modifications are complementary to each other so as to promote association of the two polypeptides. For example, a modification promoting heterodimerization may alter the structure or charge of one or both of the polypeptides desired to form a dimer so as to make their association sterically or electrostatically favorable, respectively. Heterodimerization occurs between two non-identical polypeptides, such as two immunoglobulin heavy chains wherein further immunoconjugate components fused to each of the heavy chains (e.g. IL-2 polypeptide) are not the same. In the immunoconjugates of the present invention, the modification promoting heterodimerization is in the heavy chain(s), specifically in the Fc domain, of an immunoglobulin molecule. In some embodiments the modification promoting heterodimerziation comprises an amino acid mutation, specifically an amino acid substitution. In a particular embodiment, the modification promoting heterodimerization comprises a separate amino acid mutation, specifically an amino acid substitution, in each of the two immunoglobulin heavy chains.

An "activating Fc receptor" is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcαRI (CD89).

The term "effector functions" when used in reference to antibodies refer to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

As used herein, the term "effector cells" refers to a population of lymphocytes that display effector moiety receptors, e.g. cytokine receptors, and/or Fc receptors on their surface through which they bind an effector moiety, e.g. a cytokine, and/or an Fc region of an antibody and contribute to the destruction of target cells, e.g. tumor cells. Effector cells may for example mediate cytotoxic or phagocytic effects. Effector cells include, but are not limited to, effector T cells such as CD8$^+$cytotoxic T cells, CD4$^+$ helper T cells, γδ T cells, NK cells, lymphokine-activated killer (LAK) cells and macrophages/monocytes.

As used herein, the terms "engineer, engineered, engineering," are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches. "Engineering", particularly with the prefix "glyco-", as well as the term "glycosylation engineering" includes metabolic engineering of the glycosylation machinery of a cell, including genetic manipulations of the oligosaccharide synthesis pathways to achieve altered glycosylation of glycoproteins expressed in cells. Furthermore, glycosylation engineering includes the effects of mutations and cell environment on glycosylation. In one embodiment, the glycosylation engineering is an alteration in glycosyltransferase activity. In a particular embodiment, the engineering results in altered glucosaminyltransferase activity and/or fucosyltransferase activity. Glycosylation engineering can be used to obtain a "host cell having increased GnTIII activity" (e.g. a host cell that has been manipulated to express increased levels of one or more polypeptides having β(1,4)-N-acetylglucosaminyltransferase III (GnTIII) activity), a "host cell having increased ManII activity" (e.g. a host cell that has been manipulated to express increased levels of one or more polypeptides having α-mannosidase II (ManII) activity), or a "host cell having decreased α(1,6) fucosyltransferase activity" (e.g. a host cell that has been manipulated to express decreased levels of α(1,6) fucosyltransferase).

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate proteins used for the present invention. In one embodiment, the host cell is engineered to allow the production of an antibody with modified oligosaccharides. In certain embodiments, the host cells have been manipulated to express increased levels of one or more polypeptides having $\beta(1,4)$-N-acetylglucosaminyltransferase III (GnTIII) activity. In certain embodiments the host cells have been further manipulated to express increased levels of one or more polypeptides having $\alpha$-mannosidase II (ManII) activity. Host cells include cultured cells, e.g. mammalian cultured cells, such as CHO cells, BHK cells, NSO cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

As used herein, the term "polypeptide having GnTIII activity" refers to polypeptides that are able to catalyze the addition of a N-acetylglucosamine (GlcNAc) residue in $\beta$-1,4 linkage to the $\beta$-linked mannoside of the trimannosyl core of N-linked oligosaccharides. This includes fusion polypeptides exhibiting enzymatic activity similar to, but not necessarily identical to, an activity of $\beta(1,4)$-N-acetylglucosaminyltransferase III, also known as $\beta$-1,4-mannosyl-glyco-protein 4-beta-N-acetylglucosaminyl-transferase (EC 2.4.1.144), according to the Nomenclature Committee of the International Union of Biochemistry and Molecular Biology (NC-IUBMB), as measured in a particular biological assay, with or without dose dependency. In the case where dose dependency does exist, it need not be identical to that of GnTIII, but rather substantially similar to the dose-dependency in a given activity as compared to the GnTIII (i.e. the candidate polypeptide will exhibit greater activity or not more than about 25-fold less and, preferably, not more than about ten-fold less activity, and most preferably, not more than about three-fold less activity relative to the GnTIII). In certain embodiments the polypeptide having GnTIII activity is a fusion polypeptide comprising the catalytic domain of GnTIII and the Golgi localization domain of a heterologous Golgi resident polypeptide. Particularly, the Golgi localization domain is the localization domain of mannosidase II or GnTI, most particularly the localization domain of mannosidase II. Alternatively, the Golgi localization domain is selected from the group consisting of: the localization domain of mannosidase I, the localization domain of GnTII, and the localization domain of $\alpha$1,6 core fucosyltransferase. Methods for generating such fusion polypeptides and using them to produce antibodies with increased effector functions are disclosed in WO2004/065540, U.S. Provisional Pat. Appl. No. 60/495,142 and U.S. Pat. Appl. Publ. No. 2004/0241817, the entire contents of which are expressly incorporated herein by reference.

As used herein, the term "Golgi localization domain" refers to the amino acid sequence of a Golgi resident polypeptide which is responsible for anchoring the polypeptide to a location within the Golgi complex. Generally, localization domains comprise amino terminal "tails" of an enzyme.

As used herein, the term "polypeptide having ManII activity" refers to polypeptides that are able to catalyze the hydrolysis of the terminal 1,3- and 1,6-linked $\alpha$-D-mannose residues in the branched $GlcNAcMan_5GlcNAc_2$ mannose intermediate of N-linked oligosaccharides. This includes polypeptides exhibiting enzymatic activity similar to, but not necessarily identical to, an activity of Golgi $\alpha$-mannosidase II, also known as mannosyl oligosaccharide 1,3-1,6-$\alpha$-mannosidase II (EC 3.2.1.114), according to the Nomenclature Committee of the International Union of Biochemistry and Molecular Biology (NC-IUBMB).

Antibody-dependent cell-mediated cytotoxicity (ADCC) is an immune mechanism leading to the lysis of antibody-coated target cells by immune effector cells. The target cells are cells to which antibodies or fragments thereof comprising an Fc region specifically bind, generally via the protein part that is N-terminal to the Fc region. As used herein, the term "increased/reduced ADCC" is defined as either an increase/reduction in the number of target cells that are lysed in a given time, at a given concentration of antibody in the medium surrounding the target cells, by the mechanism of ADCC defined above, and/or a reduction/increase in the concentration of antibody, in the medium surrounding the target cells, required to achieve the lysis of a given number of target cells in a given time, by the mechanism of ADCC. The increase/reduction in ADCC is relative to the ADCC mediated by the same antibody produced by the same type of host cells, using the same standard production, purification, formulation and storage methods (which are known to those skilled in the art), but that has not been engineered. For example the increase in ADCC mediated by an antibody produced by host cells engineered to have an altered pattern of glycosylation (e.g. to express the glycosyltransferase, GnTIII, or other glycosyltransferases) by the methods described herein, is relative to the ADCC mediated by the same antibody produced by the same type of non-engineered host cells.

By "antibody having increased/reduced antibody dependent cell-mediated cytotoxicity (ADCC)" is meant an antibody having increased/reduced ADCC as determined by any suitable method known to those of ordinary skill in the art. One accepted *in vitro* ADCC assay is as follows:

1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:

i) the PBMCs are isolated using standard density centrifugation procedures and are suspended at $5 \times 10^6$

cells/ml in RPMI cell culture medium;

ii) the target cells are grown by standard tissue culture methods, harvested from the exponential growth phase with a viability higher than 90%, washed in RPMI cell culture medium, labeled with 100 micro-Curies of $^{51}$Cr, washed twice with cell culture medium, and resuspended in cell culture medium at a density of $10^5$ cells/ml;

iii) 100 microliters of the final target cell suspension above are transferred to each well of a 96-well microtiter plate;

iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;

v) for the maximum release (MR) controls, 3 additional wells in the plate containing the labeled target cells, receive 50 microliters of a 2% (V/V) aqueous solution of non-ionic detergent (Nonidet, Sigma, St. Louis), instead of the antibody solution (point iv above);

vi) for the spontaneous release (SR) controls, 3 additional wells in the plate containing the labeled target cells, receive 50 microliters of RPMI cell culture medium instead of the antibody solution (point iv above);

vii) the 96-well microtiter plate is then centrifuged at 50 x g for 1 minute and incubated for 1 hour at 4°C;

viii) 50 microliters of the PBMC suspension (point i above) are added to each well to yield an effector:target cell ratio of 25:1 and the plates are placed in an incubator under 5% $CO_2$ atmosphere at 37°C for 4 hours;

ix) the cell-free supernatant from each well is harvested and the experimentally released radioactivity (ER) is quantified using a gamma counter;

x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point v above), and SR is the average radioactivity quantified (see point ix above) for the SR controls (see point vi above);

4) "increased/reduced ADCC" is defined as either an increase/reduction in the maximum percentage of specific lysis observed within the antibody concentration range tested above, and/or a reduction/increase in the concentration of antibody required to achieve one half of the maximum percentage of specific lysis observed within the antibody concentration range tested above. The increase/reduction in ADCC is relative to the ADCC, measured with the above assay, mediated by the same antibody, produced by the same type of host cells, using the same standard production, purification, formulation and storage methods, which are known to those skilled in the art, but that has not been engineered.

As used herein, the term "immunoconjugate" refers to a polypeptide molecule that includes at least one effector moiety, such as a cytokine, and an antigen binding moiety, such as an antibody. In certain embodiments, the immunoconjugate comprises not more than one effector moiety. Particular immunoconjugates useful in the invention essentially consist of one effector moiety and an antibody joined by one or more peptide linkers. Particular immunoconjugates according to the invention are fusion proteins, i.e. the components of the immunconjugate are joined by peptide bonds.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

As used herein, the terms "first", "second", "third" etc. with respect to antigen binding moieties etc., are used for convenience of distinguishing when there is more than one of each type of moiety. Use of these terms is not intended to confer a specific order or orientation unless explicitly so stated.

The term "bispecific" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

An "activating T cell antigen" as used herein refers to an antigenic determinant expressed on the surface of a T lymphocyte, particularly a cytotoxic T lymphocyte, which is capable of inducing T cell activation upon interaction with an antigen binding molecule. Specifically, interaction of an antigen binding molecule with an activating T cell antigen may induce T cell activation by triggering the signaling cascade of the T cell receptor complex.

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. The T cell activating bispecific antigen binding molecules of the invention are capable of inducing T cell activation. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma.

A "Fab molecule" refers to a protein consisting of the VH and CH1 domain of the heavy chain (the "Fab heavy chain") and the VL and CL domain of the light chain (the "Fab light chain") of an immunoglobulin.

By "fused" is meant that the components (e.g. a Fab molecule and an Fc domain subunit) are linked by peptide bonds, either directly or via one or more peptide linkers.

An "anti-drug antibody" or "ADA" refers to an antibody that binds to a therapeutic agent and may influence serum concentrations and function of the therapeutic agent in a subject. The presence of ADAs may increase clearance of the therapeutic agent through formation of immune complexes between therapeutic agent and antibody (neutralizing, non-neutralizing or both), thus reducing the therapeutic agent's half-life. Furthermore, the activity and effectiveness of the therapeutic agent may be decreased through binding of antibody to the therapeutic agent (particularly in the case of neutralizing ADAs). ADAs can also be associated with allergic or hypersensitivity reactions and other adverse events.

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

By "therapeutic agent" is meant an active ingredient, e.g. of a pharmaceutical composition, that is administered to a subject in an attempt to alter the natural course of a disease in the subject being treated, and can be performed either for prophylaxis or during the course of clinical pathology. An "immunotherapeutic agent" refers to a therapeutic agent that is administered to a subject in an attempt to restore or enhance the subject's immune response, e.g. to a tumor.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Preferably, the individual or subject is a human.

The term "pharmaceutical composition" refers to a preparation which is in such form as to permit the biological activity of an active ingredient contained therein to be effective, and which contains no additional components which are unacceptably toxic to a subject to which the composition would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, methods of the invention are used to delay development of a disease or to slow the progression of a disease.

The term "package insert" is used to refer to instructions customarily included in commercial packages of therapeutic products, that contain information about the indications, usage, dosage, administration, combination therapy, contraindications and/or warnings concerning the use of such therapeutic products.

"CD3" refers to any native CD3 from any vertebrate source, including mammals such as primates (e.g. humans), non-human primates (e.g. cynomolgus monkeys) and rodents (e.g. mice and rats), unless otherwise indicated. The term encompasses "full-length," unprocessed CD3 as well as any form of CD3 that results from processing in the cell. The term also encompasses naturally occurring variants of CD3, e.g., splice variants or allelic variants. In one embodiment, CD3 is human CD3, particularly the epsilon subunit of human CD3 (CD3ε). The amino acid sequence of human CD3ε is shown in UniProt (www.uniprot.org) accession no. P07766 (version 144), or NCBI (www.ncbi.nlm.nih.gov/) RefSeq NP_000724.1. The amino acid sequence of cynomolgus [Macaca fascicularis] CD3ε is shown in NCBI GenBank no. BAB71849.1.

"Carcinoembryonic antigen" or "CEA" (also known as Carcinoembryonic antigen-related cell adhesion molecule 5 (CEACAM5)) refers to any native CEA from any vertebrate source, including mammals such as primates (e.g. humans), non-human primates (e.g. cynomolgus monkeys) and rodents (e.g. mice and rats), unless otherwise indicated. The term encompasses "full-length," unprocessed CEA as well as any form of CEA that results from processing in the cell. The term also encompasses naturally occurring variants of CEA, e.g., splice variants or allelic variants. In one embodiment, CEA is human CEA. The amino acid sequence of human CEA is shown in UniProt (www.uniprot.org) accession no. P06731, or NCBI (www.ncbi.nlm.nih.gov/) RefSeq NP_004354.2.

"Fibroblast activation protein" or "FAP" (also known as seprase) refers to any native FAP from any vertebrate source, including mammals such as primates (e.g. humans), non-human primates (e.g. cynomolgus monkeys) and rodents (e.g. mice and rats), unless otherwise indicated. The term encompasses "full-length," unprocessed FAP as well as any form of FAP that results from processing in the cell. The term also encompasses naturally occurring variants of FAP, e.g., splice variants or allelic variants. In one embodiment, FAP is human FAP. The amino acid sequence of human FAP is shown in UniProt (www.uniprot.org) accession no. Q12884, or NCBI (www.ncbi.nlm.nih.gov/) RefSeq NP_004451.2.

By a "crossover" Fab molecule (also termed "Crossfab") is meant a Fab molecule wherein the variable domains or the constant domains of the Fab heavy and light chain are exchanged (i.e. replaced by each other), i.e. the crossover Fab molecule comprises a peptide chain composed of the light chain variable domain VL and the heavy chain constant domain 1 CH1 (VL-CH1, in N- to C-terminal direction), and a peptide chain composed of the heavy chain variable domain VH and the light chain constant domain CL (VH-CL, in N- to C-terminal direction). For clarity, in a crossover Fab molecule wherein the variable domains of the Fab light chain and the Fab heavy chain are exchanged, the peptide chain comprising the heavy chain constant domain 1 CH1 is referred to herein as the "heavy chain" of the (crossover) Fab molecule. Conversely, in a crossover Fab molecule wherein the constant domains of the Fab light chain and the Fab heavy chain are exchanged, the peptide chain comprising the heavy chain variable domain VH is referred to herein as the "heavy chain" of the (crossover) Fab molecule.

In contrast thereto, by a "conventional" Fab molecule is meant a Fab molecule in its natural format, i.e. comprising a heavy chain composed of the heavy chain variable and constant domains (VH-CH1, in N- to C-terminal direction), and a light chain composed of the light chain variable and constant domains (VL-CL, in N- to C-terminal direction).

**Anti-CD20 antibodies**

The CD20 molecule (also called human B-lymphocyte-restricted differentiation antigen or Bp35) is a hydrophobic transmembrane protein expressed on the surface of malignant and non-malignant pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-212; Stamenkovic, I., et al., J. Exp. Med. 167 (1988) 1975-1980; Tedder, T.F., et al., J. Immunol. 142 (1989) 2560-2568). CD20 is highly expressed by over 90% of B cell non-Hodgkin's lymphomas (NHL) (Anderson, K.C., et al., Blood 63 (1984) 1424-1433) but is not found on hematopoietic stem cells, pro-B cells, normal plasma cells, or other normal tissues (Tedder, T.F., et al., J, Immunol. 135 (1985) 973- 979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I anti-CD20 antibodies primarily utilize complement to kill target cells, while Type II antibodies primarily operate through direct induction of cell death.

Type I and Type II anti-CD20 antibodies and their characteristics are reviewed e.g. in Klein et al., mAbs 5 (2013), 22-33. Type II anti-CD20 antibodies do not localize CD20 to lipid rafts, show low CDC activity, show only about half the binding capacity to B cells as compared to Type I anti-CD20 antibodies, and induce homotypic aggregation and direct cell death. In constrast thereto, Type I antibodies localize CD20 to lipid rafts, show high CDC activity, full binding capacity to B cells, and only weak induction of homotypic aggregation and direct cell death.

Obinutuzumab and tositumomab (CAS number 192391-48) are examples of Type II anti-CD20 antibodies, while rituximab, ofatumumab, veltuzumab, ocaratuzumab, ocrelizumab, PRO131921 and ublituximab are examples of Type I anti-CD20 antibodies.

In one embodiment according to the present invention, the anti-CD20 antibody is capable of reducing the number of B cells in a subject. In one embodiment the anti-CD20 antibody is a Type II anti-CD20 antibody. In one embodiment, the anti-CD20 antibody is an IgG antibody, particularly an IgG1 antibody. In one embodiment, the anti-CD20 antibody is a full-length antibody. In one embodiment, the anti-CD20 antibody comprises an Fc region, particularly an IgG Fc region or, more particularly, an IgG1 Fc region. In one embodiment the anti-CD20 antibody is a humanized B-Ly1 antibody. Particularly, the anti-CD20 antibody is a humanized, IgG-class Type II anti-CD20 antibody, having the binding specificity of the murine B-Ly1 antibody (Poppema and Visser, Biotest Bulletin 3, 131-139 (1987); SEQ ID NOs 2 and 3).

In one embodiment, the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9. Particularly, the heavy chain variable region framework regions (FRs) FR1, FR2, and FR3 of said anti-CD20 antibody are human FR sequences encoded by the VH1_10 human germ-line sequence, the heavy chain variable region FR4 of said anti-CD20 antibody is a human FR sequence encoded by the JH4 human germ-line sequence, the light chain variable region FRs FR1, FR2, and FR3 of said anti-CD20 antibody are human FR sequences encoded by the VK_2_40 human germ-line sequence, and the light chain variable region FR4 of said anti-CD20 antibody is a human FR sequence encoded by the JK4 human germ-line sequence. In one embodiment, the anti-CD20 antibody comprises the heavy chain variable region sequence of SEQ ID NO: 10 and the light chain variable region sequence of SEQ ID NO: 11. In a particular embodiment, the anti-CD20 antibody is obinutuzumab (recommended INN, WHO Drug Information, Vol. 26, No. 4, 2012, p. 453). As used herein, obinutuzumab is synonymous for GA101. The tradename is GAZYVA® or GAZYVARO®. This replaces all previous versions (e.g. Vol. 25, No. 1, 2011, p.75-76), and is formerly known as afutuzumab (recommended INN, WHO Drug Information, Vol. 23, No. 2, 2009, p. 176; Vol. 22, No. 2, 2008, p. 124). In one embodiment, the anti-CD20 antibody is tositumomab.

The anti-CD20 antibody useful in the present invention may be engineered to have increased effector function, as compared to a corresponding non-engineered antibody. In one embodiment the antibody engineered to have increased effector function has at least 2-fold, at least 10-fold or even at least 100-fold increased effector function, compared to a corresponding non-engineered antibody. The increased effector function can include, but is not limited to, one or more of the following: increased Fc receptor binding, increased C1q binding and complement dependent cytotoxicity (CDC), increased antibody-dependent cell-mediated cytotoxicity (ADCC), increased antibody-dependent cellular phagocytosis (ADCP), increased cytokine secretion, increased immune complex-mediated antigen uptake by antigen-presenting cells, increased binding to NK cells, increased binding to macrophages, increased binding to monocytes, increased binding to polymorphonuclear cells, increased direct signaling inducing apoptosis, increased crosslinking of target-bound antibodies, increased dendritic cell maturation, or increased T cell priming.

In one embodiment the increased effector function one or more selected from the group of increased Fc receptor binding, increased CDC, increased ADCC, increased ADCP, and increased cytokine secretion. In one embodiment the increased effector function is increased binding to an activating Fc receptor. In one such embodiment the binding affinity to the activating Fc receptor is increased at least 2-fold, particularly at least 10-fold, compared to the binding affinity of a corresponding non-engineered antibody. In a specific embodiment the activating Fc receptor is selected from the group of FcγRIIIa, FcγRI, and FcγRIIa. In one embodiment the activating Fc receptor is FcγRIIIa, particularly human FcγRIIIa. In another embodiment the increased effector function is increased ADCC. In one such embodiment the ADCC is increased at least 10-fold, particularly at least 100-fold, compared to the ADCC mediated by a corresponding non-engineered antibody. In yet another embodiment the increased effector function is increased binding to an activating Fc receptor and increased ADCC.

Increased effector function can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g. in a animal model such as that disclosed in Clynes et al., Proc Natl Acad Sci USA 95, 652-656 (1998). Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. According to a particular embodiment, binding affinity to an activating Fc receptor is measured by surface plasmon resonance using a BIACORE® T100 machine (GE Healthcare) at 25°C. Alternatively, binding affinity of antibodies for Fc receptors may be evaluated using cell lines known to express particular Fc receptors, such as NK cells expressing FcγIIIa receptor. C1q binding assays may also be carried out to determine whether the antibody is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

Increased effector function may result e.g. from glycoengineering of the Fc region or the introduction of amino acid mutations in the Fc region of the antibody. In one embodiment the anti-CD20 antibody is engineered by introduction of one or more amino acid mutations in the Fc region. In a specific embodiment the amino acid mutations are amino acid substitutions. In an even more specific embodiment the amino acid substitutions are at positions 298, 333, and/or 334 of the Fc region (EU numbering of residues). Further suitable amino acid mutations are described e.g. in Shields et al., J Biol Chem 9(2), 6591-6604 (2001); U.S. Patent No. 6,737,056; WO 2004/063351 and WO 2004/099249. Mutant Fc regions can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

In another embodiment the anti-CD20 antibody is engineered by modification of the glycosylation in the Fc region. In a specific embodiment the anti-CD20 antibody is engineered to have an increased proportion of non-fucosylated oligosaccharides in the Fc region as compared to a non-engineered antibody. An increased proportion of non-fucosylated oligosaccharides in the Fc region of an antibody results in the antibody having increased effector function, in particular increased ADCC.

In a more specific embodiment, at least about 20%, about 25%, about 30%, about 35%, about 40%, about 45%, about 50%, about 55%, about 60%, about 65%, about 70%, about 75%, about 80%, about 85%, about 90%, about 95%, or about 100%, preferably at least about 40%, of the N-linked oligosaccharides in the Fc region of the anti-CD20 antibody are non-fucosylated. In one embodiment, between about 40% and about 80% of the N-linked oligosaccharides in the Fc region of the anti-CD20 antibody are non-fucosylated. In one embodiment, between about 40% and about 60% of the N-linked oligosaccharides in the Fc region of the anti-CD20 antibody are non-fucosylated. The non-fucosylated oligosaccharides may be of the hybrid or complex type.

In another specific embodiment the anti-CD20 antibody is engineered to have an increased proportion of bisected oligosaccharides in the Fc region as compared to a non-engineered antibody. In a more specific embodiment, at least about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, preferably at least about 40%, of the N-linked oligosaccharides in the Fc region of the anti-CD20 antibody are bisected. In one embodiment, between about 40% and about 80% of the N-linked oligosaccharides in the Fc region of the anti-CD20 antibody are bisected. In one embodiment, between about 40% and about 60% of the N-linked oligosaccharides in the Fc region of the anti-CD20 antibody are bisected. The bisected oligosaccharides may be of the hybrid or complex type.

In yet another specific embodiment the anti-CD20 antibody is engineered to have an increased proportion of bisected, non-fucosylated oligosaccharides in the Fc region, as compared to a non-engineered antibody. In a more specific embodiment, at least about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, preferably at least about 15%, more preferably at least about 25%, of the N-linked oligosaccharides in the Fc region of the anti-CD20 antibody are bisected, non-fucosylated. The bisected, non-fucosylated oligosaccharides may be of the hybrid or complex type.

The oligosaccharide structures in the antibody Fc region can be analysed by methods well known in the art, e.g. by MALDI TOF mass spectrometry as described in Umana et al., Nat Biotechnol 17, 176-180 (1999) or Ferrara et al., Biotechn Bioeng 93, 851-861 (2006). The percentage of non-fucosylated oligosaccharides is the amount of oligosaccharides lacking fucose residues, relative to all oligosaccharides attached to Asn 297 (e. g. complex, hybrid and high mannose structures) and identified in an N-glycosidase F treated sample by MALDI TOF MS. Asn 297 refers to the asparagine residue located at about position 297 in the Fc region (EU numbering of Fc region residues); however, Asn297 may also be located about $\pm$ 3 amino acids upstream or downstream of position 297, i.e., between positions 294 and 300, due to minor sequence variations in antibodies. The percentage of bisected, or bisected non-fucosylated, oligosaccharides is determined analogously.

In one embodiment the anti-CD20 antibody is engineered to have modified glycosylation in the Fc region, as compared to a non-engineered antibody, by producing the antibody in a host cell having altered activity of one or more glycosyltransferase. Glycosyltransferases include $\beta(1,4)$-N-acetylglucosaminyltransferase III (GnTIII), $\beta(1,4)$-galactosyltransferase (GalT), $\beta(1,2)$-N-acetylglucosaminyltransferase I (GnTI), $\beta(1,2)$-N-acetylglucosaminyltransferase II (GnTII) and $\alpha(1,6)$-fucosyltransferase. In a specific embodiment the anti-CD20 antibody is engineered to have an increased proportion of non-fucosylated oligosaccharides in the Fc region, as compared to a non-engineered antibody, by producing the antibody in a host cell having increased $\beta(1,4)$-N-acetylglucosaminyltransferase III (GnTIII) activity. In an even more specific embodiment the host cell additionally has increased $\alpha$-mannosidase II (ManII) activity. The glycoengineering methodology that can be used for engineering antibodies useful for the present invention has been described in greater detail in Umana et al., Nat Biotechnol 17, 176-180 (1999); Ferrara et al., Biotechn Bioeng 93, 851-861 (2006); WO 99/54342 (U.S. Pat. No. 6,602,684; EP 1071700); WO 2004/065540 (U.S. Pat. Appl. Publ. No. 2004/0241817; EP 1587921), WO 03/011878 (U.S. Pat. Appl. Publ. No. 2003/0175884), the entire content of each of which is incorporated herein by reference in its entirety. Antibodies glycoengineered using this methodology are referred to as GlycoMabs herein.

Generally, any type of cultured cell line, including the cell lines discussed herein, can be used to generate cell lines for the production of anti-TNC A2 antibodies with altered glycosylation pattern. Particular cell lines include CHO cells, BHK cells, NS0 cells, SP2/0 cells, YO myeloma cells, P3X63 mouse myeloma cells, PER cells, PER.C6 cells or hybridoma cells, and other mammalian cells. In certain embodiments, the host cells have been manipulated to express increased levels of one or more polypeptides having $\beta$(1,4)-N-acetylglucosaminyltransferase III (GnTIII) activity. In certain embodiments the host cells have been further manipulated to express increased levels of one or more polypeptides having $\alpha$-mannosidase II (ManII) activity. In a specific embodiment, the polypeptide having GnTIII activity is a fusion polypeptide comprising the catalytic domain of GnTIII and the Golgi localization domain of a heterologous Golgi resident polypeptide. Particularly, said Golgi localization domain is the Golgi localization domain of mannosidase II. Methods for generating such fusion polypeptides and using them to produce antibodies with increased effector functions are disclosed in Ferrara et al., Biotechn Bioeng 93, 851-861 (2006) and WO2004/065540, the entire contents of which are expressly incorporated herein by reference.

The host cells which contain the coding sequence of an antibody useful for the invention and/or the coding sequence of polypeptides having glycosyltransferase activity, and which express the biologically active gene products may be identified e.g. by DNA-DNA or DNA-RNA hybridization; the presence or absence of "marker" gene functions; assessing the level of transcription as measured by the expression of the respective mRNA transcripts in the host cell; or detection of the gene product as measured by immunoassay or by its biological activity - methods which are well known in the art. GnTIII or Man II activity can be detected e.g. by employing a lectin which binds to biosynthetis products of GnTIII or ManII, respectively. An example for such a lectin is the $E_4$-PHA lectin which binds preferentially to oligosaccharides containing bisecting GlcNAc. Biosynthesis products (i.e. specific oligosaccharide structures) of polypeptides having GnTIII or ManII activity can also be detected by mass spectrometric analysis of oligosaccharides released from glycoproteins produced by cells expressing said polypeptides. Alternatively, a functional assay which measures the increased effector function, e.g. increased Fc receptor binding, mediated by antibodies produced by the cells engineered with the polypeptide having GnTIII or ManII activity may be used.

In another embodiment the anti-CD20 antibody is engineered to have an increased proportion of non-fucosylated oligosaccharides in the Fc region, as compared to a non-engineered antibody, by producing the antibody in a host cell having decreased $\alpha$(1,6)-fucosyltransferase activity. A host cell having decreased $\alpha$(1,6)-fucosyltransferase activity may be a cell in which the $\alpha$(1,6)-fucosyltransferase gene has been disrupted or otherwise deactivated, e.g. knocked out (see Yamane-Ohnuki et al., Biotech Bioeng 87, 614 (2004); Kanda et al., Biotechnol Bioeng, 94(4), 680-688 (2006); Niwa et al., J Immunol Methods 306, 151-160 (2006)).

Other examples of cell lines capable of producing defucosylated antibodies include Lec13 CHO cells deficient in protein fucosylation (Ripka et al., Arch Biochem Biophys 249, 533-545 (1986); US Pat. Appl. No. US 2003/0157108; and WO 2004/056312, especially at Example 11). The antibodies useful in the present invention can alternatively be glycoengineered to have reduced fucose residues in the Fc region according to the techniques disclosed in EP 1 176 195 A1, WO 03/084570, WO 03/085119 and U.S. Pat. Appl. Pub. Nos. 2003/0115614, 2004/093621, 2004/110282, 2004/110704, 2004/132140, US Pat. No. 6,946,292 (Kyowa), e.g. by reducing or abolishing the activity of a GDP-fucose transporter protein in the host cells used for antibody production.

Glycoengineered antibodies useful in the invention may also be produced in expression systems that produce modified glycoproteins, such as those taught in WO 03/056914 (GlycoFi, Inc.) or in WO 2004/057002 and WO 2004/024927 (Greenovation).

**Therapeutic agents**

The present invention is useful in connection with various therapeutic agents, particularly with therapeutic agents that are immunogenic in the subject, i.e. have the ability of inducing an immune response in the subject. Such therapeutic agents include, for example, recombinant proteins.

In one embodiment, the therapeutic agent induces the formation of ADAs in a subject when administered to the subject in a treatment regimen without the administration of an anti-CD20 antibody.
In one embodiment, the therapeutic agent is a biologic agent. In one embodiment, the therapeutic agent comprises a polypeptide that does not naturally occur in the subject and/or is immunogenic in the subject. In one embodiment, the therapeutic agent comprises a polypeptide, particularly a recombinant polypeptide.
In one embodiment, the therapeutic agent comprises a polypeptide selected from the group of an antibody, an antibody fragment, an Fc domain, and an immunoconjugate. In one embodiment, the therapeutic agent comprises an antibody. In one embodiment, the antibody is a monoclonal antibody. In one embodiment, the antibody is a polyclonal antibody. In one embodiment the antibody is a human antibody. In one embodiment, the antibody is humanized antibody. In one embodiment the antibody is a chimeric antibody. In one embodiment the antibody is full-length antibody. In one embodiment the antibody is an IgG-class antibody, particularly an IgG1 subclass antibody. In one embodiment, the antibody is a recombinant antibody.

In certain embodiments, the therapeutic agent comprises an antibody fragment. Antibody fragments include, but are not limited to, Fab, Fab', Fab'-SH, F(ab')$_2$, Fv, and scFv fragments, and other fragments described below. For a review of certain antibody fragments, see Hudson et al. Nat. Med. 9:129-134 (2003). For a review of scFv fragments, see, e.g., Plückthun, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046. In one embodiment, the antibody fragment is a Fab fragment or a scFv fragment.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

In certain embodiments, the therapeutic agent comprises a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, the therapeutic agent comprises a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

In certain embodiments, the therapeutic agent comprises a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HuMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

Antibodies comprised in the therapeutic agent may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

In certain embodiments, the therapeutic agent comprises a multispecific antibody, e.g. a bispecific antibody. Multispecific antibodies are monoclonal antibodies that have binding specificities for at least two different sites. In certain embodiments, the binding specificities are for different antigens. In certain embodiments, the binding specificities are for different epitopes on the same antigen. Bispecific antibodies may also be used to localize cytotoxic agents to cells which express an antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bispecific antibodies (see, e.g., Kostelny et al., J. Immunol.., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to two different antigens (see, US 2008/0069820, for example).

"Crossmab" antibodies are also included herein (see e.g. WO2009080251, WO2009080252, WO2009080253, WO2009080254).

Another technique for making bispecific antibody fragments is the "bispecific T cell engager" or BiTE® approach (see, e.g., WO2004/106381, WO2005/061547, WO2007/042261, and WO2008/119567). This approach utilizes two antibody variable domains arranged on a single polypeptide. For example, a single polypeptide chain includes two single chain Fv (scFv) fragments, each having a variable heavy chain (VH) and a variable light chain (VL) domain separated by a polypeptide linker of a length sufficient to allow intramolecular association between the two domains. This single polypeptide further includes a polypeptide spacer sequence between the two scFv fragments. Each scFv recognizes a different epitope, and these epitopes may be specific for different cell types, such that cells of two different cell types are brought into close proximity or tethered when each scFv is engaged with its cognate epitope. One particular embodiment of this approach includes a scFv recognizing a cell-surface antigen expressed by an immune cell, e.g., a CD3 polypeptide on a T cell, linked to another scFv that recognizes a cell-surface antigen expressed by a target cell, such as a malignant or tumor cell.

As it is a single polypeptide, the bispecific T cell engager may be expressed using any prokaryotic or eukaryotic cell expression system known in the art, e.g., a CHO cell line. However, specific purification techniques (see, e.g., EP1691833) may be necessary to separate monomeric bispecific T cell engagers from other multimeric species, which may have biological activities other than the intended activity of the monomer. In one exemplary purification scheme, a solution containing secreted polypeptides is first subjected to a metal affinity chromatography, and polypeptides are eluted with a gradient of imidazole concentrations. This eluate is further purified using anion exchange chromatography, and polypeptides are eluted using with a gradient of sodium chloride concentrations. Finally, this eluate is subjected to size exclusion chromatography to separate monomers from multimeric species.

Antibodies with more than two valencies are contemplated. For example, trispecific antibodies can be prepared. Tuft et al. J. Immunol. 147: 60 (1991).

In certain embodiments, an antibody comprised in the therapeutic agent may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolpropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

The therapeutic agent may also comprise an antibody conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, the therapeutic agent comprises an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, the therapeutic agent comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, the therapeutic agent comprises an antibody as described herein conjugated to a radioactive atom to form a radioconjugate. A variety of radioactive isotopes are available for the production of radioconjugates. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the radioconjugate is used for detection, it may comprise a radioactive atom for scintigraphic studies, for example $Tc^{99m}$ or $I^{123}$, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

In some embodiments, the therapeutic agent may comprise an monoclonal antibody such as, but not limited to, alemtuzumab (LEMTRADA®), bevacizumab (AVASTIN®), cetuximab (ERBITUX®), panitumumab (VECTIBIX®), pertuzumab (OMNTTARG®, 2C4), trastuzumab (HERCEPTIN®), tositumomab (Bexxar®), abciximab (REOPRO®), adalimumab (HUMIRA®), apolizumab, aselizumab, atlizumab, bapineuzumab, basiliximab (SIMULECT®), bavituximab, belimumab (BENLYSTA®) briankinumab, canakinumab (ILARIS®), cedelizumab, certolizumab pegol (CIMZIA®), cidfusituzumab, cidtuzumab, cixutumumab, clazakizumab, crenezumab, daclizumab (ZENAPAX®), dalotuzumab, denosumab (PROLIA®, XGEVA®), eculizumab (SOLIRIS®), efalizumab, epratuzumab, erlizumab, felvizumab, fontolizumab, golimumab (SIMPONI®), ipilimumab, imgatuzumab, infliximab (REMICADE®), labetuzumab, lebrikizumab, lexatumumab, lintuzumab, lucatumumab, lulizumab pegol, lumretuzumab, mapatumumab, matuzumab, mepolizumab, mogamulizumab, motavizumab, motovizumab, muronomab, natalizumab (TYSABRI®), necitumumab (PORTRAZZA®), nimotuzumab (THERACIM®), nolovizumab, numavizumab, olokizumab, omalizumab (XOLAIR®), onartuzumab (also known as MetMAb), palivizumab (SYNAGIS®), pascolizumab, pecfusituzumab, pectuzumab, pembrolizumab (KEYTRUDA®), pexelizumab, priliximab, ralivizumab, ranibizumab (LUCENTIS®), reslivizumab, reslizumab, resyvizumab, robatumumab, rontalizumab, rovelizumab, ruplizumab, sarilumab, secukinumab, senbantumab, sifalimumab, sibrotuzumab, siltuximab (SYLVANT®) siplizumab, sontuzumab, tadocizumab, talizumab, tefibazumab, tocilizumab (ACTEMRA®), toralizumab, tucusituzumab, umavizumab, urtoxazumab, ustekinumab (STELARA®), vedolizumab (ENTYVIO®), visilizumab, zanolimumab, zalutumumab.

In one embodiment, the therapeutic agent comprises an antibody indicated for the treatment of cancer. In one embodiment, the therapeutic agent comprises an antibody indicated for the treatment of an autoimmune disease. In one embodiment, the therapeutic agent is an immunotherapeutic agent. In one embodiment the therapeutic agent in indicated for the treatment of cancer. In one embodiment, the therapeutic agent is an immunosuppressive agent. In one embodiment, the therapeutic agent is indicated for the treatment of an autoimmune disease.

Without wishing to be bound to theory, it is thought that enhancing T cell stimulation, by promoting an activating co-stimulatory molecule or by inhibiting a negative co-stimulatory molecule, may promote tumor cell death thereby treating or delaying progression of cancer. In some embodiments, the therapeutic agent may comprise an agonist directed against an activating co-stimulatory molecule. In some embodiments, an activating co-stimulatory molecule may include CD40, CD226, CD28, OX40, GITR, CD137, CD27, HVEM, or CD127. In some embodiments, the agonist directed against an activating co-stimulatory molecule is an agonist antibody that binds to CD40, CD226, CD28, OX40, GITR, CD137, CD27, HVEM, or CD127. In some embodiments, the therapeutic agent may comprise an antibody targeting GITR. In some embodiments, the antibody targeting GITR is TRX518. In some the therapeutic agent may comprise an antagonist directed against an inhibitory co-stimulatory molecule. In some embodiments, an inhibitory co-stimulatory molecule may include CTLA-4 (also known as CD152), PD-1, TIM-3, BTLA, VISTA, LAG-3, B7-H3, B7-H4, IDO, TIGIT, MICA/B, or arginase. In some embodiments, the antagonist directed against an inhibitory co-stimulatory molecule is an antagonist antibody that binds to CTLA-4, PD-1, TIM-3, BTLA, VISTA, LAG-3, B7-H3, B7-H4, IDO, TIGIT, MICA/B, or arginase.

In some embodiments, the therapeutic agent may comprise an anti-PD-1 antibody. In one embodiment the anti-PD-1 antibody is selected from the group consisting of MDX-1106 (nivolumab), MK-3475 (pembrolizumab, formerly known as lambrolizumab), CT-011 (pidilizumab). MDX-1106, also known as MDX-1106-04, ONO-4538, BMS-936558, or nivolumab, is an anti-PD-1 antibody described in WO2006/121168. MK-3475, also known as pembrolizumab or (formerly) lambrolizumab, is an anti-PD-1 antibody described in WO2009/114335. CT-011, also known as hBAT, hBAT-1 or pidilizumab, is an anti-PD-1 antibody described in WO2009/101 611.

In some embodiments, the therapeutic agent may comprise an immunoadhesin (e.g., an immunoadhesin comprising an extracellular or PD-1 binding portion of PD-L1 or PD-L2 fused to a constant region (e.g., an Fc region of an immunoglobulin sequence)). In one embodiment, the therapeutic agent may comprise AMP-224, also known as B7-DCIg (a PD-L2-Fc fusion soluble receptor described in WO2010/027827 and WO2011/066342).

In some embodiments, the therapeutic agent may comprise an anti-PD-L1 antibody. In one embodiment the anti-PD-L1 antibody is selected from the group consisting of YW243.55.S70, MPDL3280A, MDX-1105, and MEDI4736. Antibody YW243.55.S70 is an anti-PD-L1 antibody described in WO 2010/077634. MDX-1105, also known as BMS-936559, is an anti-PD-L1 antibody described in WO2007/005874. MEDI4736 is an anti-PD-L1 monoclonal antibody described in WO2011/066389 and US2013/034559. In one embodiment, the anti-PD-L1 antibody is atezolizumab.

In some embodiments, the therapeutic agent may comprise an antagonist directed against CTLA-4 (also known as CDR152), for example, a blocking antibody. In some embodiments, the therapeutic agent may comprise ipilimumab (also known as MDX-010, MDX-101, or YERVOY®). In some embodiments, the therapeutic agent may comprise tremelimumab (also known as ticilimumab or CP-675,206). In some embodiments, the therapeutic agent may comprise an antagonist directed against B7-H3 (also known as CD276), for example, a blocking antibody. In some embodiments, the therapeutic agent may comprise MGA271. In some embodiments, the therapeutic agent may comprise an antagonist directed against a TGF beta, for example, metelimumab (also known as CAT-192), fresolimumab (also known as GC1008), or LY2157299.

In some embodiments, the therapeutic agent may comprise an agonist directed against CD 137 (also known as TNFRSF9, 4-1BB, or ILA), for example, an activating antibody. In some embodiments, the therapeutic agent may comprise urelumab (also known as BMS-663513). In some embodiments, the therapeutic agent may comprise ligand of CD137 (also known as TNFRSF9, 4-1BB, or ILA), such as 4-1BBL. In some embodiments, the therapeutic agent may comprise an agonist directed against CD40, for example, an activating antibody. In some embodiments, the therapeutic agent may comprise CP-870893. In some embodiments, the therapeutic agent may comprise an agonist directed against OX40 (also known as CDC134), for example, an activating antibody. In some embodiments, the therapeutic agent may comprise an anti-OX40 antibody (e.g., AgonOX). In some embodiments, the therapeutic agent may comprise a ligand of OX40, such as OX40L. In some embodiments, the therapeutic agent may comprise an agonist directed against CD27, for example, an activating antibody. In some embodiments, the therapeutic agent may comprise CDX-1127.

In some embodiments, the therapeutic agent may comprise a T cell (e.g., a cytotoxic T cell or CTL) expressing a chimeric antigen receptor (CAR). In some embodiments, the therapeutic agent may comprise a T cell comprising a dominant-negative TGF beta receptor, e,g, a dominant-negative TGF beta type II receptor.

In some embodiments, the therapeutic agent may comprise an antibody-drug conjugate. In some embodiments, the antibody-drug conjugate comprises mertansine or monomethyl auristatin E (MMAE). In some embodiments, the therapeutic agent may comprise an anti-NaPi2b antibody-MMAE conjugate (also known as DNIB0600A or RG7599). In some embodiments, the therapeutic agent may comprise trastuzumab emtansine (also known as T-DM1, ado-trastuzumab emtansine, or KADCYLA®). In some embodiments, the therapeutic agent may comprise DMUC5754A. In some embodiments, the therapeutic agent may comprise an antibody-drug conjugate targeting the endothelin B receptor (EDNBR), for example, an antibody directed against EDNBR conjugated with MMAE (also known as DEDN6526A). In some embodiments, the therapeutic agent may comprise gemtuzumab ozogamicin (MYLOTARG®). In some embodiments, the therapeutic agent may comprise inotuzumab ozogamicin. In some embodiments, the therapeutic agent may comprise bivatuzumab mertansine. In some embodiments, the therapeutic agent may comprise cantuzumab mertansine. In some embodiments, the therapeutic agent may comprise cantuzumab ravtansine. In some embodiments, the therapeutic agent may comprise brentuximab vedotin (ADECTRIS®). In some embodiments, the therapeutic agent may comprise pinatuzumab vedotin. In some embodiments, the therapeutic agent may comprise polatuzumab vedotin In some embodiments, the therapeutic agent may comprise glembatumumab vedotin. In some embodiments, the therapeutic agent may comprise lorvotuzumab mertansine. In some embodiments, the therapeutic agent may comprise tacatuzumab tetraxetan. In some embodiments, the therapeutic agent may comprise vandortuzumab vedotin (DSTP3086S). In some embodiments, the therapeutic agent may comprise ibritumomab tiuxetan (ZEVALIN®)

In some embodiments, the therapeutic agent may comprise an antibody directed against angiopoietin 2 (also known as Ang2). In some embodiments, the therapeutic agent may comprise MEDI3617.

In some embodiments, the therapeutic agent may comprise an antibody targeting CSF-1R (also known as M-CSFR or CD115). In some embodiments, the therapeutic agent may comprise IMC-CS4 (LY3022855)). In some embodiments, the therapeutic agent may comprise emactuzumab.

In some embodiment, the therapeutic agent may comprise a cytokine. In some embodiments, the therapeutic agent may comprise an interferon, for example interferon alpha or interferon gamma. In some embodiments the therapeutic agent may comprise Roferon-A (also known as recombinant Interferon alpha-2a). In some embodiments, the therapeutic agent may comprise GM-CSF (also known as recombinant human granulocyte macrophage colony stimulating factor, rhu GM-CSF, sargramostim, or LEUKIN E®). In some embodiments, the therapeutic agent may comprise aldesleukin (PROLEUKIN®). In some embodiments, the therapeutic agent may comprise IL-12. In some embodiments, the therapeutic agent may comprise IL-10.

In some embodiments, the therapeutic agent may comprise an IL-2 fusion protein. In some embodiments, the therapeutic agent may comprise tucotuzumab celmoleukin. In some embodiments, the therapeutic agent may comprise darleukin. In some embodiments, the therapeutic agent may comprise teleukin.

In some embodiments, the therapeutic agent may comprise an IL-10 fusion protein. In some embodiments, the therapeutic agent may comprise dekavil. In some embodiments, the therapeutic agent may comprise a TNF fusion protein. In some embodiments, the therapeutic agent may comprise fibromun.

In some embodiments, the therapeutic agent may comprise a bispecific antibody. In some embodiments, the therapeutic agent may comprise a bispecific antibody, such as, but not limited to, duligotuzumab, MM-111, MM141, TF2, ABT-981, ABT-122, LY3164530, SAR156597, GSK2434735, ozoralizumab, ALX-0761, ALX-0061, ALX-0141, ACE910.

In some embodiments, the therapeutic agent may comprise a bispecific antibody capable of binding to a T cell and a target cell, e.g. a tumor cell. In some embodiment, the therapeutic agent may comprise a bispecific antibody that specifically binds to CD3 on a T cell and to a target cell antigen. In some embodiment, the therapeutic agent may comprise a bispecific T cell engager (BiTE®). In some embodiments, the therapeutic agent may comprise a bispecific antibody directed against CD3 and CD19. In one embodiment, the bispecific antibody is blinatumomab (BLINCYTO®). In one embodiment, the bispecific antibody is AFM11. In some embodiments, the therapeutic agent may comprise a bispecific antibody directed against CD3 and EpCAM. In one embodiment, the bispecific antibody is catumaxomab (REVOMAB®). In one embodiment, the bispecific antibody is solitomab (AMG 110, MT110). In some embodiments, the therapeutic agent may comprise a bispecific antibody directed against CD3 and Her2. In one embodiment, the bispecific antibody is ertumaxomab. In some embodiments, the therapeutic agent may comprise a bispecific antibody directed against CD3 and PSMA. In one embodiment, the bispecific antibody is BAY2010112 (AMG212, MT112). In some embodiments, the therapeutic agent may comprise a bispecific antibody directed against CD3 and CEA. In one embodiment, the bispecific antibody is MEDI565 (AMG211, MT111). In some embodiments, the therapeutic agent may comprise a bispecific antibody directed against CD3 and CD33. In one embodiment, the bispecific antibody is AMG330. In some embodiments, the therapeutic agent may comprise a bispecific antibody directed against CD3 and CD123. In one embodiment, the bispecific antibody is MGD006. In one embodiment, the bispecific antibody is XmAb®14045. In some embodiments, the therapeutic agent may comprise a bispecific antibody directed against CD3 and CD38. In some embodiments, the therapeutic agent may comprise a bispecific antibody directed against CD3 and gpA33. In one embodiment, the bispecific antibody is MGD007. In some embodiments, the therapeutic agent may comprise a bispecific antibody directed against CD3 and CD20. In one embodiment, the bispecific antibody is XmAb®13676. In one embodiment, the bispecific antibody is REGN1979. In one embodiment, the bispecific antibody is FBTA05 (Lymphomun).

In some embodiments, the therapeutic agent may comprise a bispecific antibody directed against CD30 and CD16A. In one embodiment, the bispecific antibody is AFM13. In some embodiments, the therapeutic agent may comprise a bispecific antibody directed against DR5 and FAP. In some embodiments, the therapeutic agent may comprise a bispecific antibody directed against Ang2 and VEGF. In one embodiment, the bispecific antibody is vanucizumab.

In some embodiments, the therapeutic agent may comprise an Fc domain. In some embodiments, the therapeutic agent may comprise a fusion protein comprising an Fc domain.

In some embodiments, the therapeutic agent may comprise a recombinant receptor or a fragment thereof. In some embodiments, the receptor is a T cell receptor. In some embodiments, the receptor is a TNF receptor. In some embodiments, the therapeutic agent may comprise etanercept (ENBREL®). In some embodiments, the receptor is a VEGF receptor. In some embodiments, the therapeutic agent may comprise ziv-aflibercept (ZALTRAP®). In some embodiments, the therapeutic agent may comprise aflibercept (EYLEA®). In some embodiments, the receptor is an IL-1 receptor. In some embodiments, the therapeutic agent may comprise rilonacept (ARCALYST®). In some embodiments, the therapeutic agent may comprise IMCgp100. In some embodiments, the therapeutic agent may comprise a chimeric antigen receptor (CAR). In some embodiments, the therapeutic agent may comprise a Factor IX-Fc fusion protein. In some embodiments, the therapeutic agent may comprise a Factor VIII-Fc fusion protein. In some embodiments, the therapeutic agent may comprise a CTLA-4-Fc fusion protein, such as e.g. belatacept, abatacept (ORENCIA®). In one embodiment, the therapeutic agent may comprise romiplostin.

In some embodiments, the therapeutic agent may comprise a recombinant receptor ligand, such as a TNF receptor ligand.

In some embodiments, the therapeutic agent may comprise a generic, biosimilar or non-comparable biologic version of an agent, e.g. an antibody, named herein.

In one embodiment, the therapeutic agent does not comprise obinutuzumab.

In some embodiments, the therapeutic agent comprises an antibody that specifically binds to carcinoembryonic antigen (CEA). In one embodiment, the antibody that specifically binds to CEA comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19. In a further embodiment, the antibody that specifically binds CEA comprises a heavy chain variable region sequence that is at least 80%, 85%, 90%, 95%, 96%, 97%, 98%, or 99% identical to of SEQ ID NO: 20 and a light chain variable region sequence that is at least 80%, 85%, 90%, 95%, 96%, 97%, 98%, or 99% identical to the sequence of SEQ ID NO: 21. In a further embodiment, the antibody that specifically binds CEA comprises the heavy chain variable region sequence of SEQ ID NO: 20 and the light chain variable region sequence of SEQ ID NO: 21.

In one embodiment, the antibody that specifically binds to CEA is a full-length antibody. In one embodiment, the antibody that specifically binds to CEA is an antibody of the human IgG class, particularly an antibody of the human IgG$_1$ class. In one embodiment, the antibody that specifically binds to CEA is an antibody fragment, particularly a Fab molecule or a scFv molecule, more particularly a Fab molecule. In one embodiment, the antibody that specifically binds to CEA is a humanized antibody.

In some embodiments, the therapeutic agent comprises an antibody that specifically binds to fibroblast activation protein (FAP). In one embodiment, the antibody that specifically binds FAP comprises a heavy chain variable region sequence that is at least 80%, 85%, 90%, 95%, 96%, 97%, 98%, or 99% identical to of SEQ ID NO: 25 and a light chain variable region sequence that is at least 80%, 85%, 90%, 95%, 96%, 97%, 98%, or 99% identical to the sequence of SEQ ID NO: 26. In a further embodiment, the antibody that specifically binds FAP comprises the heavy chain variable region sequence of SEQ ID NO: 25 and the light chain variable region sequence of SEQ ID NO: 26.

In one embodiment, the antibody that specifically binds to FAP is a full-length antibody. In one embodiment, the antibody that specifically binds to FAP is an antibody of the human IgG class, particularly an antibody of the human IgG$_1$ class. In one embodiment, the antibody that specifically binds to FAP is an antibody fragment, particularly a Fab molecule or a scFv molecule, more particularly a Fab molecule. In one embodiment, the antibody that specifically binds to FAP is a human antibody.

In some embodiments, the therapeutic agent comprises an antibody that specifically binds to CD3, particularly CD3 epsilon. In one embodiment, the antibody that specifically binds to CD3 comprises a heavy chain variable region comprising the heavy chain CDR (HCDR) 1 of SEQ ID NO: 32, the HCDR2 of SEQ ID NO: 33, and the HCDR3 of SEQ ID NO: 34; and a light chain variable region comprising the light chain CDR (LCDR) 1 of SEQ ID NO: 35, the LCDR2 of SEQ ID NO: 36 and the LCDR3 of SEQ ID NO: 37. In a further embodiment, the antibody that specifically binds CD3 comprises a heavy chain variable region sequence that is at least 80%, 85%, 90%, 95%, 96%, 97%, 98%, or 99% identical to of SEQ ID NO: 38 and a light chain variable region sequence that is at least 80%, 85%, 90%, 95%, 96%, 97%, 98%, or 99% identical to the sequence of SEQ ID NO: 39. In a further embodiment, the antibody that specifically binds CD3 comprises the heavy chain variable region sequence of SEQ ID NO: 38 and the light chain variable region sequence of SEQ ID NO: 39.

In one embodiment, the antibody that specifically binds to CD3 is a full-length antibody. In one embodiment, the antibody that specifically binds to CD3 is an antibody of the human IgG class, particularly an antibody of the human IgG$_1$ class. In one embodiment, the antibody that specifically binds to CD3 is an antibody fragment, particularly a Fab molecule or a scFv molecule, more particularly a Fab molecule. In a particular embodiment, the antibody that specifically binds to CD3 is a crossover Fab molecule wherein the variable domains or the constant domains of the Fab heavy and light chain are exchanged (i.e. replaced by each other). In one embodiment, the antibody that specifically binds to CD3 is a humanized antibody.

In some embodiments, the therapeutic agent comprises a cytokine. In one embodiment the cytokine is selected from the group consisting of , GM-CSF, IL-1α, IL-1β, IL-2, IL-3, IL-4, IL-5, IL-6, IL-7, IL-8, IL-10, IL-12, IL-15, IFN-α, If-β, IFN-γ, MIP-1α, MIP-1β, TNF-β, TNF-α, and TGF-β. In one embodiment, the cytokine is IL-2, particularly human IL-2. The sequence of wild-type human IL-2 is shown in SEQ ID NO: 12.

In one embodiment, the therapeutic agent comprises a mutant IL-2 polypeptide having reduced binding affinity to the α-subunit of the IL-2 receptor as compared to wild-type IL-2. Together with the β- and γ-subunits (also known as CD122 and CD132, respectively), the α-subunit (also known as CD25) forms the heterotrimeric high-affinity IL-2 receptor, while the dimeric receptor consisting only of the α- and γ-subunits is termed the intermediate-affinity IL-2 receptor. A mutant IL-2 polypeptide with reduced binding to the α-subunit of the IL-2 receptor has a reduced ability to induce IL-2 signaling in regulatory T (T$_{reg}$) cells, induces less activation-induced cell death (AICD) in T cells, and has a reduced toxicity profile in vivo, compared to a wild-type IL-2 polypeptide (see e.g. WO 2012/107417, incorporated herein by reference in its entirety).

In a more specific embodiment, the mutant IL-2 polypeptide comprises three amino acid substitutions at the positions corresponding to residue 42, 45 and 72 of human IL-2. In an even more specific embodiment, the mutant IL-2 polypeptide is a human IL-2 polypeptide comprising the amino acid substitutions F42A, Y45A and L72G (numbering relative to the human IL-2 sequence SEQ ID NO: 12). In one embodiment the mutant IL-2 polypeptide additionally comprises an amino acid mutation at a position corresponding to position 3 of human IL-2, which eliminates the O-glycosylation site of IL-2. In one embodiment said amino acid mutation which eliminates the O-glycosylation site of IL-2 at a position corresponding to residue 3 of human IL-2 is an amino acid substitution selected from the group of T3A, T3G, T3Q, T3E, T3N, T3D, T3R, T3K, and T3P. Particularly, said additional amino acid mutation is an amino acid substitution replacing a threonine residue by an alanine residue. A particular mutant IL-2 polypeptide useful in the invention comprises four amino acid substitutions at positions corresponding to residues 3, 42, 45 and 72 of human IL-2. Specific amino acid substitutions are T3A, F42A, Y45A and L72G. This mutant IL-2 polypeptide exhibits no detectable binding to CD25, reduced ability to induce apoptosis in T cells, reduced ability to induce IL-2 signaling in $T_{reg}$ cells, and a reduced toxicity profile in vivo (see e.g. WO 2012/107417, incorporated herein by reference in its entirety). However, it retains ability to activate IL-2 signaling in effector cells, to induce proliferation of effector cells, and to generate IFN-γ as a secondary cytokine by NK cells. The IL-2 or mutant IL-2 polypeptide according to any of the above embodiments may comprise additional mutations that provide further advantages such as increased expression or stability. For example, the cysteine at position 125 may be replaced with a neutral amino acid such as serine, alanine, threonine or valine, yielding C125S IL-2, C125A IL-2, C125T IL-2 or C125V IL-2 respectively, as described in U.S. Patent no. 4,518,584. As described therein, one may also delete the N-terminal alanine residue of IL-2 yielding such mutants as des-A1 C125S or des-A1 C125A. Alternatively or conjunctively, the IL-2 mutant may include a mutation whereby methionine normally occurring at position 104 of wild-type human IL-2 is replaced by a neutral amino acid such as alanine (see U.S. Patent no. 5,206,344). The resulting mutants, e. g., des-A1 M104A IL-2, des-A1 M104A C125S IL-2, M104A IL-2, M104A C125A IL-2, des-A1 M104A C125A IL-2, or M104A C125S IL-2 (these and other mutants may be found in U.S. Patent No. 5,116,943 and in Weiger et al., Eur J Biochem 180, 295-300 (1989)) may be used in conjunction with the particular IL-2 mutations described herein. Thus, in certain embodiments the IL-2 or mutant IL-2 polypeptide comprises an additional amino acid mutation at a position corresponding to residue 125 of human IL-2. In one embodiment said additional amino acid mutation is the amino acid substitution C125A.

In certain embodiments the mutant IL-2 polypeptide is essentially a full-length IL-2 molecule, particularly a human full-length IL-2 molecule. In one embodiment, the mutant IL-2 polypeptide comprises a polypeptide sequence that is at least 80%, at least 85%, or at least 90% identical to the sequence of SEQ ID NO: 12. In a specific embodiment the mutant IL-2 polypeptide comprises the polypeptide sequence of SEQ ID NO: 13.

In some embodiments, the therapeutic agent comprises an immunoconjugate. Particular immunoconjugates are described in WO 2012/107417 and WO 2012/146628 (each incorporated herein by reference in its entirety).

In one embodiment, the immunoconjugate comprises an antibody that specifically binds to CEA as described herein, and a mutant IL-2 polypeptide as described herein. In one embodiment, the antibody is a full-length antibody.

In one embodiment the therapeutic agent comprises an immunoconjugate comprising

(i) an antibody of the human IgG$_1$ subclass that specifically binds to CEA and comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19; and
(ii) a mutant human IL-2 polypeptide comprising the amino acid substitutions F42A, Y45A and L72G (numbering relative to the human IL-2 sequence SEQ ID NO: 12).

In one embodiment, the immunoconjugate comprises an antibody that specifically binds to FAP as described herein, and a mutant IL-2 polypeptide as described herein. In one embodiment, the antibody is a full-length antibody.

In one embodiment the therapeutic agent comprises an immunoconjugate comprising

(i) an antibody of the human IgG$_1$ subclass that specifically binds to FAP and comprises the heavy chain variable region of SEQ ID NO: 25; and the light chain variable region of SEQ ID NO: 26; and
(ii) a mutant human IL-2 polypeptide comprising the amino acid substitutions F42A, Y45A and L72G (numbering relative to the human IL-2 sequence SEQ ID NO: 12).

In one embodiment, the immunoconjugate comprises no more than one mutant IL-2 polypeptide. In one embodiment, the mutant IL-2 polypeptide is fused to the carboxy-terminal amino acid of one of the antibody heavy chains, optionally through a linker peptide. Suitable, non-immunogenic linker peptides include, for example, $(G_4S)_n$, $(SG_4)_n$ or $G_4(SG_4)_n$ linker peptides, wherein n is generally a number between 1 and 10, typically between 2 and 4. In one embodiment, the linker peptide is $(G_4S)_3$.

In one embodiment, the immunoconjugate comprises a polypeptide comprising a sequence that is at least 80%, 85%, 90%, 95%, 96%, 97%, 98%, or 99% identical to the sequence of SEQ ID NO: 22, a polypeptide comprising a sequence that is at least 80%, 85%, 90%, 95%, 96%, 97%, 98%, or 99% identical to the sequence of SEQ ID NO: 23, and a polypeptide comprising a sequence that is at least 80%, 85%, 90%, 95%, 96%, 97%, 98%, or 99% identical to the sequence of SEQ ID NO: 24. In one embodiment, the immunoconjugate comprises a polypeptide comprising the sequence of SEQ ID NO: 22, a polypeptide comprising the sequence of SEQ ID NO: 23, and a polypeptide comprising the sequence of SEQ ID NO: 24. (CEA IL2v)

In one embodiment, the immunoconjugate comprises a polypeptide comprising a sequence that is at least 80%, 85%, 90%, 95%, 96%, 97%, 98%, or 99% identical to the sequence of SEQ ID NO: 27, a polypeptide comprising a sequence that is at least 80%, 85%, 90%, 95%, 96%, 97%, 98%, or 99% identical to the sequence of SEQ ID NO: 28, and a polypeptide comprising a sequence that is at least 80%, 85%, 90%, 95%, 96%, 97%, 98%, or 99% identical to the sequence of SEQ ID NO: 29.

In one embodiment, the immunoconjugate comprises a polypeptide comprising the sequence of SEQ ID NO: 27, a polypeptide comprising the sequence of SEQ ID NO: 28, and a polypeptide comprising the sequence of SEQ ID NO: 29.

In one embodiment, the therapeutic agent comprises a bispecific antibody. Particular bispecific antibodies are described in WO 2013/026833 and WO 2014/131712 (each incorporated herein by reference in its entirety).

In one embodiment, the bispecific antibody comprises an antibody that specifically binds to CEA as described herein, and an antibody that specifically binds to CD3 as described herein. In one embodiment, the bispecific antibody comprises a first antibody that specifically binds to CD3 as described herein, and a second and a third antibody that specifically bind to CEA as described herein. In one embodiment, the first antibody is a crossover Fab molecule as described herein, and the second and the first antibody are each a conventional Fab molecule. In one embodiment, the bispecific antibody further comprises an Fc domain as described herein.

In one embodiment the therapeutic antibody comprises a bispecific antibody comprising

(i) a first antigen binding moiety that specifically binds to CD3, comprising a heavy chain variable region comprising the heavy chain CDR (HCDR) 1 of SEQ ID NO: 32, the HCDR2 of SEQ ID NO: 33, and the HCDR3 of SEQ ID NO: 34; and a light chain variable region comprising the light chain CDR (LCDR) 1 of SEQ ID NO: 35, the LCDR2 of SEQ ID NO: 36 and the LCDR3 of SEQ ID NO: 37, wherein the first antigen binding moiety is a crossover Fab molecule wherein either the variable or the constant regions, particularly the constant regions, of the Fab light chain and the Fab heavy chain are exchanged;

(ii) a second and a third antigen binding moiety that specifically bind to CEA, comprising a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19, wherein the second and third antigen binding moiety are each a Fab molecule, particularly a conventional Fab molecule;

(iii) an Fc domain composed of a first and a second subunit capable of stable association,

wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain.

In one embodiment, the antigen binding moieties and the Fc region are fused to each other by peptide linkers, particularly by peptide linkers as in SEQ ID NO: 42 and SEQ ID NO: 43.

In one embodiment, the bispecific antibody comprises a polypeptide comprising a sequence that is at least 80%, 85%, 90%, 95%, 96%, 97%, 98%, or 99% identical to the sequence of SEQ ID NO: 40, a polypeptide comprising a sequence that is at least 80%, 85%, 90%, 95%, 96%, 97%, 98%, or 99% identical to the sequence of SEQ ID NO: 41, a polypeptide comprising a sequence that is at least 80%, 85%, 90%, 95%, 96%, 97%, 98%, or 99% identical to the sequence of SEQ ID NO: 42, and a polypeptide comprising a sequence that is at least 80%, 85%, 90%, 95%, 96%, 97%, 98%, or 99% identical to the sequence of SEQ ID NO: 43.

In one embodiment, the bispecific antibody comprises a polypeptide comprising the sequence of SEQ ID NO: 40, a polypeptide comprising the sequence of SEQ ID NO: 41, a polypeptide comprising the sequence of SEQ ID NO: 42, and a polypeptide comprising the sequence of SEQ ID NO: 43. (CEA TCB)

*Fc domain*

An antibody, e.g. a bispecific antibody or an immunoconjugate, comprised in the therapeutic agent may comprise an Fc domain which consists of a pair of polypeptide chains comprising heavy chain domains of an antibody molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

In one embodiment, the Fc domain is an IgG Fc domain. In a particular embodiment the Fc domain is an $IgG_1$ Fc domain. In another embodiment the Fc domain is an $IgG_4$ Fc domain. In a more specific embodiment, the Fc domain is an $IgG_4$ Fc domain comprising an amino acid substitution at position S228 (Kabat numbering), particularly the amino acid substitution S228P. This amino acid substitution reduces in vivo Fab arm exchange of IgG$_4$ antibodies (see Stuben-rauch et al., Drug Metabolism and Disposition 38, 84-91 (2010)). In a further particular embodiment the Fc domain is human. An exemplary sequence of a human IgG$_1$ Fc region is given in SEQ ID NO: 30.

*Fc domain modifications promoting heterodimerization*

Antibodies, particularly bispecific antibodies or immunoconjugates, comprised in the therapeutic agent may comprise different components (e.g. antigen binding domains, cytokines) fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of such antibodies in recombinant production, it will thus be advantageous to introduce in the Fc domain of the antibody a modification promoting the association of the desired polypeptides.

Accordingly, in particular embodiments the Fc domain comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

There exist several approaches for modifications in the CH3 domain of the Fc domain in order to enforce heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291. Typically, in all such approaches the CH3 domain of the first subunit of the Fc domain and the CH3 domain of the second subunit of the Fc domain are both engineered in a complementary manner so that each CH3 domain (or the heavy chain comprising it) can no longer homodimerize with itself but is forced to heterodimerize with the complementarily engineered other CH3 domain (so that the first and second CH3 domain heterodimerize and no homodimers between the two first or the two second CH3 domains are formed). These different approaches for improved heavy chain heterodimerization are contemplated as different alternatives in combination with heavy-light chain modifications (e.g. variable or constant region exchange/replacement in Fab arms, or introduction of substitutions of charged amino acids with opposite charges in the CH1/CL interface) which reduce light chain mispairing and Bence Jones-type side products.

In a specific embodiment said modification promoting the association of the first and the second subunit of the Fc domain is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

Preferably said amino acid residue having a larger side chain volume is selected from the group consisting of arginine (R), phenylalanine (F), tyrosine (Y), and tryptophan (W).

Preferably said amino acid residue having a smaller side chain volume is selected from the group consisting of alanine (A), serine (S), threonine (T), and valine (V).

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain (the "knobs" subunit) the threonine residue at position 366 is replaced with a tryptophan residue (T366W), and in the CH3 domain of the second subunit of the Fc domain (the "hole" subunit) the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A) (numberings according to Kabat EU index).

In yet a further embodiment, in the first subunit of the Fc domain additionally the serine residue at position 354 is replaced with a cysteine residue (S354C) or the glutamic acid residue at position 356 is replaced with a cysteine residue (E356C), and in the second subunit of the Fc domain additionally the tyrosine residue at position 349 is replaced by a cysteine residue (Y349C) (numberings according to Kabat EU index). Introduction of these two cysteine residues results in formation of a disulfide bridge between the two subunits of the Fc domain, further stabilizing the dimer (Carter, J Immunol Methods 248, 7-15 (2001)).

In a particular embodiment, the first subunit of the Fc domain comprises amino acid substitutions S354C and T366W, and the second subunit of the Fc domain comprises amino acid substitutions Y349C, T366S, L368A and Y407V (numbering according to Kabat EU index).

In a particular embodiment the mutant IL-2 polypeptide in the immunoconjugate described herein is fused to the first subunit of the Fc domain (comprising the "knob" modification). Without wishing to be bound by theory, fusion of the IL-2 polypeptide to the knob-containing subunit of the Fc domain will (further) minimize the generation of immunoconjugates comprising two IL-2 polypeptides (steric clash of two knob-containing polypeptides).

Other techniques of CH3-modification for enforcing the heterodimerization are contemplated as alternatives according to the invention and are described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

In one embodiment the heterodimerization approach described in EP 1870459 A1, is used alternatively. This approach is based on the introduction of charged amino acids with opposite charges at specific amino acid positions in the CH3/CH3 domain interface between the two subunits of the Fc domain. One preferred embodiment are amino acid mutations R409D; K370E in one of the two CH3 domains (of the Fc domain) and amino acid mutations D399K; E357K in the other one of the CH3 domains of the Fc domain (numbering according to Kabat EU index).

In another embodiment the antibody comprises amino acid mutation T366W in the CH3 domain of the first subunit of the Fc domain and amino acid mutations T366S, L368A, Y407V in the CH3 domain of the second subunit of the Fc domain, and additionally amino acid mutations R409D; K370E in the CH3 domain of the first subunit of the Fc domain and amino acid mutations D399K; E357K in the CH3 domain of the second subunit of the Fc domain (numberings according to Kabat EU index).

In another embodiment the antibody comprises amino acid mutations S354C, T366W in the CH3 domain of the first subunit of the Fc domain and amino acid mutations Y349C, T366S, L368A, Y407V in the CH3 domain of the second subunit of the Fc domain, or the antibody comprises amino acid mutations Y349C, T366W in the CH3 domain of the first subunit of the Fc domain and amino acid mutations S354C, T366S, L368A, Y407V in the CH3 domains of the second subunit of the Fc domain and additionally amino acid mutations R409D; K370E in the CH3 domain of the first subunit of the Fc domain and amino acid mutations D399K; E357K in the CH3 domain of the second subunit of the Fc domain (all numberings according to Kabat EU index).

In one embodiment the heterodimerization approach described in WO 2013/157953 is used alternatively. In one embodiment a first CH3 domain comprises amino acid mutation T366K and a second CH3 domain comprises amino acid mutation L351D (numberings according to Kabat EU index). In a further embodiment the first CH3 domain comprises further amino acid mutation L351K. In a further embodiment the second CH3 domain comprises further an amino acid mutation selected from Y349E, Y349D and L368E (preferably L368E) (numberings according to Kabat EU index).

In one embodiment the heterodimerization approach described in WO 2012/058768 is used alternatively. In one embodiment a first CH3 domain comprises amino acid mutations L351Y, Y407A and a second CH3 domain comprises amino acid mutations T366A, K409F. In a further embodiment the second CH3 domain comprises a further amino acid mutation at position T411, D399, S400, F405, N390, or K392, e.g. selected from a) T411N, T411R, T411Q, T411K, T411D, T411E or T411W, b) D399R, D399W, D399Y or D399K, c) S400E, S400D, S400R, or S400K, d) F405I, F405M, F405T, F405S, F405V or F405W, e) N390R, N390K or N390D, f) K392V, K392M, K392R, K392L, K392F or K392E (numberings according to Kabat EU index). In a further embodiment a first CH3 domain comprises amino acid mutations L351Y, Y407A and a second CH3 domain comprises amino acid mutations T366V, K409F. In a further embodiment a first CH3 domain comprises amino acid mutation Y407A and a second CH3 domain comprises amino acid mutations T366A, K409F. In a further embodiment the second CH3 domain further comprises amino acid mutations K392E, T411E, D399R and S400R (numberings according to Kabat EU index).

In one embodiment the heterodimerization approach described in WO 2011/143545 is used alternatively, e.g. with the amino acid modification at a position selected from the group consisting of 368 and 409 (numbering according to Kabat EU index).

In one embodiment the heterodimerization approach described in WO 2011/090762, which also uses the knobs-into-holes technology described above, is used alternatively. In one embodiment a first CH3 domain comprises amino acid mutation T366W and a second CH3 domain comprises amino acid mutation Y407A. In one embodiment a first CH3 domain comprises amino acid mutation T366Y and a second CH3 domain comprises amino acid mutation Y407T (numberings according to Kabat EU index).

In one embodiment the antibody or its Fc domain is of IgG$_2$ subclass and the heterodimerization approach described in WO 2010/129304 is used alternatively.

In an alternative embodiment a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable. In one such embodiment a first CH3 domain comprises amino acid substitution of K392 or N392 with a negatively charged amino acid (e.g. glutamic acid (E), or aspartic acid (D), preferably K392D or N392D) and a second CH3 domain comprises amino acid substitution of D399, E356, D356, or E357 with a positively charged amino acid (e.g. lysine (K) or arginine (R), preferably D399K, E356K, D356K, or E357K, and more preferably D399K and E356K). In a further embodiment the first CH3 domain further comprises amino acid substitution of K409 or R409 with a negatively charged amino acid (e.g. glutamic acid (E), or aspartic acid (D), preferably K409D or R409D). In a further embodiment the first CH3 domain further or alternatively comprises amino acid substitution of K439 and/or K370 with a negatively charged amino acid (e.g. glutamic acid (E), or aspartic acid (D)) (all numberings according to Kabat EU index).

In yet a further embodiment the heterodimerization approach described in WO 2007/147901 is used alternatively. In one embodiment a first CH3 domain comprises amino acid mutations K253E, D282K, and K322D and a second CH3 domain comprises amino acid mutations D239K, E240K, and K292D (numberings according to Kabat EU index).

In still another embodiment the heterodimerization approach described in WO 2007/110205 can be used alternatively.

In one embodiment, the first subunit of the Fc domain comprises amino acid substitutions K392D and K409D, and the second subunit of the Fc domain comprises amino acid substitutions D356K and D399K (numbering according to Kabat EU index).

*Fc domain modifications reducing Fc receptor binding and/or effector function*

The Fc domain confers to an antibody, such as a bispecific antibody or immunoconjugate, favorable pharmacokinetic properties, including a long serum half-life which contributes to good accumulation in the target tissue and a favorable tissue-blood distribution ratio. At the same time it may, however, lead to undesirable targeting of the antibody to cells expressing Fc receptors rather than to the preferred antigen-bearing cells. Moreover, the co-activation of Fc receptor signaling pathways may lead to cytokine release which, in combination with other immunostimulatory properties the antibody may have and the long half-life of the antibody, results in excessive activation of cytokine receptors and severe side effects upon systemic administration.

Accordingly, in particular embodiments, the Fc domain of the antibody, particularly bispecific antibody or immunoconjugate, comprised in the therapeutic agent exhibits reduced binding affinity to an Fc receptor and/or reduced effector function, as compared to a native IgG$_1$ Fc domain. In one such embodiment the Fc domain (or the molecule, e.g. antibody, comprising said Fc domain) exhibits less than 50%, preferably less than 20%, more preferably less than 10% and most preferably less than 5% of the binding affinity to an Fc receptor, as compared to a native IgG$_1$ Fc domain (or a corresponding molecule comprising a native IgG$_1$ Fc domain), and/or less than 50%, preferably less than 20%, more preferably less than 10% and most preferably less than 5% of the effector function, as compared to a native IgG$_1$ Fc domain domain (or a corresponding molecule comprising a native IgG$_1$ Fc domain). In one embodiment, the Fc domain (or the molecule, e.g. antibody, comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcγRIIIa, FcγRI or FcγRIIa, most specifically human FcγRIIIa. In one embodiment the effector function is one or more selected from the group of CDC, ADCC, ADCP, and cytokine secretion. In a particular embodiment the effector function is ADCC. In one embodiment the Fc domain exhibits substantially similar binding affinity to neonatal Fc receptor (FcRn), as compared to a native IgG$_1$ Fc domain domain. Substantially similar binding to FcRn is achieved when the Fc domain (or the molecule, e.g. antibody, comprising said Fc domain) exhibits greater than about 70%, particularly greater than about 80%, more particularly greater than about 90% of the binding affinity of a native IgG$_1$ Fc domain (or the corresponding molecule comprising a native IgG$_1$ Fc domain) to FcRn.

In certain embodiments the Fc domain is engineered to have reduced binding affinity to an Fc receptor and/or reduced effector function, as compared to a non-engineered Fc domain. In particular embodiments, the Fc domain comprises one or more amino acid mutation that reduces the binding affinity of the Fc domain to an Fc receptor and/or effector function. Typically, the same one or more amino acid mutation is present in each of the two subunits of the Fc domain. In one embodiment the amino acid mutation reduces the binding affinity of the Fc domain to an Fc receptor. In one embodiment the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the molecule, e.g. antibody, comprising an engineered Fc domain exhibits less than 20%, particularly less than 10%, more particularly less than 5% of the binding affinity to an Fc receptor as compared to a corresponding molecule comprising a non-engineered Fc domain. In a particular embodiment the Fc receptor is an Fcγ receptor. In some embodiments the Fc receptor is a human Fc receptor. In some embodiments the Fc receptor is an activating Fc receptor. In a specific embodiment the Fc receptor is an activating human Fcγ receptor, more specifically human FcγRIIIa, FcγRI or FcγRIIa, most specifically human FcγRIIIa. Preferably, binding to each of these receptors is reduced. In some embodiments binding affinity to a complement component, specifically binding affinity to C1q, is also reduced. In one embodiment binding affinity to neonatal Fc receptor (FcRn) is not reduced. Substantially similar binding to FcRn, i.e. preservation of the binding affinity of the Fc domain to said receptor, is achieved when the Fc domain (or the molecule, e.g. antibody, comprising said Fc domain) exhibits greater than about 70% of the binding affinity of a non-engineered form of the Fc domain (or a corresponding molecule comprising said non-engineered form of the Fc domain) to FcRn. The Fc domain, or molecule (e.g. antibody) comprising said Fc domain, may exhibit greater than about 80% and even greater than about 90% of such affinity. In certain embodiments the Fc domain is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment the reduced effector function is one or more selected from the group of reduced CDC, reduced ADCC, reduced ADCP, and reduced cytokine secretion. In a particular embodiment the reduced effector function is reduced ADCC. In one embodiment the reduced ADCC is less than 20% of the ADCC induced by a non-engineered Fc domain (or a corresponding molecule comprising a non-engineered Fc domain).

In one embodiment the amino acid mutation that reduces the binding affinity of the Fc domain to an Fc receptor and/or effector function is an amino acid substitution. In one embodiment the Fc domain comprises an amino acid substitution at a position selected from the group of E233, L234, L235, N297, P331 and P329 (numberings according to Kabat EU index). In a more specific embodiment the Fc domain comprises an amino acid substitution at a position selected from the group of L234, L235 and P329 (numberings according to Kabat EU index). In some embodiments the Fc domain comprises the amino acid substitutions L234A and L235A (numberings according to Kabat EU index). In one such embodiment, the Fc domain is an IgG$_1$ Fc domain, particularly a human IgG$_1$ Fc domain. In one embodiment the Fc domain comprises an amino acid substitution at position P329. In a more specific embodiment the amino acid substitution is P329A or P329G, particularly P329G (numberings according to Kabat EU index). In one embodiment the Fc domain comprises an amino acid substitution at position P329 and a further amino acid substitution at a position selected from E233, L234, L235, N297 and P331 (numberings according to Kabat EU index). In a more specific embodiment the further amino acid substitution is E233P, L234A, L235A, L235E, N297A, N297D or P331S. In particular embodiments the Fc domain comprises amino acid substitutions at positions P329, L234 and L235 (numberings according to Kabat EU index). In more particular embodiments the Fc domain comprises the amino acid mutations L234A, L235A and P329G ("P329G LALA"). In one such embodiment, the Fc domain is an IgG$_1$ Fc domain, particularly a human IgG$_1$ Fc domain. The "P329G LALA" combination of amino acid substitutions almost completely abolishes Fcγ receptor (as well as complement) binding of a human IgG$_1$ Fc domain, as described in PCT publication no. WO 2012/130831, incorporated herein by reference in its entirety. WO 2012/130831 also describes methods of preparing such mutant Fc domains and methods for determining its properties such as Fc receptor binding or effector functions.

IgG$_4$ antibodies exhibit reduced binding affinity to Fc receptors and reduced effector functions as compared to IgG$_1$ antibodies. Hence, in some embodiments the Fc domain is an IgG$_4$ Fc domain, particularly a human IgG$_4$ Fc domain. In one embodiment the IgG$_4$ Fc domain comprises amino acid substitutions at position S228, specifically the amino acid substitution S228P (numberings according to Kabat EU index). To further reduce its binding affinity to an Fc receptor and/or its effector function, in one embodiment the IgG$_4$ Fc domain comprises an amino acid substitution at position L235, specifically the amino acid substitution L235E (numberings according to Kabat EU index). In another embodiment, the IgG$_4$ Fc domain comprises an amino acid substitution at position P329, specifically the amino acid substitution P329G (numberings according to Kabat EU index). In a particular embodiment, the IgG$_4$ Fc domain comprises amino acid substitutions at positions S228, L235 and P329, specifically amino acid substitutions S228P, L235E and P329G (numberings according to Kabat EU index). Such IgG$_4$ Fc domain mutants and their Fcγ receptor binding properties are described in PCT publication no. WO 2012/130831, incorporated herein by reference in its entirety.

In a particular embodiment the Fc domain exhibiting reduced binding affinity to an Fc receptor and/or reduced effector function, as compared to a native IgG$_1$ Fc domain, is a human IgG$_1$ Fc domain comprising the amino acid substitutions L234A, L235A and optionally P329G, or a human $IgG_4$ Fc domain comprising the amino acid substitutions S228P, L235E and optionally P329G (numberings according to Kabat EU index).

In certain embodiments N-glycosylation of the Fc domain has been eliminated. In one such embodiment the Fc domain comprises an amino acid mutation at position N297, particularly an amino acid substitution replacing asparagine by alanine (N297A) or aspartic acid (N297D) or glycine (N297G) (numberings according to Kabat EU index).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056) (numberings according to Kabat EU index). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. Alternatively, binding affinity of Fc domains or molecules comprising an Fc domain for Fc receptors may be evaluated using cell lines known to express particular Fc receptors, such as human NK cells expressing FcγIIIa receptor.

Effector function of an Fc domain, or a molecule (e.g. an antibody) comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the Fc domain, or molecule (e.g. antibody) comprising the Fc domain, is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

**Treatment regimen**

According to the invention, the Type II anti-CD20 antibody and the therapeutic agent may be administered in various ways (e.g. with regard to the route of administration, dose and/or timing), as long as the anti-CD20 antibody is administered prior to the therapeutic agent and that the administration of the CD20 antibody has effectively induced a reduction of the number of B cells in the treated subject by the time the therapeutic agent is administered. Without wishing to be bound by theory, the reduction of the number of B cells in the subject prior to administration of the therapeutic agent will reduce or prevent the formation of anti-drug antibodies (ADAs) to the therapeutic agent, and will thus reduce or prevent a loss of efficacy of the therapeutic agent and/or adverse events in the subject associated with ADAs.

In one embodiment, the treatment regimen effectively reduces the formation of anti-drug antibodies (ADAs) in the subject in response to the administration of the therapeutic agent as compared to a corresponding treatment regimen without the administration of the anti-CD20 antibody. In one embodiment, the formation of ADAs is reduced at least 2-fold, at least 3-fold, at least 4-fold, at least 5-fold, at least 10-fold, at least 20-fold, at least 50-fold, or at least 100-fold as compared to a corresponding treatment regimen without the administration of the anti-CD20 antibody. In one embodiment, the formation of ADAs is essentially prevented. In one embodiment, the reduction or prevention of the formation of ADAs is 1, 2, 3, 4, 5, 6, 7, 8, 9, 10, 11, 12, 13, 14, 15, 16, 17, 18, 19, 20 or 21 days, or 1 month, 2 months, 3 months, 4 months, 5 months, 6 months, 12 months, or more, after administration of the therapeutic agent. In one embodiment, the reduction or prevention of ADA is about 2 months after administration of the therapeutic agent.

In one embodiment, the ADA titer in the subject after administration of the therapeutic agent does not exceed the ADA titer in the subject prior to administration of the therapeutic agent. In one embodiment, the ADA titer in the subject after administration of the therapeutic agent does not exceed the ADA titer in the subject prior to administration of the therapeutic agent by more than 1.1-fold, more than 1.2-fold, more than 1.5-fold, more than 2-fold, more than 3-fold, more than 4-fold, more than 5-fold, or more than 10-fold. In one embodiment, the ADA titer in the subject after administration of the therapeutic agent is increased less than 1.1-fold, less than 1.2-fold, less than 1.5-fold, less than 2-fold, less than 3-fold, less than 4-fold, less than 5-fold, or less than 10-fold, as compared to the ADA titer in the subject prior to administration of the therapeutic agent. In one embodiment, the ADA titer in the subject after administration of the therapeutic agent is the ADA titer at 1, 2, 3, 4, 5, 6, 7, 8, 9, 10, 11, 12, 13, 14, 15, 16, 17, 18, 19, 20 or 21 days, or 1 month, 2 months, 3 months, 4 months, 5 months, 6 months, 12 months, or more, after administration of the therapeutic agent. In one embodiment, the ADA titer in the subject after administration of the therapeutic agent is the ADA titer at about 2 months after administration of the therapeutic agent.

In one embodiment, essentially no ADAs are detectable in the subject after administration of the therapeutic agent, particularly 1, 2, 3, 4, 5, 6, 7, 8, 9, 10, 11, 12, 13, 14, 15, 16, 17, 18, 19, 20 or 21 days, or 1 month, 2 months, 3 months, 4 months, 5 months, 6 months, 12 months, or more, after administration of the therapeutic agent.

ADAs can be detected by methods known in the art (see e.g. Mire-Sluis et al., J Immunol Methods (2004) 289, 1-16; Nencini et al., Drug Dev Res (2014), 75 Suppl 1, S4-6; Schouwenburg et al., Nat Rev Rheumatol (2015) 9, 164-172). An exemplary method to detect ADAs is a sandwich ELISA, in which the therapeutic agent (e.g. an antibody) is coated to the assay plate, is exposed to serum of the treated subject, and the presence of ADAs is detected by labelled therapeutic agent. Another exemplary method to detect ADAs is an antigen binding test wherein immunoglobulins from the treated subject's serum are aggregated on a protein (e.g. Protein A Sepharose) and the presence of ADAs is detected by labelled therapeutic agent.

ADAs can be detected e.g. in a blood sample taken from the subject. In one embodiment, the ADA titer in the subject (as measured e.g. in a blood sample taken from the subject) does not exceed a titer (i.e. highest possible dilution of the sample giving an assay signal above the assay cut point) of about 10, of about 20, of about 30, of about 40, of about 50, of about 100, of about 200, or about 500, or of about 1000 after the administration of the therapeutic agent, particularly 1, 2, 3, 4, 5, 6, 7, 8, 9, 10, 11, 12, 13, 14, 15, 16, 17, 18, 19, 20 or 21 days, or 1 month, 2 months, 3 months, 4 months, 5 months, 6 months, 12 months, or more, after administration of the therapeutic agent.

In some embodiments, the treatment regimen increases efficacy of the therapeutic agent, as compared to a treatment regimen without as compared to a corresponding treatment regimen without the administration of the anti-CD20 antibody. In some embodiments, the treatment regimen increases overall survival of the subject, as compared to a corresponding treatment regimen without the administration of the anti-CD20 antibody. In some embodiments, the treatment regimen increases progression-free survival of the subject, as compared to a corresponding treatment regimen without the administration of the anti-CD20 antibody.

In some embodiments, the treatment regimen increases the serum half-life of the therapeutic agent, as compared to a corresponding treatment regimen without the administration of the anti-CD20 antibody.

In some embodiments, the treatment regimen reduces toxicity of the therapeutic agent, as compared to a corresponding treatment regimen without the administration of the anti-CD20 antibody.

According to the invention, the period of time between the administration of the anti-CD20 antibody and the administration of the therapeutic agent is sufficient for reduction of the number of B-cells in the subject in response to the administration of the CD20 antibody.

In one embodiment, the period of time is 3 days to 21 days, 5 days to 20 days, 7 days to 21 days, 7 days to 14 days, 5 days to 15 days, 7 days to 15 days, 8 days to 15 days, 10 days to 20 days, 10 days to 15 days, 11 days to 14 days, or 12 days to 13 days. In one embodiment, the period of time is 7 days to 14 days. In a particular embodiment, the period of time is 10 days to 15 days. In one embodiment, the period of time is 8 days to 15 days.

In one embodiment, the period of time is about about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, or about 30 days.

In one embodiment, the period of time is at least 3 days, at least 4 days, at least 5 days, at least 6 days, at least 7 days, at least 8 days, at least 9 days, at least 10 days, at least 11 days, at least 12 days, at least 13 days, at least 14 days, or at least 15 days. In a particular embodiment, the period of time is at least 5 days. In a further particular embodiment, the period of time is at least 7 days.

In one embodiment, the period of time is between the last administration of the anti-CD20 antibody and the (first, if several) administration of the therapeutic agent. In one embodiment, no administration of the therapeutic agent is made during the period of time.

In a particular embodiment, the reduction of the number of B cells is in the blood of the subject. In one embodiment, the B cells are peripheral blood B cells.

In some embodiments, the reduction of B cells is in a tissue of the subject. In one embodiment, the tissue is a tumor. In one embodiment, the tissue is a lymph node. In one embodiment, the tissue is spleen. In one embodiment, the tissue is the marginal zone of spleen. In one embodiment, the B cells are lymph node B cells. In one embodiment, the B cells are splenic B cells. In one embodiment, the B cells are splenic marginal zone B cells. In one embodiment, the B cells are CD20-positive B cells, i.e. B cells expressing CD20 on their surface.

In one embodiment, the reduction of the number of B cells is a reduction of at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95%. In one embodiment, the reduction of the number of B cells is a complete elimination of B cells. In a particular embodiment, the reduction of the number of B cells is a reduction of at least 90%, particularly at least 95%, of the number of B cells in the (peripheral) blood of the subject. In one embodiment, the reduction of the number of B cells is a reduction as compared to the number of B cells in the subject prior to the (first, if several) administration of the anti-CD20 antibody to the subject.

The number of B cells in the subject may be determined by any method known in the art suitable for quantifying B cells in patient blood or tissue, such as flow cytometric, immunohistochemical or immunofluorescent methods, using antibodies against B cell markers such as CD20, CD 19, and/or PAX5.

The number of B cells may also be determined indirectly, by quantification of protein or mRNA levels of B-cell markers in patient blood or tissues. Suitable methods known in the art for the determination of specific protein levels include immunoassay methods such as enzyme-linked immunosorbent assay (ELISA), or Western Blot, methods for determination of mRNA levels include for example quantitative RT-PCR or microarray technologies.

All the above mentioned methods and technologies are well known in the art and can be deduced from standard textbooks such as Lottspeich (Bioanalytik, Spektrum Akademisher Verlag, 1998) or Sambrook and Russell (Molecular Cloning: A Laboratory Manual, CSH Press, Cold Spring Harbor, NY, U.S.A., 2001).

In certain embodiments, the reduction of the number of B cells is determined by quantification of B cells in the blood of the subject (e.g. in a blood sample taken from the subject). In one such embodiment, B cells are quantified by flow cytometric analysis. Flow cytometric methods (FACS) are well known in the art for the quantification of cells in blood or tissue samples. In particular, they allow determining the number of cells expressing a specific antigen (e.g. CD20 and/or CD19) among a defined total number of cells in a blood or tissue sample (e.g. a blood sample, or (part of) a tissue biopsy). In one embodiment, B cells are quantified by flow cytometric analysis using an anti-CD 19 antibody and/or an anti-CD20 antibody.

In other embodiments, the reduction of the number of B cells is determined by quantification of B cells in a tissue, e.g. a tumor, of said individual (e.g. in a tissue biopsy taken from the subject). In one such embodiment, B cells are quantified by immunohistochemical or immunofluorescent analysis. In one embodiment, B cells are quantified by immunohistochemical analysis using an anti-CD 19 antibody, an anti-CD20 antibody and/or an anti-PAX5 antibody.

Methods of the present invention can be applied in the treatment of a variety of diseases, depending on the therapeutic agent(s) used.

In certain embodiments the disease to be treated is a proliferative disorder, particularly cancer. Non-limiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a method of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. In some embodiments, the cancer is a solid tumor. In some embodiments, the cancer expresses the target of the therapeutic agent, e.g. an antibody. In one embodiment, the cancer expresses CEA. In another embodiment, the cancer expresses FAP.

In some embodiments, the disease to be treated is an inflammatory disorder. In some embodiments, the disease to be treated is an autoimmune disease (i.e. a non-malignant disease or disorder arising from and directed against an individual's own tissues). Examples of autoimmune diseases or disorders include, but are not limited to, inflammatory responses such as inflammatory skin diseases including psoriasis and dermatitis (e.g. atopic dermatitis); responses associated with inflammatory bowel disease (such as Crohn's disease and ulcerative colitis); dermatitis; allergic conditions such as eczema and asthma; rheumatoid arthritis; systemic lupus erythematosus (SLE) (including but not limited to lupus nephritis, cutaneous lupus); diabetes mellitus (e.g. type 1 diabetes mellitus or insulin dependent diabetes mellitus); multiple sclerosis and juvenile onset diabetes. In one embodiment, the disease is transplant rejection or graft-versus-host disease.

In some embodiments, the disease to be treated is an infectious disease, such as viral infection or a bacterial infection. In other embodiments, the disease to be treated is neurological disorder. In still further embodiments, the disease to be treated is a metabolic disorder.

A skilled artisan readily recognizes that in many cases the therapeutic agent may not provide a cure but may only provide partial benefit. In some embodiments, a physiological change having some benefit is also considered therapeutically beneficial. Thus, in some embodiments, an amount of therapeutic agent that provides a physiological change is considered an "effective amount" or a "therapeutically effective amount".

The subject, patient, or individual in need of treatment is typically a mammal, more specifically a human. In certain embodiments, the subject is a human. In one embodiment, the subject suffers from a locally advanced and/or metastatic solid tumor and has progressed on or is intolerant to the standard of care therapy. In one embodiment, the tumor is a CEA-expressing tumor. In another embodiment, the tumor is a FAP-expressing tumor. Expression of CEA and/or FAP in a tumor may be determined for example by immunohistochemical analysis of a tumor biopsy taken from the subject.

*Administration of the Type II anti-CD20 antibody*

According to the invention, the period of time between the administration of the anti-CD20 antibody and the administration of the therapeutic agent and the dose of the anti-CD20 antibody are chosen such as to effectively reduce the number of B cells in the subject prior to administration of the therapeutic agent.

The anti-CD20 antibody can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein. In one embodiment, the anti-CD20 antibody is administered parenterally, particularly intravenously, e.g. by intravenous infusion.

The anti-CD20 antibody would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one embodiment, the administration of the anti-CD20 antibody is a single administration. In another embodiment, the administration of the anti-CD20 antibody is two or more separate administrations. In one embodiment, the two or more separate administrations are on two or more consecutive days. In one embodiment, no further administration of the anti-CD20 antibody is made to the subject before or after the administration of the therapeutic agent. In one embodiment, the administration of the anti-CD20 antibody is a single administration, or two administrations on two consecutive days, and no further administration of the anti-CD20 antibody is made. In one embodiment, the period of time is between the last administration of the anti-CD20 antibody and the (first, if several) administration of the therapeutic agent.

In one embodiment, the administration of the anti-CD20 antibody is a dose of anti-CD20 antibody effective for the reduction of B cells in the subject. In one embodiment, the dose of anti-CD20 antibody is effective in reducing the number of B cells in the subject within the period of time between the administration of the anti-CD20 antibody and the administration of the therapeutic agent. In one embodiment, the period of time between the administration of the anti-CD20 antibody and the administration of the therapeutic agent and the administered dose of anti-CD20 antibody is sufficient for reduction of the number of B-cells in the subject in response to the administration of the CD20 antibody.

In one embodiment, the administration of the anti-CD20 antibody is a dose of about 2 g anti-CD20 antibody. The dose of about 2 g anti-CD20 antibody may be administered to the subject as a single administration of about 2 g, or as several administrations, e.g. two administrations of about 1 g each or three administrations of e. g. 100 mg, 900 mg and 1000 mg. In one embodiment, one administration of about 2 g anti-CD20 antibody is made to the subject. In another embodiment, two administrations of about 1 g anti-CD20 antibody each are made to the subject on two consecutive days. In still another embodiment, three administrations ((i) to (iii)) of (i) about 100 mg anti-CD20 antibody, (ii) about 900 mg anti-CD20 antibody, and (iii) about 1000 mg anti-CD20 antibody are made to the subject on three consecutive days. In one embodiment, two administration of about 1 g anti-CD20 antibody are made to the subject on two consecutive days, 10 days to 15 days before the administration of the therapeutic agent. In one embodiment, one administration of about 2 g anti-CD20 antibody is made to the subject 10 days to 15 days before the administration of the therapeutic agent. In one embodiment, no further administration of the anti-CD20 antibody is made to the subject. In one embodiment, no administration of the therapeutic agent is made to the subject prior to the administration of the anti-CD20 antibody (at least not within the same course of treatment).

In one embodiment, the treatment regimen further comprises administration of premedication prior to the administration of the anti-CD20 antibody. In embodiment the premedication comprises a corticosteroid (such as e.g. prednisolone), paracetamol/acetaminophen, and/or an anti-histamine (such as e.g. diphenhydramine). In one embodiment, the premedication is administered at least 60 minutes prior to the administration of the anti-CD20 antibody.

In one embodiment, the treatment regimen does not comprise administration of an immunosuppressive agent other than the anti-CD20 antibody (and optionally the above-described premedication) prior to the administration of the therapeutic agent. In one embodiment, the treatment regimen does not comprise administration of an agent selected from the group of methotrexate, azathioprine, 6-mercaptopurine, leflunomide, cyclosporine, tacrolimus/FK506, mycophenolate mofetil and mycophenolate sodium prior to the administration of the therapeutic agent. In one embodiment, the treatment regimen does not comprise administration of a further antibody in addition to the anti-CD20 antibody prior to the administration of the therapeutic agent.

*Administration of the therapeutic agent*

According to the invention, the therapeutic agent can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein. In one embodiment, the therapeutic agent is administered parenterally, particularly intravenously, e.g. by intravenous infusion.

The therapeutic agent would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutic agent need not be, but is optionally formulated with one or more agents currently used to prevent or treat the disorder in question. The effective amount of such other agents depends on the amount of therapeutic agent present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of the therapeutic agent (when used alone or in combination with one or more other additional therapeutic agents) will depend on the type of disease to be treated, the type of therapeutic agent, the severity and course of the disease, whether the therapeutic agent is administered for preventive or therapeutic purposes, previous therapy, the patient's clinical history and response to the therapeutic agent, and the discretion of the attending physician. The therapeutic agent is suitably administered to the patient at one time or over a series of treatments. Depending on the type and severity of the disease, about 1 $\mu$g/kg to 15 mg/kg (e.g. 0.1 mg/kg - 10 mg/kg) of therapeutic agent can be an initial candidate dosage for administration to the subject, whether, for example, by one or more separate administrations, or by continuous infusion. One typical daily dosage might range from about 1 $\mu$g/kg to 100 mg/kg or more, depending on the factors mentioned above. For repeated administrations over several days or longer, depending on the condition, the treatment would generally be sustained until a desired suppression of disease symptoms occurs. One exemplary dosage of the therapeutic agent would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the subject. Such doses may be administered intermittently, e.g. every week, every two weeks, or every three weeks (*e.g.* such that the subject receives from about two to about twenty, or *e.g.* about six doses of the therapeutic agent). An initial higher loading dose, followed by one or more lower doses, or an initial lower dose, followed by one or more higher doses may be administered. An exemplary dosing regimen comprises administering an initial dose of about 10 mg, followed by a bi-weekly dose of about 20 mg of the therapeutic agent. However, other dosage regimens may be useful. The progress of this therapy is easily monitored by conventional techniques and assays.

In one embodiment, the administration of the therapeutic agent is a single administration. In certain embodiments, the administration of the therapeutic agent is two or more administrations. In one such embodiment, the therapeutic agent is administered every week, every two weeks, or every three weeks, particularly every two weeks. In one embodiment, the therapeutic agent is administered in a therapeutically effective amount. In one embodiment the therapeutic agent is administered at a dose of 10 mg - 20 mg. In one embodiment the administration of the therapeutic agent comprises an initial administration of a dose of about 10 mg therapeutic agent, and one or more subsequent administrations of a dose of about 20 mg therapeutic agent.

In one embodiment, the administration of the therapeutic agent in the treatment regimen according to the invention is the first administration of that therapeutic agent to the subject (at least within the same course of treatment). In one embodiment, no administration of the therapeutic agent is made to the subject prior to the administration of the anti-CD20 antibody.

In the present invention, the therapeutic agent can be used either alone or in combination with other agents in a therapy. For instance, the therapeutic agent may be co-administered with at least one additional therapeutic agent. In certain embodiments, an additional therapeutic agent is an immunotherapeutic agent. In some embodiment, the additional therapeutic agent comprises an agent as described herein in relation to the therapeutic agent. The invention is particularly useful in relation to combinations of several therapeutic agents which may induce the formation of ADAs in a subject when used in a treatment regimen without the administration of the Type II anti-CD20 antibody. Such combinations may include various therapeutic agents as described herein, and may particularly include one or more immunotherapeutic agents, e.g. for the treatment of cancer (cancer immunotherapy).

Such combination therapies noted above encompass combined administration (where two or more therapeutic agents are included in the same or separate formulations), and separate administration, in which case, administration of the therapeutic agent can occur prior to, simultaneously, and/or following, administration of an additional therapeutic agent or agents. In one embodiment, administration of the therapeutic agent and administration of an additional therapeutic agent occur within about one month, or within about one, two or three weeks, or within about one, two, three, four, five, or six days, of each other.

**Articles of manufacture**

In another aspect of the invention, an article of manufacture, e.g. a kit, is provided, containing materials useful for the treatment, prevention and/or diagnosis of a disease, or for the reduction of the formation of anti-drug antibodies (ADAs) as described herein. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition, or holds a composition which is effective for reducing the formation of ADAs, and may have a sterile access port (for example the container may be an intravenous solution bag or a vial having a stopper pierceable by a hypodermic injection needle). At least one active agent in the composition is a Type II anti-CD20 antibody or a therapeutic agent as described herein. The label or package insert indicates that the composition is used for treating the condition of choice and/or to reduce the formation of ADAs. Moreover, the article of manufacture may comprise (a) a first container with a composition contained therein, wherein the composition comprises a Type II anti-CD20 antibody as described herein; and (b) a second container with a composition contained therein, wherein the composition comprises a therapeutic agent as described herein. The article of manufacture in this embodiment of the invention may further comprise a package insert indicating that the compositions can be used to treat a particular condition and/or to reduce the formation of ADAs. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Embodiments**

In the following, some of the embodiments of the invention are listed.

1. A method of treating a disease in a subject, the method comprising a treatment regimen comprising

(i) administration to the subject of a Type II anti-CD20 antibody,
and consecutively after a period of time
(ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

2. The method of embodiment 1, wherein the treatment regimen effectively reduces the formation of anti-drug antibodies (ADAs) in the subject in response to the administration of the therapeutic agent as compared to a corresponding treatment regimen without the administration of the anti-CD20 antibody.

3. A method for reducing the formation of anti-drug antibodies (ADAs) against a therapeutic agent in a subject, comprising administration of an anti-CD20 antibody to the subject prior to administration of the therapeutic agent.

4. The method of embodiment 3, wherein the period of time between the administration of the anti-CD20 antibody and administration of the therapeutic agent is sufficient for reduction of the number of B-cells in the subject in response to the administration of the CD20 antibody.

5. The method of any one of the preceding embodiments, wherein the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

6. The method of any one of the preceding embodiments, wherein the anti-CD20 antibody comprises the heavy chain variable region sequence of SEQ ID NO: 10 and the light chain variable region sequence of SEQ ID NO: 11.

7. The method of any one of the preceding embodiments, wherein the anti-CD20 antibody is an IgG antibody, particularly an $IgG_1$ antibody.

8. The method of any one of the preceding embodiments, wherein the anti-CD20 antibody is engineered to have an increased proportion of non-fucosylated oligosaccharides in the Fc region as compared to a non-engineered antibody.

9. The method of any one of the preceding embodiments, wherein at least about 40% of the N-linked oligosaccharides in the Fc region of the anti-CD20 antibody are non-fucosylated.

10. The method of any one of the preceding embodiments, wherein the anti-CD20 antibody is obinutuzumab.

11. The method of any one of the preceding embodiments, wherein the therapeutic agent comprises a polypeptide.

12. The method of any one of the preceding embodiments, wherein the therapeutic agent comprises an antibody.

13. The method of embodiment 12, wherein the antibody specifically binds to carcinoembryonic antigen (CEA).

14. The method of embodiment 13, wherein the antibody comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19.

15. The method of embodiment 13 or 14, wherein the antibody comprises the heavy chain variable region sequence of SEQ ID NO: 20 and the light chain variable region sequence of SEQ ID NO: 21.

16. The method of any one of the preceding embodiments, wherein the therapeutic agent comprises a cytokine.

17. The method of embodiment 16, wherein the cytokine is interleukin-2 (IL-2).

18. The method of embodiment 16 or 17, wherein the cytokine is a mutant human IL-2 polypeptide comprising the amino acid substitutions F42A, Y45A and L72G (numbering relative to the human IL-2 sequence SEQ ID NO: 12).

19. The method of any one of the preceding embodiments, wherein the therapeutic agent comprises an immunoconjugate.

20. The method of embodiment 19, wherein the immunoconjugate comprises an antibody as defined in any one of embodiments 13 to 15, and a cytokine as defined in embodiment 17 or 18.

21. The method of any one of the preceding embodiments, wherein the therapeutic agent comprises CEA-IL2v.

22. A Type II anti-CD20 antibody for use in a method of treating a disease in a subject, the method comprising a treatment regimen comprising

    (i) administration to the subject of the anti-CD20 antibody,
    and consecutively after a period of time
    (ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

23. The Type II anti-CD20 antibody of embodiment 22, wherein the treatment regimen effectively reduces the formation of anti-drug antibodies (ADAs) in the subject in response to the administration of the therapeutic agent as compared to a corresponding treatment regimen without the administration of the anti-CD20 antibody.

24. A Type II anti-CD20 antibody for use in a method for reducing the formation of anti-drug antibodies (ADAs) against a therapeutic agent in a subject, comprising administration of the anti-CD20 antibody to the subject prior to administration of the therapeutic agent.

25. The Type II anti-CD20 antibody of embodiment 24, wherein the period of time between the administration of the anti-CD20 antibody and administration of the therapeutic agent is sufficient for reduction of the number of B-cells in the subject in response to the administration of the CD20 antibody.

26. The Type II anti-CD20 antibody of any one of embodiments 22 to 25, wherein the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

27. The Type II anti-CD20 antibody of any one of embodiments 22 to 26, wherein the anti-CD20 antibody comprises the heavy chain variable region sequence of SEQ ID NO: 10 and the light chain variable region sequence of SEQ ID NO: 11.

28. The Type II anti-CD20 antibody of any one of embodiments 22 to 27, wherein the anti-CD20 antibody is an IgG antibody, particularly an $IgG_1$ antibody.

29. The Type II anti-CD20 antibody of any one of embodiments 22 to 28, wherein the anti-CD20 antibody is engineered to have an increased proportion of non-fucosylated oligosaccharides in the Fc region as compared to a non-engineered antibody.

30. The Type II anti-CD20 antibody of any one of embodiments 22 to 29, wherein at least about 40% of the N-linked oligosaccharides in the Fc region of the anti-CD20 antibody are non-fucosylated.

31. The Type II anti-CD20 antibody of any one of embodiments 22 to 30, wherein the anti-CD20 antibody is obinutuzumab.

32. The Type II anti-CD20 antibody of any one of embodiments 22 to 31, wherein the therapeutic agent comprises a polypeptide.

33. The Type II anti-CD20 antibody of any one of embodiments 22 to 32, wherein the therapeutic agent comprises an antibody.

34. The Type II anti-CD20 antibody of embodiment 33, wherein the antibody specifically binds to carcinoembryonic antigen (CEA).

35. The Type II anti-CD20 antibody of embodiment 34, wherein the antibody comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19.

36. The Type II anti-CD20 antibody of embodiment 34 or 35, wherein the antibody comprises the heavy chain variable region sequence of SEQ ID NO: 20 and the light chain variable region sequence of SEQ ID NO: 21.

37. The Type II anti-CD20 antibody of any one of embodiments 22 to 36, wherein the therapeutic agent comprises a cytokine.

38. The Type II anti-CD20 antibody of embodiment 37, wherein the cytokine is interleukin-2 (IL-2).

39. The Type II anti-CD20 antibody of embodiment 37 or 38, wherein the cytokine is a mutant human IL-2 polypeptide comprising the amino acid substitutions F42A, Y45A and L72G (numbering relative to the human IL-2 sequence SEQ ID NO: 12).

40. The Type II anti-CD20 antibody of any one of embodiments 22 to 39, wherein the therapeutic agent comprises an immunoconjugate.

41. The Type II anti-CD20 antibody of embodiment 40, wherein the immunoconjugate comprises an antibody as defined in any one of embodiments 34 to 36, and a cytokine as defined in embodiment 38 or 39.

42. The Type II anti-CD20 antibody of any one of embodiments 22 to 41, wherein the therapeutic agent comprises CEA-IL2v.

43. Use of a Type II anti-CD20 antibody in the manufacture of a medicament for reduction of the formation of anti-drug antibodies (ADAs) against a therapeutic agent in a subject,
wherein the medicament is to be used in a treatment regimen comprising

   (i) administration to the subject of the anti-CD20 antibody,
   and consecutively after a period of time
   (ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

44. Use of a therapeutic agent in the manufacture of a medicament for treatment of a disease in a subject, wherein the treatment comprises a treatment regimen comprising

   (i) administration to the subject of a Type II anti-CD20 antibody,
   and consecutively after a period of time
   (ii) administration to the subject of the therapeutic agent,

wherein the period of time between the administration of the anti-CD20 antibody and the administration of the therapeutic agent is sufficient for reduction of the number of B-cells in the subject in response to the administration of the CD20 antibody.

45. The use of embodiment 43 or 44, wherein the treatment regimen effectively reduces the formation of anti-drug antibodies (ADAs) against the therapeutic agent in the subject as compared to a corresponding treatment regimen without the administration of the anti-CD20 antibody.

46. The use of any one of embodiments 43 to 45, wherein the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

47. The use of any one of embodiments 43 to 46, wherein the anti-CD20 antibody comprises the heavy chain variable region sequence of SEQ ID NO: 10 and the light chain variable region sequence of SEQ ID NO: 11.

48. The use of any one of embodiments 43 to 47, wherein the anti-CD20 antibody is an IgG antibody, particularly an IgG$_1$ antibody.

49. The use of any one of embodiments 43 to 48, wherein the anti-CD20 antibody is engineered to have an increased proportion of non-fucosylated oligosaccharides in the Fc region as compared to a non-engineered antibody.

50. The use of any one of embodiments 43 to 49, wherein at least about 40% of the N-linked oligosaccharides in the Fc region of the anti-CD20 antibody are non-fucosylated.

51. The use of any one of embodiments 43 to 50, wherein the anti-CD20 antibody is obinutuzumab.

52. The use of any one of embodiments 43 to 51, wherein the therapeutic agent comprises a polypeptide.

53. The use of any one of embodiments 43 to 52, wherein the therapeutic agent comprises an antibody.

54. The use of embodiment 53, wherein the antibody specifically binds to carcinoembryonic antigen (CEA).

55. The use of embodiment 54, wherein the antibody comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19.

56. The use of embodiment 54 or 55, wherein the antibody comprises the heavy chain variable region sequence of SEQ ID NO: 20 and the light chain variable region sequence of SEQ ID NO: 21.

57. The use of any one of embodiments 43 to 56, wherein the therapeutic agent comprises a cytokine.

58. The use of embodiment 57, wherein the cytokine is interleukin-2 (IL-2).

59. The use of embodiment 57 or 58, wherein the cytokine is a mutant human IL-2 polypeptide comprising the amino acid substitutions F42A, Y45A and L72G (numbering relative to the human IL-2 sequence SEQ ID NO: 12).

60. The use of any one of embodiments 43 to 59, wherein the therapeutic agent comprises an immunoconjugate.

61. The use of embodiment 60, wherein the immunoconjugate comprises an antibody as defined in any one of embodiments 54 to 56, and a cytokine as defined in embodiment 58 or 59.

62. The use of any one of embodiments 43 to 61, wherein the therapeutic agent comprises CEA-IL2v.

63. A kit for the reduction of the formation of anti-drug antibodies (ADAs) against a therapeutic agent in a subject, comprising a package comprising a Type II anti-CD20 antibody composition and instructions for using the anti-CD20 antibody composition in a treatment regimen comprising

    (iii) administration to the subject of the anti-CD20 antibody composition,
    and consecutively after a period of time
    (iv) administration to the subject of a therapeutic agent,

wherein the period of time between the administration of the anti-CD20 antibody composition and the administration of the therapeutic agent is sufficient for reduction of the number of B-cells in the subject in response to the administration of the CD20 antibody.

64. The kit of embodiment 63, further comprising a therapeutic agent composition.

65. A kit for the treatment of a disease in a subject, comprising a package comprising a therapeutic agent composition and instructions for using the therapeutic agent composition in a treatment regimen comprising

    (iii) administration to the subject of a Type II anti-CD20 antibody,
    and consecutively after a period of time
    (iv) administration to the subject of the therapeutic agent composition,

wherein the period of time between the administration of the anti-CD20 antibody and the administration of the therapeutic agent composition is sufficient for reduction of the number of B-cells in the subject in response to the administration of the CD20 antibody.

66. The kit of embodiment 65, further comprising a Type II anti-CD20 antibody composition.

67. The kit of any one of embodiments 63 to 66, wherein the treatment regimen effectively reduces the formation of anti-drug antibodies (ADAs) against the therapeutic agent in the subject as compared to a corresponding treatment regimen without the administration of the anti-CD20 antibody composition.

68. The kit of any one of embodiments 63 to 67, wherein the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

69. The kit of any one of embodiments 63 to 68, wherein the anti-CD20 antibody comprises the heavy chain variable region sequence of SEQ ID NO: 10 and the light chain variable region sequence of SEQ ID NO: 11.

70. The kit of any one of embodiments 63 to 69, wherein the anti-CD20 antibody is an IgG antibody, particularly an IgG$_1$ antibody.

71. The kit of any one of embodiments 63 to 70, wherein the anti-CD20 antibody is engineered to have an increased proportion of non-fucosylated oligosaccharides in the Fc region as compared to a non-engineered antibody.

72. The kit of any one of embodiments 63 to 71, wherein at least about 40% of the N-linked oligosaccharides in the Fc region of the anti-CD20 antibody are non-fucosylated.

73. The kit of any one of embodiments 63 to 72, wherein the anti-CD20 antibody is obinutuzumab.

74. The kit of any one of embodiments 63 to 73, wherein the therapeutic agent comprises a polypeptide.

75. The kit of any one of embodiments 63 to 74, wherein the therapeutic agent comprises an antibody.

76. The kit of embodiment 75, wherein the antibody specifically binds to carcinoembryonic antigen (CEA).

77. The kit of embodiment 76, wherein the antibody comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19.

78. The kit of embodiment 76 or 77, wherein the antibody comprises the heavy chain variable region sequence of SEQ ID NO: 20 and the light chain variable region sequence of SEQ ID NO: 21.

79. The kit of any one of embodiments 63 to 78, wherein the therapeutic agent comprises a cytokine.

80. The kit of embodiment 79, wherein the cytokine is interleukin-2 (IL-2).

81. The kit of embodiment 79 or 80, wherein the cytokine is a mutant human IL-2 polypeptide comprising the amino acid substitutions F42A, Y45A and L72G (numbering relative to the human IL-2 sequence SEQ ID NO: 12).

82. The kit of any one of embodiments 63 to 81, wherein the therapeutic agent comprises an immunoconjugate.

83. The kit of embodiment 82, wherein the immunoconjugate comprises an antibody as defined in any one of embodiments 76 to 78, and a cytokine as defined in embodiment 80 or 81.

84. The kit of any one of embodiments 63 to 83, wherein the therapeutic agent comprises CEA-IL2v.

85. A therapeutic agent for use in a method of treating a disease in a subject, the method comprising a treatment regimen comprising

(i) administration to the subject of a Type II anti-CD20 antibody,
and consecutively after a period of time

(ii) administration to the subject of the therapeutic agent,

wherein the period of time between the administration of the anti-CD20 antibody and the administration of the therapeutic agent is sufficient for reduction of the number of B-cells in the subject in response to the administration of the CD20 antibody.

86. The therapeutic agent of embodiment 85, wherein the treatment regimen effectively reduces the formation of anti-drug antibodies (ADAs) in the subject in response to the administration of the therapeutic agent as compared to a corresponding treatment regimen without the administration of the anti-CD20 antibody.

87. The therapeutic agent of embodiment 85 or 86, wherein the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

88. The therapeutic agent of any one of embodiments 85 to 87, wherein the anti-CD20 antibody comprises the heavy chain variable region sequence of SEQ ID NO: 10 and the light chain variable region sequence of SEQ ID NO: 11.

89. The therapeutic agent of any one of embodiments 85 to 88, wherein the anti-CD20 antibody is an IgG antibody, particularly an IgG$_1$ antibody.

90. The therapeutic agent of any one of embodiments 85 to 89, wherein the anti-CD20 antibody is engineered to have an increased proportion of non-fucosylated oligosaccharides in the Fc region as compared to a non-engineered antibody.

91. The therapeutic agent of any one of embodiments 85 to 90, wherein at least about 40% of the N-linked oligosaccharides in the Fc region of the anti-CD20 antibody are non-fucosylated.

92. The therapeutic agent of any one of embodiments 85 to 91, wherein the anti-CD20 antibody is obinutuzumab.

93. The therapeutic agent of any one of embodiments 85 to 92, wherein the therapeutic agent comprises a polypeptide.

94. The therapeutic agent of any one of embodiments 85 to 93, wherein the therapeutic agent comprises an antibody.

95. The therapeutic agent of embodiment 94, wherein the antibody specifically binds to carcinoembryonic antigen (CEA).

96. The therapeutic agent of embodiment 95, wherein the antibody comprises a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19.

97. The therapeutic agent of embodiment 95 or 96, wherein the antibody comprises the heavy chain variable region sequence of SEQ ID NO: 20 and the light chain variable region sequence of SEQ ID NO: 21.

98. The therapeutic agent of any one of embodiments 85 to 97, wherein the therapeutic agent comprises a cytokine.

99. The therapeutic agent of embodiment 98, wherein the cytokine is interleukin-2 (IL-2).

100. The therapeutic agent of embodiment 98 or 99, wherein the cytokine is a mutant human IL-2 polypeptide comprising the amino acid substitutions F42A, Y45A and L72G (numbering relative to the human IL-2 sequence SEQ ID NO: 12).

101. The therapeutic agent of any one of embodiments 85 to 100, wherein the therapeutic agent comprises an immunoconjugate.

102. The therapeutic agent of embodiment 101, wherein the immunoconjugate comprises an antibody as defined in any one of embodiments 95 to 97, and a cytokine as defined in embodiment 99 or 100.

103. The therapeutic agent of any one of embodiments 85 to 102, wherein the therapeutic agent comprises CEA-IL2v.

**Examples**

The following are examples of methods and compositions of the invention. It is understood that various other embodiments may be practiced, given the general description provided above.

**Example 1**

**Prior treatment with obinutuzumab but not rituximab or vehicle results in the attenuation of tetanus toxoid specific de novo IgG antibody responses in cynomolgus monkeys**

To evaluate the functional impact that B-cell depletion with obinutuzumab or rituximab has on the humoral immune response to foreign antigens, cynomolgus monkeys were immune challenged after treatment with either a novel antigen that the animals had never experienced before (de novo response to tetanus toxoid) or with a booster immune rechallenge with an immunogen that the animals had already encountered prior to the CD20 antibody administration (memory recall response to measles/rubella).
Animals were administered on day -14 and day -7 rituximab or obinutuzumab at a dose of 30 mg/kg or vehicle by i.v. infusion. Immunization with tetanus toxoid was performed on Day 0. Naïve animals from all groups had a baseline anti-tetanus toxoid IgG measurement of around 0.1 IU/ml at day 0. At Day 7, vehicle treated animals and rituximab treated animals mounted robust humoral anti-tetanus toxoid IgG responses, with an increase to around 1.0 IU/ml, while obinutuzumab treated animals showed attenuated responses, resulting in an equal to background signal of 0.1 IU/ml. By Day 21, serological titers in vehicle treated and rituximab treated animals continued to rise to peak levels of 2.5 IU/ml (Figure 1). The obinutuzumab treated animals began to display a slight increase in titers to 0.5 IU/ml, which was significantly below of that of vehicle and rituximab treated groups. The serum IgG response waned by day 51 and 68 in all groups reaching around 1.5 IU/ml in vehicle, 1.3 IU/ml in rituximab and returning to 0.2 IU/ml in obinutuzumab treated animals. These results indicate that prior treatment with obinutuzumab, but not rituximab, results in the attenuation of tetanus toxoid specific de novo IgG antibody responses in cynomolgus monkeys.
To investigate the memory recall responses by measles specific IgG antibody production in response to immune re-challenge with a measles/rubella booster vaccination animals that had measurable baseline positive anti-measles titers were selected. Animals were administered rituximab or obinutuzumab at a dose of 30 mg/kg or vehicle by i.v. infusion on day -14 and Day -7. Immune re-challenge with a measles/rubella vaccination was performed on Day 0. Measuring the fold change at optical density OD450 nm reading over baseline Day -14 reading, resulted in measurable increase in anti-measles titers in all three groups at Day 21, Day 51 and Day 68 with no significant difference found in the IgG responses to measles amongst the three different treatment groups (Figure 2). The conclusion is that memory recall responses were left intact regardless of anti-CD20 depletion therapy. The administration of either obinutuzumab or rituximab had no measurable impact on the memory recall responses to a booster immunization against measles/rubella vaccination with established basal titers as a result of prior vaccination.
Overall, these results showed that prior treatment with obinutuzumab led to strong suppression of de novo antibody responses, but left the protective humoral memory responses intact. Without wishing to be bound by theory, the ability to block de novo humoral antibody responses may possibly be attributed to either the increased extent of endogenous B-cell depletion seen with obinutuzumab and/or the enhanced ability of obinutuzumab to deplete activated, CD20 expressing B cells.

**Example 2**

**A multi-center, randomized, open-label phase 1 study to evaluate feasibility, safety and pharmacodynamic effect of pretreatment with obinutuzumab prior to therapy with RO6895882 (CEA-IL2v), in patients with locally advanced and/or metastatic solid tumors**

Methods

An open-label, multi-center, randomized Phase 1b clinical sub-study of RO6895882 given with or without obinutuzumab as pre-treatment is undertaken.
The main objective of this sub-study is to determine if pre-treatment with obinutuzumab prevents the formation of ADAs in patients treated subsequently with RO6895882.
The trial enrolls patients with locally advanced and/or metastatic CEA-positive solid tumors that have progressed on or are intolerant to the standard of care therapy. Obinutuzumab and RO6895882 are administered intravenously (IV).

Fifteen patients were randomized into the obinutuzumab pretreatment arm and five patients to the control arm without obinutuzumab pretreatment. The patients in the obinutuzumab pretreatment arm receive 2 g of obinutuzumab, administered on two consecutive days, Day - 13 and Day -12 (+/- 2 days) before the Cycle 1 Day 1 (C1D1) RO6895882 administration. Pre-medication is given prior to each obinutuzumab dosing. On C1D1 all patients receive a fixed and flat 10 mg dose of RO6895882. At subsequent cycles, all patients receive 20 mg of RO6895882, administered over a minimum of 2 hour IV infusion bi-weekly (q2W). The patients in the control arm (without obinutuzumab pretreatment) receive RO6895882 administrations starting at C1D1 identically to the patients in the obinutuzumab pretreatment arm.

Blood samples are collected before and during the treatment period for the monitoring of B lymphocyte counts. B cell counts are obtained using flow cytometry and staining for CD 19. Blood samples for ADA determination are obtained at baseline and thereafter every second week after the RO6895882 administration.

All patients undergo baseline and on-treatment tumor biopsies. For the obinutuzumab pretreated patients, baseline biopsies are taken after randomization, before administration of obinutuzumab. The control patients have tumor biopsies taken prior to the Cycle 1 Day 1 RO6895882 administration. On-treatment tumor biopsies from the first five patients pretreated with obinutuzumab are collected on Cycle 1 Day 1 (+0/-1 days) prior to RO6895882 administration in order to confirm tissue B cell depletion before the start of RO6895882 treatment. From the remaining obinutuzumab pre-treated patients the repeated tumor biopsies are collected on Cycle 3 Day 14 (+/-2 days), prior to the fourth RO6895882 administration on Cycle 4 Day 1. One portion of the biopsy tissue is analyzed by flow cytometry and staining with CD 19 for B lymphocyte detection. The second portion is formalin fixed and embedded in paraffin and analyzed for B lymphocytes using CD20 and PAX5 staining.

The primary objectives for this study are to assess the effect of pre-treatment with obinutuzumab on decreasing the proportion of patients with ADA titer at cycle 4, and to evaluate the safety and tolerability of administration of obinutuzumab prior to treatment with RO6895882.

Secondary objectives for this study include characterization of the RO6895882 ADAs (at cycle 4) and investigation and characterization of the B-cell depletion in tissue (tumor and skin) and peripheral blood resulting from obinutuzumab pre-treatment.

Results

RO6895882 (CEA-IL2v) has been tested in Study BP28920 EIH Study (ClinicalTrials.gov identifier: NCT02004106). As of 17 November 2015, preliminary results from Study BP28920 show that in 43 of 53 patients (81%) anti-CEA-IL2v antibodies were detected. Among the 43 ADA-positive patients, 34 (79%) showed a persistent immune response while 9 (21%) showed a transient immune response. The intensity of ADA responses ranges from low to high titers (10 - 196.830), 16 out of 34 persistent ADA-positive cases (47%) show high ADA titers (>1000). The first onset of an immune response was observed after one dose of RO6895882 (CEA-IL2v) at day 8, earliest on day 5. No patient with pre-existing ADAs was identified and all ADAs were treatment induced. In ten out of 34 patients (29%) with a persistent immune response, the sustained ADA presence correlated with loss of exposure. So far no hypersensitivity reactions or signs and symptoms suggestive of hypersensitivity, nor evidence of ADA-mediated adverse events have been reported in the 107 patients who received RO6895882.

The above-described clinical sub-study was initiated to explore the potential of obinutuzumab (Gazyva®) given as a pre-treatment to administration of RO6895882 (CEA-IL2v) to attenuate development of anti-drug antibodies (ADAs). The study is ongoing.

According to the preliminary data, 11 out of 11 obinutuzumab treated patients for which ADA data is available so far remained free of ADAs against RO6895882 on Cycle 2 Day 1 (two weeks after first RO6895882 administration). Four out of five control patients who were not pre-treated with obinutuzumab developed ADAs with titers of 10 to 90 after 1 dose of RO6895882.

Based on a preliminary safety analysis, the safety profile of obinutuzumab pre-treatment was acceptable in patients with locally advanced and/or metastatic solid tumors. Patients receiving obinutuzumab/CEA-IL2v treatment did not experience unexpected AEs compared to the CEA-IL2v monotherapy. No fatal outcome due to adverse event was reported.

Flow cytometric analysis of peripheral blood indicated complete depletion of B cells after obinutuzumab treatment. Before the start of treatment with RO6895882 (C1D1), no B cells were detectable in the collected blood samples (Figure 3).

The tumor biopsy samples from obinutuzumab pre-treatment patients were analyzed by both flow cytometry and immunohistochemistry (IHC) for the frequency of B cells. Flow cytometric analysis (Figure 4) showed a strong reduction in the number and percentage of cells that stain positive for CD19, a surface protein expressed on B lymphocytes. IHC analysis of a different portion of the paired tumor biopsy samples detected a decrease in cells staining positive for CD20, a second antigen expressed on B cells (Figure 5 A and B). In order to exclude that the reduction in CD20 staining was caused by obinutuzumab which may compete with anti-CD20 staining, the results were confirmed by staining with PAX5, a B cell specific transcription factor, which was also strongly reduced (Figure 5 C and D). Taken together, these results indicate an effective reduction of B cells in the tumor and adjacent normal tissue.

In conclusion, the preliminary data from this sub-study strongly suggest that obinutuzumab is efficacious in mitigating ADAs.

**Example 3**

**Clinical evaluation of feasibility, safety and pharmacodynamic effect of pretreatment with obinutuzumab prior to therapy with RO6958688 (CEA TCB)**

In the ongoing phase I clinical trial with CEA TCB (RO6958688) (ClinicalTrials.gov identifier NCT02324257), a cohort of patients is opened that will be pretreated with obinutuzumab.

The main objective of this cohort is to determine if pre-treatment with obinutuzumab prevents the formation of ADAs in patients treated subsequently with CEA TCB (RO6958688).

The trial enrolls patients with locally advanced and/or metastatic CEA-positive solid tumors that have progressed on or are intolerant to the standard of care therapy. Obinutuzumab and CEA TCB (RO6958688) are administered intravenously (IV).

The patients in the obinutuzumab pretreatment cohort receive 2 g of obinutuzumab, administered on two consecutive days, Day -13 and Day -12 (+/- 2-5 days) before the Cycle 1 Day 1 (C1D1) CEA TCB (RO6958688) administration. Pre-medication is given prior to each obinutuzumab dosing. On C1D1 all patients receive a fixed and flat >x mg dose (as determined in the ongoing clinical trial) of CEA TCB (RO6958688). At subsequent cycles, all patients receive 20 mg of CEA TCB (RO6958688), administered over a minimum of 2 hour IV infusion bi-weekly (q2W). The patients in the control arm (without obinutuzumab pretreatment) receive CEA TCB (RO6958688) administrations starting at C1D1 identically to the patients in the obinutuzumab pretreatment arm.

Blood samples are collected before and during the treatment period for the monitoring of B lymphocyte counts. B cell counts are obtained using flow cytometry and staining for CD 19. Blood samples for ADA determination are obtained at baseline and thereafter every second week after the CEA TCB administration.

The primary objectives for this study cohort are to assess the effect of pre-treatment with obinutuzumab on decreasing the proportion of patients with ADA titer at cycle 4, and to evaluate the safety and tolerability of administration of obinutuzumab prior to treatment with CEA TCB (RO6958688).

Secondary objectives for this study include characterization of the CEA TCB (RO6958688) ADAs (at cycle 4) and investigation and characterization of the B-cell depletion in tissue (tumor and skin) and peripheral blood resulting from obinutuzumab pre-treatment.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

**Claims**

1.  A Type II anti-CD20 antibody for use in a method of treating a disease in a subject, the method comprising a treatment regimen comprising

(i) administration to the subject of the anti-CD20 antibody,
and consecutively after a period of time
(ii) administration to the subject of a therapeutic agent,

wherein the period of time between the administration of the anti-CD20 antibody and the administration of the therapeutic agent is sufficient for reduction of the number of B-cells in the subject in response to the administration of the CD20 antibody.

2. The Type II anti-CD20 antibody of claim 1, wherein the treatment regimen effectively reduces the formation of anti-drug antibodies (ADAs) in the subject in response to the administration of the therapeutic agent as compared to a corresponding treatment regimen without the administration of the anti-CD20 antibody.

3. A Type II anti-CD20 antibody for use in a method for reducing the formation of anti-drug antibodies (ADAs) against a therapeutic agent in a subject, comprising administration of the anti-CD20 antibody to the subject prior to administration of the therapeutic agent.

4. The Type II anti-CD20 antibody of claim 3, wherein the period of time between the administration of the anti-CD20 antibody and administration of the therapeutic agent is sufficient for reduction of the number of B-cells in the subject in response to the administration of the CD20 antibody.

5. The Type II anti-CD20 antibody of any one of the preceding claims, wherein the anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

6. The Type II anti-CD20 antibody of any one of the preceding claims, wherein the anti-CD20 antibody comprises the heavy chain variable region sequence of SEQ ID NO: 10 and the light chain variable region sequence of SEQ ID NO: 11.

7. The Type II anti-CD20 antibody of any one of the preceding claims, wherein the anti-CD20 antibody is an IgG antibody, particularly an IgG$_1$ antibody, and wherein at least about 40% of the N-linked oligosaccharides in the Fc region of the anti-CD20 antibody are non-fucosylated.

8. The Type II anti-CD20 antibody of any one of the preceding claims, wherein the anti-CD20 antibody is obinutuzumab.

9. The Type II anti-CD20 antibody of any one of the preceding claims, wherein the therapeutic agent comprises a polypeptide.

10. The Type II anti-CD20 antibody of any one of the preceding claims, wherein the therapeutic agent comprises an antibody.

11. The Type II anti-CD20 antibody of claim 10, wherein the antibody specifically binds to carcinoembryonic antigen (CEA).

12. The Type II anti-CD20 antibody of claim 11, wherein the antibody comprises

(i) a heavy chain variable region comprising the heavy chain CDR (HCDR) 1 of SEQ ID NO: 14, the HCDR2 of SEQ ID NO: 15, and the HCDR3 of SEQ ID NO: 16; and a light chain variable region comprising the light chain CDR (LCDR) 1 of SEQ ID NO: 17, the LCDR2 of SEQ ID NO: 18 and the LCDR3 of SEQ ID NO: 19; or
(ii) a heavy chain variable region sequence of SEQ ID NO: 20 and a light chain variable region sequence of SEQ ID NO: 21.

13. The Type II anti-CD20 antibody of any one of the preceding claims, wherein the therapeutic agent comprises a cytokine, particularly interleukin-2.

14. The Type II anti-CD20 antibody of claim 13, wherein the cytokine is a mutant human IL-2 polypeptide comprising the amino acid substitutions F42A, Y45A and L72G (numbering relative to the human IL-2 sequence SEQ ID NO: 12).

15. The Type II anti-CD20 antibody of any one of the preceding claims, wherein the therapeutic agent comprises an immunoconjugate comprising an antibody as defined in claim 12, and a cytokine as defined in claim 14.

**Fig. 1**

Fig. 2

Fig. 3

Fig. 4

Fig. 5

Europäisches
Patentamt

European
Patent Office

Office européen
des brevets

## EUROPEAN SEARCH REPORT

Application Number

EP 15 19 8715

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/095410 A1 (GENENTECH INC [US]; HOFFMANN LA ROCHE [CH]) 25 June 2015 (2015-06-25) | 1,5-10 | INV. C07K16/28 A61K39/395 C07K16/30 |
| Y | * example 1 page 112 Seq ID No. 56, 57 * | 11-15 | |
| Y | HASSAN RAFFIT ET AL: "Pretreatment with rituximab does not inhibit the human immune response against the immunogenic protein LMB-1", CLINICAL CANCER RESEARCH, THE AMERICAN ASSOCIATION FOR CANCER RESEARCH, US, vol. 10, no. 1 Pt 1, 16 January 2004 (2004-01-16), pages 16-18, XP002547018, ISSN: 1078-0432, DOI: 10.1158/1078-0432.CCR-1160-3 * abstract * | 1,5-15 | |
| Y | M. E. MOSSOBA ET AL: "Pentostatin Plus Cyclophosphamide Safely and Effectively Prevents Immunotoxin Immunogenicity in Murine Hosts", CLINICAL CANCER RESEARCH, vol. 17, no. 11, 26 April 2011 (2011-04-26), pages 3697-3705, XP055266902, US ISSN: 1078-0432, DOI: 10.1158/1078-0432.CCR-11-0493 * abstract * | 1,5-15 | TECHNICAL FIELDS SEARCHED (IPC) C07K A61K |

-/--

The present search report has been drawn up for all claims

| Place of search | Date of completion of the search | Examiner |
|---|---|---|
| Munich | 21 April 2016 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

**EUROPEAN SEARCH REPORT**

Europäisches Patentamt
European Patent Office
Office européen des brevets

Application Number

EP 15 19 8715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | M. ONDA ET AL: "Tofacitinib Suppresses Antibody Responses to Protein Therapeutics in Murine Hosts", THE JOURNAL OF IMMUNOLOGY, vol. 193, no. 1, 2 June 2014 (2014-06-02), pages 48-55, XP055266908, US ISSN: 0022-1767, DOI: 10.4049/jimmunol.1400063 * abstract * | 1,5-15 | |
| Y | Cb Siegall ET AL: "Prevention of immunotoxin-induced immunogenicity by coadministration with CTLA4Ig enhances antitumor efficacy", The Journal of Immunology, 15 November 1997 (1997-11-15), pages 5168-5173, XP055266904, UNITED STATES Retrieved from the Internet: URL:http://www.jimmunol.org/content/159/10/5168.full.pdf#page=1&view=FitH [retrieved on 2016-04-20] * abstract * | 1,5-15 | |
| Y | WO 2012/117002 A1 (ROCHE GLYCART AG [CH]; HOFER THOMAS U [CH]; HOSSE RALF [CH]; MOESSNER) 7 September 2012 (2012-09-07) * sequences 239, 209 * | 10-12 | |
| Y,D | WO 2012/107417 A1 (ROCHE GLYCART AG [CH]; AST OLIVER [CH]; BRUENKER PETER [CH]; FREIMOSER) 16 August 2012 (2012-08-16) * pages 4-7, 22-25 examples * | 9-15 | |

TECHNICAL FIELDS SEARCHED (IPC)

-/--

The present search report has been drawn up for all claims

| Place of search | Date of completion of the search | Examiner |
|---|---|---|
| Munich | 21 April 2016 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 19 8715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2009/134738 A1 (GENENTECH INC [US]; KELMAN ARIELLA [US]; TRZASKOMA BENJAMIN L [US]) 5 November 2009 (2009-11-05)<br>* examples<br>claims * | 1-15 | |
| A | CHRISTIAN KLEIN ET AL: "Epitope interactions of monoclonal antibodies targeting CD20 and their relationship to functional properties",<br>MABS,<br>vol. 5, no. 1, 1 January 2013 (2013-01-01), pages 22-33, XP055117097,<br>ISSN: 1942-0862, DOI: 10.4161/mabs.22771<br>* abstract<br>figures * | 1-15 | |
| A | MARINUS H. J. VAN OERS: "CD20 antibodies: type II to tango?",<br>BLOOD,<br>vol. 119, 31 May 2015 (2015-05-31), pages 5061-5063, XP055266601,<br>DOI: http://dx.doi.org/10.1182/blood-2012-04-420711<br>* pages 5061-5062 * | 1-15 | TECHNICAL FIELDS SEARCHED (IPC) |

The present search report has been drawn up for all claims

| Place of search | Date of completion of the search | Examiner |
|---|---|---|
| Munich | 21 April 2016 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 19 8715

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2016

| Patent document cited in search report | Publication date | Patent family member(s) | | Publication date |
|---|---|---|---|---|
| WO 2015095410 A1 | 25-06-2015 | US | 2015210772 A1 | 30-07-2015 |
| | | WO | 2015095410 A1 | 25-06-2015 |
| WO 2012117002 A1 | 07-09-2012 | AR | 085591 A1 | 16-10-2013 |
| | | AU | 2012222463 A1 | 25-07-2013 |
| | | CA | 2827722 A1 | 07-09-2012 |
| | | CN | 103403029 A | 20-11-2013 |
| | | CO | 6781491 A2 | 31-10-2013 |
| | | CR | 20130359 A | 11-11-2013 |
| | | EA | 201300978 A1 | 28-02-2014 |
| | | EC | SP13012859 A | 31-10-2013 |
| | | EP | 2681244 A1 | 08-01-2014 |
| | | JP | 2014509200 A | 17-04-2014 |
| | | KR | 20140021588 A | 20-02-2014 |
| | | MA | 34927 B1 | 01-02-2014 |
| | | NZ | 614125 A | 31-07-2015 |
| | | PE | 10172014 A1 | 25-08-2014 |
| | | SG | 192972 A1 | 30-09-2013 |
| | | US | 2012251529 A1 | 04-10-2012 |
| | | US | 2014212408 A1 | 31-07-2014 |
| | | WO | 2012117002 A1 | 07-09-2012 |
| WO 2012107417 A1 | 16-08-2012 | AR | 085335 A1 | 25-09-2013 |
| | | AU | 2012215573 A1 | 02-05-2013 |
| | | AU | 2015249085 A1 | 12-11-2015 |
| | | CA | 2824253 A1 | 16-08-2012 |
| | | CL | 2013001987 A1 | 31-01-2014 |
| | | CN | 103492411 A | 01-01-2014 |
| | | CN | 105440123 A | 30-03-2016 |
| | | CO | 6741186 A2 | 30-08-2013 |
| | | CR | 20130314 A | 09-08-2013 |
| | | EA | 201300896 A1 | 28-02-2014 |
| | | EC | SP13012815 A | 31-10-2013 |
| | | EP | 2673294 A1 | 18-12-2013 |
| | | JP | 5878182 B2 | 08-03-2016 |
| | | JP | 2014506793 A | 20-03-2014 |
| | | JP | 2015229676 A | 21-12-2015 |
| | | KR | 20130118363 A | 29-10-2013 |
| | | KR | 20150092367 A | 12-08-2015 |
| | | NZ | 611749 A | 28-08-2015 |
| | | PE | 03032014 A1 | 22-03-2014 |
| | | SG | 192673 A1 | 30-09-2013 |
| | | TW | 201237165 A | 16-09-2012 |
| | | US | 2012244112 A1 | 27-09-2012 |
| | | WO | 2012107417 A1 | 16-08-2012 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 15 19 8715

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2016

| Patent document cited in search report | Publication date | Patent family member(s) | Publication date |
|---|---|---|---|
| WO 2009134738 A1 | 05-11-2009 | US 2009269339 A1<br>WO 2009134738 A1 | 29-10-2009<br>05-11-2009 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RO 6895882
- WO 2005044859 A
- WO 2004035607 A
- WO 2005103081 A
- WO 2004056312 A
- WO 2007031875 A
- US 20030124678 A
- US 5229109 A
- US 20070036752 A
- WO 20080034473 A
- WO 2009061853 A
- WO 2012107417 A
- WO 2004065540 A
- US 495142 P
- US 20040241817 A
- US 5500362 A
- US 5821337 A
- WO 2006029879 A
- WO 2005100402 A
- US 6737056 B
- WO 2004063351 A
- WO 2004099249 A
- WO 9954342 A
- US 6602684 B
- EP 1071700 A
- EP 1587921 A
- WO 03011878 A
- US 20030175884 A
- US 20030157108 A
- EP 1176195 A1
- WO 03084570 A
- WO 03085119 A
- US 20030115614 A
- US 2004093621 A
- US 2004110282 A
- US 2004110704 A
- US 2004132140 A
- US 6946292 B
- WO 03056914 A
- WO 2004057002 A
- WO 2004024927 A
- WO 9316185 A
- US 5571894 A
- US 5587458 A
- US 5869046 A
- EP 404097 A
- WO 199301161 A
- US 6248516 B1
- US 4816567 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2009080251 A
- WO 2009080252 A
- WO 2009080253 A
- WO 2009080254 A
- WO 2004106381 A
- WO 2005061547 A
- WO 2007042261 A
- WO 2008119567 A
- EP 1691833 A
- US 5208020 A
- US 5416064 A
- EP 0425235 B1
- US 5635483 A
- US 5780588 A
- US 7498298 B
- US 5712374 A
- US 5714586 A
- US 5739116 A
- US 5767285 A
- US 5770701 A
- US 5770710 A
- US 5773001 A
- US 5877296 A
- US 6630579 B

- WO 9411026 A
- WO 2006121168 A
- WO 2009114335 A
- WO 2009101611 A
- WO 2010027827 A
- WO 2011066342 A
- WO 2010077634 A
- WO 2007005874 A
- WO 2011066389 A
- US 2013034559 A
- US 4518584 A
- US 5206344 A
- US 5116943 A
- WO 2012146628 A
- WO 2013026833 A
- WO 2014131712 A
- WO 9627011 A
- WO 98050431 A

- EP 1870459 A
- WO 2007110205 A
- WO 2007147901 A
- WO 2009089004 A
- WO 2010129304 A
- WO 201190754 A
- WO 2011143545 A
- WO 2012058768 A
- WO 2013157954 A
- WO 2013096291 A
- US 7695936 B
- EP 1870459 A1
- WO 2013157953 A
- WO 2011090762 A
- WO 2012130831 A
- US 7332581 B
- RO 6958688

**Non-patent literature cited in the description**

- **SCHELLEKENS ; CASADEVALL.** *J Neurol,* 2004, vol. 251 (2), II/4-II/9
- **MOSSOBA et al.** *Clin Cancer Res,* 2011, vol. 17 (11), 3697-3705
- **HSU et al.** *British Journal of Dermatology,* 2014, vol. 170, 261-273
- **SCHAEVERBECKE et al.** *Rheumatology,* 2015
- **MENDELSOHN et al.** *NEJM,* 2009, vol. 360 (2), 194-195
- **HASSAN et al.** *Clin Cancer Res,* 2004, vol. 10, 16-18
- **VALENTINE, M.A. et al.** *J. Biol. Chem.,* 1989, vol. 264, 11282-11287
- **TEDDER, T.F. et al.** *Proc. Natl. Acad. Sci. U.S.A.,* 1988, vol. 85, 208-212
- **STAMENKOVIC, L et al.** *J. Exp. Med.,* 1988, vol. 167, 1975-1980
- **EINFELD, D.A. et al.** *EMBO J.,* 1988, vol. 7, 711-717
- **TEDDER, T.F. et al.** *J. Immunol.,* 1989, vol. 142, 2560-2568
- **CRAGG et al.** *Blood,* 2004, vol. 103, 2738-2743
- **CRAGG et al.** *Blood,* 2003, vol. 101, 1045-1052
- **KLEIN et al.** *mAbs,* 2013, vol. 5, 22-33
- **POPPEMA, S. ; VISSER, L.** *Biotest Bulletin,* 1987, vol. 3, 131-139
- **SAUVÉ et al.** *Proc Natl Acad Sci USA,* 1991, vol. 88, 4636-40
- **HU et al.** *Blood,* 2003, vol. 101, 4853-4861
- **SHANAFELT et al.** *Nature Biotechnol,* 2000, vol. 18, 1197-1202

- **HEATON et al.** *Cancer Res,* 1993, vol. 53, 2597-602
- **OLEJNICZAK ; KASPRZAK.** *Med Sci Monit,* 2008, vol. 14, 179-189
- **SAKAGUCHI.** *Annu Rev Immunol,* 2004, vol. 22, 531-62
- **LILJEBLAD et al.** *Glyco J,* 2000, vol. 17, 323-329
- **HEELEY.** *Endocr Res,* 2002, vol. 28, 217-229
- **KINDT et al.** Kuby Immunology. W.H. Freeman and Co, 2007, 91
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **STAMENKOVIC, I. et al.** *J. Exp. Med.,* 1988, vol. 167, 1975-1980
- **ANDERSON, K.C. et al.** *Blood,* 1984, vol. 63, 1424-1433
- **TEDDER, T.F. et al.** *J, Immunol.,* 1985, vol. 135, 973-979
- **CRAGG, M.S. et al.** *Blood,* 2004, vol. 103, 2738-2743
- **CRAGG, M.S. et al.** *Blood,* 2003, vol. 101, 1045-1052
- **POPPEMA ; VISSER.** *Biotest Bulletin,* 1987, vol. 3, 131-139
- *WHO Drug Information,* 2012, vol. 26 (4), 453
- *WHO Drug Information,* 2009, vol. 23 (2), 176
- *WHO DRUG INFORMATION,* 2008, vol. 22 (2), 124
- **HELLSTROM et al.** *Proc Natl Acad Sci USA,* 1986, vol. 83, 7059-7063

- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **GAZZANO-SANTORO et al.** *J Immunol Methods,* 1996, vol. 202 (163
- **CRAGG ; GLENNIE.** *Blood,* 2004, vol. 103, 2738-2743
- **SHIELDS et al.** *J Biol Chem,* 2001, vol. 9 (2), 6591-6604
- **UMANA et al.** *Nat Biotechnol,* 1999, vol. 17, 176-180
- **FERRARA et al.** *Biotechn Bioeng,* 2006, vol. 93, 851-861
- **YAMANE-OHNUKI et al.** *Biotech Bioeng,* 2004, vol. 87, 614
- **KANDA et al.** *Biotechnol Bioeng,* 2006, vol. 94 (4), 680-688
- **NIWA et al.** *J Immunol Methods,* 2006, vol. 306, 151-160
- **RIPKA et al.** *Arch Biochem Biophys,* 1986, vol. 249, 533-545
- **HUDSON et al.** *Nat. Med.,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229 (81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147 (60
- **TUFT et al.** *J. Immunol.,* 1991, vol. 147, 60
- **HINMAN et al.** *Cancer Res.,* 1993, vol. 53, 3336-3342

- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI et al.** *Cancer Res.,* 1992, vol. 52, 127-131
- **WEIGER et al.** *Eur J Biochem,* 1989, vol. 180, 295-300
- **STUBENRAUCH et al.** *Drug Metabolism and Disposition,* 2010, vol. 38, 84-91
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **GAZZANO-SANTORO et al.** *J Immunol Methods,* 1996, vol. 202, 163
- **MIRE-SLUIS et al.** *J Immunol Methods,* 2004, vol. 289, 1-16
- **NENCINI et al.** *Drug Dev Res,* 2014, vol. 75 (1), 4-6
- **SCHOUWENBURG et al.** *Nat Rev Rheumatol,* 2015, vol. 9, 164-172
- **LOTTSPEICH.** Bioanalytik. Spektrum Akademisher Verlag, 1998
- **SAMBROOK ; RUSSELL.** Molecular Cloning: A Laboratory Manual. CSH Press, 2001